# EUROPEAN PATENT APPLICATION

(11) **EP 3 714 901 A1**
(43) Date of publication of application: **30.09.2020**
(21) Application number: 18892762.8
(22) Date of filing: 21.12.2018
(51) Int. Cl.: A61K 39/395, A61P 35/00

(54) **LAG-3 ANTIBODY PHARMACEUTICAL COMPOSITION AND USE THEREOF**

(30) Priority: 22.12.2017 CN 201711408330
(71) Applicant: Jiangsu Hengrui Medicine Co., Ltd., Lianyungang, Jiangsu 222047 (CN); Shanghai Hengrui Pharmaceutical Co., Ltd., Shanghai 200245 (CN)
(72) Inventor: WU, Tingting, Shanghai 200245 (CN); LI, Hao, Shanghai 200245 (CN); LIU, Xun, Shanghai 200245 (CN); FU, Yayuan, Shanghai 200245 (CN)
(74) Representative: Gevers & Orès
(86) International application number: PCT/CN2018/122534
(87) International publication number: WO 2019/120269

(57) **Abstract**

Disclosed are a lymphocyte-activation gene 3 (LAG-3) antibody pharmaceutical composition and the use thereof. The pharmaceutical composition comprises a LAG-3 antibody or an antigen-binding fragment thereof in an acetate buffer or in a histidine salt buffer. The pharmaceutical composition may also comprise saccharide, non-ionic surfactant and other excipients.

Sequence listing
<110>  JIANGSU HENGRUI MEDICINE CO., LTD.; SHANGHAI HENGRUI PHARMACEUTICAL
CO., LTD.

<120>  LAG-3 ANTIBODY PHARMACEUTICAL COMPOSITION AND USE THEREOF

<160>  43

<170>  SIPOSequenceListing 1.0

<210>  1
<211>  442
<212>  PRT
<213>  artificial sequence

<220>
<221>  Peptide
<223>  LAG-3 extracellular domain with a Flag tag: LAG-3-Flag

<400>  1
Met Trp Glu Ala Gln Phe Leu Gly Leu Leu Phe Leu Gln Pro Leu Trp
1               5                   10                  15
Val Ala Pro Val Lys Pro Leu Gln Pro Gly Ala Glu Val Pro Val Val
            20                  25                  30
Trp Ala Gln Glu Gly Ala Pro Ala Gln Leu Pro Cys Ser Pro Thr Ile
        35                  40                  45
Pro Leu Gln Asp Leu Ser Leu Leu Arg Arg Ala Gly Val Thr Trp Gln
    50                  55                  60
His Gln Pro Asp Ser Gly Pro Pro Ala Ala Ala Pro Gly His Pro Leu
65                  70                  75                  80
Ala Pro Gly Pro His Pro Ala Ala Pro Ser Ser Trp Gly Pro Arg Pro
                85                  90                  95
Arg Arg Tyr Thr Val Leu Ser Val Gly Pro Gly Gly Leu Arg Ser Gly
            100                 105                 110
Arg Leu Pro Leu Gln Pro Arg Val Gln Leu Asp Glu Arg Gly Arg Gln
        115                 120                 125
Arg Gly Asp Phe Ser Leu Trp Leu Arg Pro Ala Arg Arg Ala Asp Ala
    130                 135                 140
Gly Glu Tyr Arg Ala Ala Val His Leu Arg Asp Arg Ala Leu Ser Cys
145                 150                 155                 160
Arg Leu Arg Leu Arg Leu Gly Gln Ala Ser Met Thr Ala Ser Pro Pro
                165                 170                 175
Gly Ser Leu Arg Ala Ser Asp Trp Val Ile Leu Asn Cys Ser Phe Ser
            180                 185                 190
Arg Pro Asp Arg Pro Ala Ser Val His Trp Phe Arg Asn Arg Gly Gln
        195                 200                 205
Gly Arg Val Pro Val Arg Glu Ser Pro His His His Leu Ala Glu Ser
    210                 215                 220
Phe Leu Phe Leu Pro Gln Val Ser Pro Met Asp Ser Gly Pro Trp Gly
225                 230                 235                 240
Cys Ile Leu Thr Tyr Arg Asp Gly Phe Asn Val Ser Ile Met Tyr Asn
                245                 250                 255
Leu Thr Val Leu Gly Leu Glu Pro Pro Thr Pro Leu Thr Val Tyr Ala
            260                 265                 270
Gly Ala Gly Ser Arg Val Gly Leu Pro Cys Arg Leu Pro Ala Gly Val
        275                 280                 285
Gly Thr Arg Ser Phe Leu Thr Ala Lys Trp Thr Pro Pro Gly Gly Gly
    290                 295                 300
Pro Asp Leu Leu Val Thr Gly Asp Asn Gly Asp Phe Thr Leu Arg Leu
305                 310                 315                 320
Glu Asp Val Ser Gln Ala Gln Ala Gly Thr Tyr Thr Cys His Ile His
                325                 330                 335

Leu Gln Glu Gln Gln Leu Asn Ala Thr Val Thr Leu Ala Ile Ile Thr
                340                 345                 350
Val Thr Pro Lys Ser Phe Gly Ser Pro Gly Ser Leu Gly Lys Leu Leu
        355                 360                 365
Cys Glu Val Thr Pro Val Ser Gly Gln Glu Arg Phe Val Trp Ser Ser
    370                 375                 380
Leu Asp Thr Pro Ser Gln Arg Ser Phe Ser Gly Pro Trp Leu Glu Ala
385                 390                 395                 400
Gln Glu Ala Gln Leu Leu Ser Gln Pro Trp Gln Cys Gln Leu Tyr Gln
                405                 410                 415
Gly Glu Arg Leu Leu Gly Ala Ala Val Tyr Phe Thr Glu Leu Ser Ser
                420                 425                 430
Pro Gly Asp Tyr Lys Asp Asp Asp Asp Lys
        435                 440

<210>  2
<211>  525
<212>  PRT
<213>  homo sapiens

<220>
<221>  Peptide
<223>  full-length LAG3


<400>  2
Met Trp Glu Ala Gln Phe Leu Gly Leu Leu Phe Leu Gln Pro Leu Trp
1               5                   10                  15
Val Ala Pro Val Lys Pro Leu Gln Pro Gly Ala Glu Val Pro Val Val
                20                  25                  30
Trp Ala Gln Glu Gly Ala Pro Ala Gln Leu Pro Cys Ser Pro Thr Ile
        35                  40                  45
Pro Leu Gln Asp Leu Ser Leu Leu Arg Arg Ala Gly Val Thr Trp Gln
    50                  55                  60
His Gln Pro Asp Ser Gly Pro Pro Ala Ala Ala Pro Gly His Pro Leu
65                  70                  75                  80
Ala Pro Gly Pro His Pro Ala Ala Pro Ser Ser Trp Gly Pro Arg Pro
                85                  90                  95
Arg Arg Tyr Thr Val Leu Ser Val Gly Pro Gly Gly Leu Arg Ser Gly
                100                 105                 110
Arg Leu Pro Leu Gln Pro Arg Val Gln Leu Asp Glu Arg Gly Arg Gln
        115                 120                 125
Arg Gly Asp Phe Ser Leu Trp Leu Arg Pro Ala Arg Arg Ala Asp Ala
    130                 135                 140
Gly Glu Tyr Arg Ala Ala Val His Leu Arg Asp Arg Ala Leu Ser Cys
145                 150                 155                 160
Arg Leu Arg Leu Arg Leu Gly Gln Ala Ser Met Thr Ala Ser Pro Pro
                165                 170                 175
Gly Ser Leu Arg Ala Ser Asp Trp Val Ile Leu Asn Cys Ser Phe Ser
                180                 185                 190
Arg Pro Asp Arg Pro Ala Ser Val His Trp Phe Arg Asn Arg Gly Gln
        195                 200                 205
Gly Arg Val Pro Val Arg Glu Ser Pro His His His Leu Ala Glu Ser
    210                 215                 220
Phe Leu Phe Leu Pro Gln Val Ser Pro Met Asp Ser Gly Pro Trp Gly
225                 230                 235                 240
Cys Ile Leu Thr Tyr Arg Asp Gly Phe Asn Val Ser Ile Met Tyr Asn
                245                 250                 255
Leu Thr Val Leu Gly Leu Glu Pro Pro Thr Pro Leu Thr Val Tyr Ala
                260                 265                 270
Gly Ala Gly Ser Arg Val Gly Leu Pro Cys Arg Leu Pro Ala Gly Val
        275                 280                 285
Gly Thr Arg Ser Phe Leu Thr Ala Lys Trp Thr Pro Pro Gly Gly Gly

290                 295                 300
Pro Asp Leu Leu Val Thr Gly Asp Asn Gly Asp Phe Thr Leu Arg Leu
305                 310                 315                 320
Glu Asp Val Ser Gln Ala Gln Ala Gly Thr Tyr Thr Cys His Ile His
                325                 330                 335
Leu Gln Glu Gln Gln Leu Asn Ala Thr Val Thr Leu Ala Ile Ile Thr
            340                 345                 350
Val Thr Pro Lys Ser Phe Gly Ser Pro Gly Ser Leu Gly Lys Leu Leu
        355                 360                 365
Cys Glu Val Thr Pro Val Ser Gly Gln Glu Arg Phe Val Trp Ser Ser
    370                 375                 380
Leu Asp Thr Pro Ser Gln Arg Ser Phe Ser Gly Pro Trp Leu Glu Ala
385                 390                 395                 400
Gln Glu Ala Gln Leu Leu Ser Gln Pro Trp Gln Cys Gln Leu Tyr Gln
                405                 410                 415
Gly Glu Arg Leu Leu Gly Ala Ala Val Tyr Phe Thr Glu Leu Ser Ser
            420                 425                 430
Pro Gly Ala Gln Arg Ser Gly Arg Ala Pro Gly Ala Leu Pro Ala Gly
        435                 440                 445
His Leu Leu Leu Phe Leu Ile Leu Gly Val Leu Ser Leu Leu Leu Leu
    450                 455                 460
Val Thr Gly Ala Phe Gly Phe His Leu Trp Arg Arg Gln Trp Arg Pro
465                 470                 475                 480
Arg Arg Phe Ser Ala Leu Glu Gln Gly Ile His Pro Pro Gln Ala Gln
                485                 490                 495
Ser Lys Ile Glu Glu Leu Glu Gln Glu Pro Glu Pro Glu Pro Glu Pro
            500                 505                 510
Glu Pro Glu Pro Glu Pro Glu Pro Glu Pro Glu Gln Leu
        515                 520                 525

<210>  3
<211>  675
<212>  PRT
<213>  artificial sequence

<220>
<221>  Peptide
<223>  Fusion protein of LAG-3 extracellular region and hIgG1 Fc


<400>  3
Met Trp Glu Ala Gln Phe Leu Gly Leu Leu Phe Leu Gln Pro Leu Trp
1               5                   10                  15
Val Ala Pro Val Lys Pro Leu Gln Pro Gly Ala Glu Val Pro Val Val
            20                  25                  30
Trp Ala Gln Glu Gly Ala Pro Ala Gln Leu Pro Cys Ser Pro Thr Ile
        35                  40                  45
Pro Leu Gln Asp Leu Ser Leu Leu Arg Arg Ala Gly Val Thr Trp Gln
    50                  55                  60
His Gln Pro Asp Ser Gly Pro Pro Ala Ala Ala Pro Gly His Pro Leu
65                  70                  75                  80
Ala Pro Gly Pro His Pro Ala Ala Pro Ser Ser Trp Gly Pro Arg Pro
                85                  90                  95
Arg Arg Tyr Thr Val Leu Ser Val Gly Pro Gly Gly Leu Arg Ser Gly
            100                 105                 110
Arg Leu Pro Leu Gln Pro Arg Val Gln Leu Asp Glu Arg Gly Arg Gln
        115                 120                 125
Arg Gly Asp Phe Ser Leu Trp Leu Arg Pro Ala Arg Arg Ala Asp Ala
    130                 135                 140
Gly Glu Tyr Arg Ala Ala Val His Leu Arg Asp Arg Ala Leu Ser Cys
145                 150                 155                 160
Arg Leu Arg Leu Arg Leu Gly Gln Ala Ser Met Thr Ala Ser Pro Pro
                165                 170                 175

Gly Ser Leu Arg Ala Ser Asp Trp Val Ile Leu Asn Cys Ser Phe Ser
                180                 185                 190
Arg Pro Asp Arg Pro Ala Ser Val His Trp Phe Arg Asn Arg Gly Gln
            195                 200                 205
Gly Arg Val Pro Val Arg Glu Ser Pro His His His Leu Ala Glu Ser
    210                 215                 220
Phe Leu Phe Leu Pro Gln Val Ser Pro Met Asp Ser Gly Pro Trp Gly
225                 230                 235                 240
Cys Ile Leu Thr Tyr Arg Asp Gly Phe Asn Val Ser Ile Met Tyr Asn
                245                 250                 255
Leu Thr Val Leu Gly Leu Glu Pro Pro Thr Pro Leu Thr Val Tyr Ala
                260                 265                 270
Gly Ala Gly Ser Arg Val Gly Leu Pro Cys Arg Leu Pro Ala Gly Val
            275                 280                 285
Gly Thr Arg Ser Phe Leu Thr Ala Lys Trp Thr Pro Pro Gly Gly Gly
    290                 295                 300
Pro Asp Leu Leu Val Thr Gly Asp Asn Gly Asp Phe Thr Leu Arg Leu
305                 310                 315                 320
Glu Asp Val Ser Gln Ala Gln Ala Gly Thr Tyr Thr Cys His Ile His
                325                 330                 335
Leu Gln Glu Gln Gln Leu Asn Ala Thr Val Thr Leu Ala Ile Ile Thr
                340                 345                 350
Val Thr Pro Lys Ser Phe Gly Ser Pro Gly Ser Leu Gly Lys Leu Leu
            355                 360                 365
Cys Glu Val Thr Pro Val Ser Gly Gln Glu Arg Phe Val Trp Ser Ser
    370                 375                 380
Leu Asp Thr Pro Ser Gln Arg Ser Phe Ser Gly Pro Trp Leu Glu Ala
385                 390                 395                 400
Gln Glu Ala Gln Leu Leu Ser Gln Pro Trp Gln Cys Gln Leu Tyr Gln
                405                 410                 415
Gly Glu Arg Leu Leu Gly Ala Ala Val Tyr Phe Thr Glu Leu Ser Ser
                420                 425                 430
Pro Gly Asp Asp Asp Asp Lys Gly Ser Gly Ser Gly Glu Pro Lys Ser
            435                 440                 445
Cys Asp Lys Thr His Thr Cys Pro Pro Cys Pro Ala Pro Glu Leu Leu
    450                 455                 460
Gly Gly Pro Ser Val Phe Leu Phe Pro Pro Lys Pro Lys Asp Thr Leu
465                 470                 475                 480
Met Ile Ser Arg Thr Pro Glu Val Thr Cys Val Val Val Asp Val Ser
                485                 490                 495
His Glu Asp Pro Glu Val Lys Phe Asn Trp Tyr Val Asp Gly Val Glu
                500                 505                 510
Val His Asn Ala Lys Thr Lys Pro Arg Glu Glu Gln Tyr Asn Ser Thr
            515                 520                 525
Tyr Arg Val Val Ser Val Leu Thr Val Leu His Gln Asp Trp Leu Asn
    530                 535                 540
Gly Lys Glu Tyr Lys Cys Lys Val Ser Asn Lys Ala Leu Pro Ala Pro
545                 550                 555                 560
Ile Glu Lys Thr Ile Ser Lys Ala Lys Gly Gln Pro Arg Glu Pro Gln
                565                 570                 575
Val Tyr Thr Leu Pro Pro Ser Arg Glu Glu Met Thr Lys Asn Gln Val
                580                 585                 590
Ser Leu Thr Cys Leu Val Lys Gly Phe Tyr Pro Ser Asp Ile Ala Val
            595                 600                 605
Glu Trp Glu Ser Asn Gly Gln Pro Glu Asn Asn Tyr Lys Thr Thr Pro
    610                 615                 620
Pro Val Leu Asp Ser Asp Gly Ser Phe Phe Leu Tyr Ser Lys Leu Thr
625                 630                 635                 640
Val Asp Lys Ser Arg Trp Gln Gln Gly Asn Val Phe Ser Cys Ser Val
                645                 650                 655
Met His Glu Ala Leu His Asn His Tyr Thr Gln Lys Ser Leu Ser Leu
                660                 665                 670
Ser Pro Gly

        675

<210>  4
<211>  677
<212>  PRT
<213>  artificial sequence

<220>
<221>  Peptide
<223>  Fusion protein of LAG-3 extracellular region and mIgG2a Fc


<400>  4
Met Trp Glu Ala Gln Phe Leu Gly Leu Leu Phe Leu Gln Pro Leu Trp
1               5                   10                  15
Val Ala Pro Val Lys Pro Leu Gln Pro Gly Ala Glu Val Pro Val Val
            20                  25                  30
Trp Ala Gln Glu Gly Ala Pro Ala Gln Leu Pro Cys Ser Pro Thr Ile
        35                  40                  45
Pro Leu Gln Asp Leu Ser Leu Leu Arg Arg Ala Gly Val Thr Trp Gln
    50                  55                  60
His Gln Pro Asp Ser Gly Pro Pro Ala Ala Ala Pro Gly His Pro Leu
65                  70                  75                  80
Ala Pro Gly Pro His Pro Ala Ala Pro Ser Ser Trp Gly Pro Arg Pro
                85                  90                  95
Arg Arg Tyr Thr Val Leu Ser Val Gly Pro Gly Gly Leu Arg Ser Gly
            100                 105                 110
Arg Leu Pro Leu Gln Pro Arg Val Gln Leu Asp Glu Arg Gly Arg Gln
        115                 120                 125
Arg Gly Asp Phe Ser Leu Trp Leu Arg Pro Ala Arg Arg Ala Asp Ala
    130                 135                 140
Gly Glu Tyr Arg Ala Ala Val His Leu Arg Asp Arg Ala Leu Ser Cys
145                 150                 155                 160
Arg Leu Arg Leu Arg Leu Gly Gln Ala Ser Met Thr Ala Ser Pro Pro
                165                 170                 175
Gly Ser Leu Arg Ala Ser Asp Trp Val Ile Leu Asn Cys Ser Phe Ser
            180                 185                 190
Arg Pro Asp Arg Pro Ala Ser Val His Trp Phe Arg Asn Arg Gly Gln
        195                 200                 205
Gly Arg Val Pro Val Arg Glu Ser Pro His His His Leu Ala Glu Ser
    210                 215                 220
Phe Leu Phe Leu Pro Gln Val Ser Pro Met Asp Ser Gly Pro Trp Gly
225                 230                 235                 240
Cys Ile Leu Thr Tyr Arg Asp Gly Phe Asn Val Ser Ile Met Tyr Asn
                245                 250                 255
Leu Thr Val Leu Gly Leu Glu Pro Pro Thr Pro Leu Thr Val Tyr Ala
            260                 265                 270
Gly Ala Gly Ser Arg Val Gly Leu Pro Cys Arg Leu Pro Ala Gly Val
        275                 280                 285
Gly Thr Arg Ser Phe Leu Thr Ala Lys Trp Thr Pro Pro Gly Gly Gly
    290                 295                 300
Pro Asp Leu Leu Val Thr Gly Asp Asn Gly Asp Phe Thr Leu Arg Leu
305                 310                 315                 320
Glu Asp Val Ser Gln Ala Gln Ala Gly Thr Tyr Thr Cys His Ile His
                325                 330                 335
Leu Gln Glu Gln Gln Leu Asn Ala Thr Val Thr Leu Ala Ile Ile Thr
            340                 345                 350
Val Thr Pro Lys Ser Phe Gly Ser Pro Gly Ser Leu Gly Lys Leu Leu
        355                 360                 365
Cys Glu Val Thr Pro Val Ser Gly Gln Glu Arg Phe Val Trp Ser Ser
    370                 375                 380
Leu Asp Thr Pro Ser Gln Arg Ser Phe Ser Gly Pro Trp Leu Glu Ala
385                 390                 395                 400

Gln Glu Ala Gln Leu Leu Ser Gln Pro Trp Gln Cys Gln Leu Tyr Gln
                405                 410                 415
Gly Glu Arg Leu Leu Gly Ala Ala Val Tyr Phe Thr Glu Leu Ser Ser
            420                 425                 430
Pro Gly Asp Asp Asp Asp Lys Gly Ser Gly Ser Gly Glu Pro Arg Gly
        435                 440                 445
Pro Thr Ile Lys Pro Cys Pro Pro Cys Lys Cys Pro Ala Pro Asn Leu
    450                 455                 460
Leu Gly Gly Pro Ser Val Phe Ile Phe Pro Pro Lys Ile Lys Asp Val
465                 470                 475                 480
Leu Met Ile Ser Leu Ser Pro Ile Val Thr Cys Val Val Val Asp Val
                485                 490                 495
Ser Glu Asp Asp Pro Asp Val Gln Ile Ser Trp Phe Val Asn Asn Val
            500                 505                 510
Glu Val His Thr Ala Gln Thr Gln Thr His Arg Glu Asp Tyr Asn Ser
        515                 520                 525
Thr Leu Arg Val Val Ser Ala Leu Pro Ile Gln His Gln Asp Trp Met
    530                 535                 540
Ser Gly Lys Glu Phe Lys Cys Lys Val Asn Asn Lys Asp Leu Pro Ala
545                 550                 555                 560
Pro Ile Glu Arg Thr Ile Ser Lys Pro Lys Gly Ser Val Arg Ala Pro
                565                 570                 575
Gln Val Tyr Val Leu Pro Pro Pro Glu Glu Glu Met Thr Lys Lys Gln
            580                 585                 590
Val Thr Leu Thr Cys Met Val Thr Asp Phe Met Pro Glu Asp Ile Tyr
        595                 600                 605
Val Glu Trp Thr Asn Asn Gly Lys Thr Glu Leu Asn Tyr Lys Asn Thr
    610                 615                 620
Glu Pro Val Leu Asp Ser Asp Gly Ser Tyr Phe Met Tyr Ser Lys Leu
625                 630                 635                 640
Arg Val Glu Lys Lys Asn Trp Val Glu Arg Asn Ser Tyr Ser Cys Ser
                645                 650                 655
Val Val His Glu Gly Leu His Asn His His Thr Thr Lys Ser Phe Ser
            660                 665                 670
Arg Thr Pro Gly Lys
        675

<210>  5
<211>  124
<212>  PRT
<213>  Mus musculus

<220>
<221>  Domain
<223>  mAb229-VH


<400>  5
Gln Ile Gln Leu Val Gln Ser Gly Pro Glu Leu Lys Lys Pro Gly Glu
1               5                   10                  15
Thr Val Lys Ile Ser Cys Lys Ala Ser Gly Tyr Thr Phe Thr Thr Ser
            20                  25                  30
Gly Met Ser Trp Val Lys Gln Ala Pro Gly Lys Gly Leu Lys Trp Met
        35                  40                  45
Gly Trp Ile Asn Thr Tyr Ser Gly Val Pro Thr Tyr Ala Asp Asp Phe
    50                  55                  60
Lys Gly Arg Phe Ala Phe Ser Leu Glu Thr Ser Ala Ser Thr Ala Tyr
65                  70                  75                  80
Leu Gln Ile Asn Asn Leu Lys Asn Glu Asp Thr Ala Thr Tyr Phe Cys
                85                  90                  95
Ala Arg Asp Asn Tyr Asp Ala Arg Asp Val Tyr Tyr Tyr Ala Met Asp
            100                 105                 110
Tyr Trp Gly Gln Gly Thr Ser Val Thr Val Ser Ser

115                 120

<210>  6
<211>  107
<212>  PRT
<213>  Mus musculus

<220>
<221>  Domain
<223>  mAb229-VL


<400>  6
Asp Ile Gln Met Thr Gln Ser Pro Ala Ser Leu Ser Val Ser Val Gly
1               5                   10                  15
Glu Thr Val Thr Ile Thr Cys Arg Ala Ser Glu Asn Ile Tyr Ser Asn
            20                  25                  30
Leu Ala Trp Tyr Gln Gln Lys Gln Gly Lys Ser Pro Gln Leu Leu Val
        35                  40                  45
Tyr Ala Ala Thr Asn Leu Ala Asp Gly Val Pro Ser Arg Phe Ser Gly
    50                  55                  60
Ser Gly Ser Gly Thr Gln Tyr Ser Leu Lys Ile Asn Ser Leu Gln Ser
65                  70                  75                  80
Glu Asp Phe Gly Ser Tyr Tyr Cys Gln His Phe Trp Ile Thr Pro Trp
                85                  90                  95
Thr Phe Gly Gly Gly Thr Lys Leu Glu Ile Lys
            100                 105

<210>  7
<211>  120
<212>  PRT
<213>  Mus musculus

<220>
<221>  Domain
<223>  mAb303-VH


<400>  7
Glu Val Gln Leu Gln Gln Ser Gly Pro Val Leu Val Lys Pro Gly Ala
1               5                   10                  15
Ser Val Lys Met Ser Cys Lys Ala Ser Gly Tyr Thr Leu Thr Asp Tyr
            20                  25                  30
Tyr Met Asn Trp Val Lys Gln Ser His Gly Lys Ser Leu Glu Trp Ile
        35                  40                  45
Gly Val Ile Asn Pro Tyr Asn Gly Asp Thr Ala Tyr Asn Gln Lys Phe
    50                  55                  60
Lys Gly Lys Ala Thr Leu Thr Val Asp Lys Ser Ser Asn Thr Ala Tyr
65                  70                  75                  80
Met Glu Ile Asn Ser Leu Thr Ser Glu Asp Ser Ala Val Tyr Tyr Cys
                85                  90                  95
Thr Arg Asp Asp Gly Tyr Tyr Asp Tyr Tyr Phe Asp Val Trp Gly Thr
            100                 105                 110
Gly Thr Thr Val Thr Val Ser Ser
        115                 120

<210>  8
<211>  107
<212>  PRT
<213>  Mus musculus

<220>
<221>  Domain

<223>  mAb303-VL

<400>  8
Asp Ile Gln Met Thr Gln Ser Pro Ser Ser Leu Ser Ala Ser Leu Gly
1               5                   10                  15
Glu Arg Val Ile Leu Thr Cys Arg Ala Ser Gln Asp Ile Gly Ser Arg
            20                  25                  30
Leu Asn Trp Leu Gln Gln Gly Pro Asp Gly Thr Phe Lys Arg Leu Ile
        35                  40                  45
Tyr Ala Thr Ser Thr Leu Asp Ser Gly Val Pro Lys Arg Phe Ser Gly
    50                  55                  60
Ser Arg Ser Gly Ser Asp Phe Ser Leu Thr Ile Ser Ser Leu Glu Ser
65                  70                  75                  80
Glu Asp Phe Val Asp Tyr Tyr Cys Leu Gln Leu Ala Ser Ser Pro Pro
                85                  90                  95
Thr Phe Gly Gly Gly Thr Lys Leu Glu Ile Lys
            100                 105

<210>  9
<211>  5
<212>  PRT
<213>  Mus musculus

<220>
<221>  Domain
<223>  mAb229 HCDR1

<400>  9
Thr Ser Gly Met Ser
1               5

<210>  10
<211>  17
<212>  PRT
<213>  Mus musculus

<220>
<221>  Domain
<223>  mAb229 HCDR2

<400>  10
Trp Ile Asn Thr Tyr Ser Gly Val Pro Thr Tyr Ala Asp Asp Phe Lys
1               5                   10                  15
Gly

<210>  11
<211>  15
<212>  PRT
<213>  Mus musculus

<220>
<221>  Domain
<223>  mAb229 HCDR3

<400>  11
Asp Asn Tyr Asp Ala Arg Asp Val Tyr Tyr Tyr Ala Met Asp Tyr
1               5                   10                  15

<210>  12
<211>  5
<212>  PRT
<213>  Mus musculus

<220>
<221>  Domain
<223>  mAb303 HCDR1


<400>  12
Asp Tyr Tyr Met Asn
1               5

<210>  13
<211>  17
<212>  PRT
<213>  Mus musculus

<220>
<221>  Domain
<223>  mAb303 HCDR2


<400>  13
Val Ile Asn Pro Tyr Asn Gly Asp Thr Ala Tyr Asn Gln Lys Phe Lys
1               5                   10                  15
Gly


<210>  14
<211>  11
<212>  PRT
<213>  Mus musculus

<220>
<221>  Domain
<223>  mAb303 HCD3


<400>  14
Asp Asp Gly Tyr Tyr Asp Tyr Tyr Phe Asp Val
1               5                   10

<210>  15
<211>  11
<212>  PRT
<213>  Mus musculus

<220>
<221>  Domain
<223>  mAb229 LCDR1


<400>  15
Arg Ala Ser Glu Asn Ile Tyr Ser Asn Leu Ala
1               5                   10

<210>  16
<211>  7
<212>  PRT
<213>  Mus musculus

<220>
<221>  Domain
<223>  mAb229 LCDR2


<400>  16
Ala Ala Thr Asn Leu Ala Asp
1               5

<210>  17
<211>  9
<212>  PRT
<213>  Mus musculus

<220>
<221>  Domain
<223>  mAb229 LCDR3


<400>  17
Gln His Phe Trp Ile Thr Pro Trp Thr
1               5

<210>  18
<211>  11
<212>  PRT
<213>  Mus musculus

<220>
<221>  Domain
<223>  mAb303 LCDR1


<400>  18
Arg Ala Ser Gln Asp Ile Gly Ser Arg Leu Asn
1               5                   10

<210>  19
<211>  7
<212>  PRT
<213>  Mus musculus

<220>
<221>  Domain
<223>  mAb303 LCDR2


<400>  19
Ala Thr Ser Thr Leu Asp Ser
1               5

<210>  20
<211>  9
<212>  PRT
<213>  Mus musculus

<220>
<221>  Domain
<223>  mAb303 LCDR3


<400>  20
Leu Gln Leu Ala Ser Ser Pro Pro Thr

1               5

<210>  21
<211>  124
<212>  PRT
<213>  artificial sequence

<220>
<221>  Domain
<223>  Hu229VH.1

<400>  21
Gln Val Gln Leu Val Gln Ser Gly Ser Glu Leu Lys Lys Pro Gly Ala
1               5                   10                  15
Ser Val Lys Val Ser Cys Lys Ala Ser Gly Tyr Thr Phe Thr Thr Ser
            20                  25                  30
Gly Met Ser Trp Val Arg Gln Ala Pro Gly Gln Gly Leu Glu Trp Met
        35                  40                  45
Gly Trp Ile Asn Thr Tyr Ser Gly Val Pro Thr Tyr Ala Asp Asp Phe
    50                  55                  60
Lys Gly Arg Phe Val Phe Ser Leu Asp Thr Ser Val Ser Thr Ala Tyr
65                  70                  75                  80
Leu Gln Ile Ser Ser Leu Lys Ala Glu Asp Thr Ala Val Tyr Tyr Cys
                85                  90                  95
Ala Arg Asp Asn Tyr Asp Ala Arg Asp Val Tyr Tyr Tyr Ala Met Asp
            100                 105                 110
Tyr Trp Gly Gln Gly Thr Thr Val Thr Val Ser Ser
        115                 120

<210>  22
<211>  107
<212>  PRT
<213>  artificial sequence

<220>
<221>  Domain
<223>  Hu229VL.1

<400>  22
Asp Ile Gln Met Thr Gln Ser Pro Ser Ser Leu Ser Ala Ser Val Gly
1               5                   10                  15
Asp Arg Val Thr Ile Thr Cys Arg Ala Ser Glu Asn Ile Tyr Ser Asn
            20                  25                  30
Leu Ala Trp Tyr Gln Gln Lys Pro Gly Lys Ala Pro Lys Leu Leu Ile
        35                  40                  45
Tyr Ala Ala Thr Asn Leu Ala Asp Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Thr Tyr Tyr Cys Gln His Phe Trp Ile Thr Pro Trp
                85                  90                  95
Thr Phe Gly Gly Gly Thr Lys Val Glu Ile Lys
            100                 105

<210>  23
<211>  124
<212>  PRT
<213>  artificial sequence

<220>
<221>  Domain

<223>  Hu229VH.1A

<400>  23
Gln Val Gln Leu Val Gln Ser Gly Ser Glu Leu Lys Lys Pro Gly Ala
1               5                   10                  15
Ser Val Lys Val Ser Cys Lys Ala Ser Gly Tyr Thr Phe Thr Thr Ser
            20                  25                  30
Gly Met Ser Trp Val Arg Gln Ala Pro Gly Gln Gly Leu Lys Trp Met
        35                  40                  45
Gly Trp Ile Asn Thr Tyr Ser Gly Val Pro Thr Tyr Ala Asp Asp Phe
    50                  55                  60
Lys Gly Arg Phe Val Phe Ser Leu Asp Thr Ser Val Ser Thr Ala Tyr
65                  70                  75                  80
Leu Gln Ile Ser Ser Leu Lys Ala Glu Asp Thr Ala Val Tyr Tyr Cys
                85                  90                  95
Ala Arg Asp Asn Tyr Asp Ala Arg Asp Val Tyr Tyr Tyr Ala Met Asp
            100                 105                 110
Tyr Trp Gly Gln Gly Thr Thr Val Thr Val Ser Ser
        115                 120

<210>  24
<211>  124
<212>  PRT
<213>  artificial sequence

<220>
<221>  Domain
<223>  Hu229VH.1B

<400>  24
Gln Val Gln Leu Val Gln Ser Gly Ser Glu Leu Lys Lys Pro Gly Ala
1               5                   10                  15
Ser Val Lys Val Ser Cys Lys Ala Ser Gly Tyr Thr Phe Thr Thr Ser
            20                  25                  30
Gly Met Ser Trp Val Lys Gln Ala Pro Gly Gln Gly Leu Lys Trp Met
        35                  40                  45
Gly Trp Ile Asn Thr Tyr Ser Gly Val Pro Thr Tyr Ala Asp Asp Phe
    50                  55                  60
Lys Gly Arg Phe Val Phe Ser Leu Asp Thr Ser Val Ser Thr Ala Tyr
65                  70                  75                  80
Leu Gln Ile Ser Ser Leu Lys Ala Glu Asp Thr Ala Thr Tyr Tyr Cys
                85                  90                  95
Ala Arg Asp Asn Tyr Asp Ala Arg Asp Val Tyr Tyr Tyr Ala Met Asp
            100                 105                 110
Tyr Trp Gly Gln Gly Thr Thr Val Thr Val Ser Ser
        115                 120

<210>  25
<211>  124
<212>  PRT
<213>  artificial sequence

<220>
<221>  Domain
<223>  Hu229VH.1C

<400>  25
Gln Val Gln Leu Val Gln Ser Gly Ser Glu Leu Lys Lys Pro Gly Ala
1               5                   10                  15
Ser Val Lys Val Ser Cys Lys Ala Ser Gly Tyr Thr Phe Thr Thr Ser

20                  25                  30
Gly Met Ser Trp Val Lys Gln Ala Pro Gly Gln Gly Leu Lys Trp Met
        35                  40                  45
Gly Trp Ile Asn Thr Tyr Ser Gly Val Pro Thr Tyr Ala Asp Asp Phe
    50                  55                  60
Lys Gly Arg Phe Val Phe Ser Leu Asp Thr Ser Val Ser Thr Ala Tyr
65                  70                  75                  80
Leu Gln Ile Ser Ser Leu Lys Ala Glu Asp Thr Ala Thr Tyr Phe Cys
                85                  90                  95
Ala Arg Asp Asn Tyr Asp Ala Arg Asp Val Tyr Tyr Tyr Ala Met Asp
            100                 105                 110
Tyr Trp Gly Gln Gly Thr Thr Val Thr Val Ser Ser
        115                 120

<210>  26
<211>  107
<212>  PRT
<213>  artificial sequence

<220>
<221>  Domain
<223>  Hu229VL.1A


<400>  26
Asp Ile Gln Met Thr Gln Ser Pro Ser Ser Leu Ser Ala Ser Val Gly
1               5                   10                  15
Asp Arg Val Thr Ile Thr Cys Arg Ala Ser Glu Asn Ile Tyr Ser Asn
            20                  25                  30
Leu Ala Trp Tyr Gln Gln Lys Pro Gly Lys Ala Pro Lys Leu Leu Val
        35                  40                  45
Tyr Ala Ala Thr Asn Leu Ala Asp Gly Val Pro Ser Arg Phe Ser Gly
    50                  55                  60
Ser Gly Ser Gly Thr Asp Tyr Thr Leu Thr Ile Ser Ser Leu Gln Pro
65                  70                  75                  80
Glu Asp Phe Ala Thr Tyr Tyr Cys Gln His Phe Trp Ile Thr Pro Trp
                85                  90                  95
Thr Phe Gly Gly Gly Thr Lys Val Glu Ile Lys
            100                 105

<210>  27
<211>  107
<212>  PRT
<213>  artificial sequence

<220>
<221>  Domain
<223>  Hu229VL.1B


<400>  27
Asp Ile Gln Met Thr Gln Ser Pro Ser Ser Leu Ser Ala Ser Val Gly
1               5                   10                  15
Asp Arg Val Thr Ile Thr Cys Arg Ala Ser Glu Asn Ile Tyr Ser Asn
            20                  25                  30
Leu Ala Trp Tyr Gln Gln Lys Pro Gly Lys Ala Pro Lys Leu Leu Val
        35                  40                  45
Tyr Ala Ala Thr Asn Leu Ala Asp Gly Val Pro Ser Arg Phe Ser Gly
    50                  55                  60
Ser Gly Ser Gly Thr Gln Tyr Thr Leu Thr Ile Ser Ser Leu Gln Pro
65                  70                  75                  80
Glu Asp Phe Ala Thr Tyr Tyr Cys Gln His Phe Trp Ile Thr Pro Trp
                85                  90                  95

Thr Phe Gly Gly Gly Thr Lys Val Glu Ile Lys
            100                 105

<210>  28
<211>  107
<212>  PRT
<213>  artificial sequence

<220>
<221>  Domain
<223>  Hu229VL.1C


<400>  28
Asp Ile Gln Met Thr Gln Ser Pro Ser Ser Leu Ser Ala Ser Val Gly
1               5                   10                  15
Asp Arg Val Thr Ile Thr Cys Arg Ala Ser Glu Asn Ile Tyr Ser Asn
            20                  25                  30
Leu Ala Trp Tyr Gln Gln Lys Pro Gly Lys Ser Pro Lys Leu Leu Val
        35                  40                  45
Tyr Ala Ala Thr Asn Leu Ala Asp Gly Val Pro Ser Arg Phe Ser Gly
    50                  55                  60
Ser Gly Ser Gly Thr Gln Tyr Thr Leu Thr Ile Ser Ser Leu Gln Pro
65                  70                  75                  80
Glu Asp Phe Ala Thr Tyr Tyr Cys Gln His Phe Trp Ile Thr Pro Trp
                85                  90                  95
Thr Phe Gly Gly Gly Thr Lys Val Glu Ile Lys
            100                 105

<210>  29
<211>  120
<212>  PRT
<213>  artificial sequence

<220>
<221>  Domain
<223>  Hu303_VH.1


<400>  29
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Asp Tyr
            20                  25                  30
Tyr Met Asn Trp Val Arg Gln Ala Pro Gly Gln Arg Leu Glu Trp Met
        35                  40                  45
Gly Val Ile Asn Pro Tyr Asn Gly Asp Thr Ala Tyr Asn Gln Lys Phe
    50                  55                  60
Lys Gly Arg Val Thr Ile Thr Arg Asp Thr Ser Ala Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Asp Asp Gly Tyr Tyr Asp Tyr Tyr Phe Asp Val Trp Gly Gln
            100                 105                 110
Gly Thr Thr Val Thr Val Ser Ser
        115                 120

<210>  30
<211>  107
<212>  PRT
<213>  artificial sequence

<220>

<221>  Domain
<223>  Hu303_VL.1

<400>  30
Asp Ile Gln Met Thr Gln Ser Pro Ser Ser Leu Ser Ala Ser Val Gly
1               5                   10                  15
Asp Arg Val Thr Ile Thr Cys Arg Ala Ser Gln Asp Ile Gly Ser Arg
            20                  25                  30
Leu Asn Trp Tyr Gln Gln Lys Pro Gly Lys Ala Pro Lys Leu Leu Ile
        35                  40                  45
Tyr Ala Thr Ser Thr Leu Asp Ser Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Thr Tyr Tyr Cys Leu Gln Leu Ala Ser Ser Pro Pro
                85                  90                  95
Thr Phe Gly Gly Gly Thr Lys Val Glu Ile Lys
            100                 105

<210>  31
<211>  120
<212>  PRT
<213>  artificial sequence

<220>
<221>  Domain
<223>  Hu303_VH.1A

<400>  31
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Asp Tyr
            20                  25                  30
Tyr Met Asn Trp Val Arg Gln Ala Pro Gly Gln Arg Leu Glu Trp Met
        35                  40                  45
Gly Val Ile Asn Pro Tyr Asn Gly Asp Thr Ala Tyr Asn Gln Lys Phe
    50                  55                  60
Lys Gly Arg Val Thr Ile Thr Val Asp Lys Ser Ala Ser Thr Ala Tyr
65                  70                  75                  80
Met Glu Leu Ser Ser Leu Arg Ser Glu Asp Thr Ala Val Tyr Tyr Cys
                85                  90                  95
Thr Arg Asp Asp Gly Tyr Tyr Asp Tyr Tyr Phe Asp Val Trp Gly Gln
            100                 105                 110
Gly Thr Thr Val Thr Val Ser Ser
        115                 120

<210>  32
<211>  120
<212>  PRT
<213>  artificial sequence

<220>
<221>  Domain
<223>  Hu303_VH.1B

<400>  32
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Leu Thr Asp Tyr
            20                  25                  30

Tyr Met Asn Trp Val Arg Gln Ala Pro Gly Gln Arg Leu Glu Trp Met
        35                  40                  45
Gly Val Ile Asn Pro Tyr Asn Gly Asp Thr Ala Tyr Asn Gln Lys Phe
    50                  55                  60
Lys Gly Arg Val Thr Ile Thr Val Asp Lys Ser Ala Ser Thr Ala Tyr
65                  70                  75                  80
Met Glu Leu Ser Ser Leu Arg Ser Glu Asp Thr Ala Val Tyr Tyr Cys
                85                  90                  95
Thr Arg Asp Asp Gly Tyr Tyr Asp Tyr Tyr Phe Asp Val Trp Gly Gln
            100                 105                 110
Gly Thr Thr Val Thr Val Ser Ser
        115                 120

<210> 33
<211> 120
<212> PRT
<213> artificial sequence

<220>
<221> Domain
<223> Hu303_VH.1C

<400> 33
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Leu Thr Asp Tyr
            20                  25                  30
Tyr Met Asn Trp Val Arg Gln Ala Pro Gly Gln Arg Leu Glu Trp Ile
        35                  40                  45
Gly Val Ile Asn Pro Tyr Asn Gly Asp Thr Ala Tyr Asn Gln Lys Phe
    50                  55                  60
Lys Gly Arg Ala Thr Leu Thr Val Asp Lys Ser Ala Ser Thr Ala Tyr
65                  70                  75                  80
Met Glu Leu Ser Ser Leu Arg Ser Glu Asp Thr Ala Val Tyr Tyr Cys
                85                  90                  95
Thr Arg Asp Asp Gly Tyr Tyr Asp Tyr Tyr Phe Asp Val Trp Gly Gln
            100                 105                 110
Gly Thr Thr Val Thr Val Ser Ser
        115                 120

<210> 34
<211> 107
<212> PRT
<213> artificial sequence

<220>
<221> Domain
<223> Hu303_VL.1A

<400> 34
Asp Ile Gln Met Thr Gln Ser Pro Ser Ser Leu Ser Ala Ser Val Gly
1               5                   10                  15
Asp Arg Val Thr Ile Thr Cys Arg Ala Ser Gln Asp Ile Gly Ser Arg
            20                  25                  30
Leu Asn Trp Tyr Gln Gln Lys Pro Gly Lys Ala Pro Lys Arg Leu Ile
        35                  40                  45
Tyr Ala Thr Ser Thr Leu Asp Ser Gly Val Pro Ser Arg Phe Ser Gly
    50                  55                  60
Ser Arg Ser Gly Thr Asp Phe Thr Leu Thr Ile Ser Ser Leu Gln Pro
65                  70                  75                  80
Glu Asp Phe Ala Thr Tyr Tyr Cys Leu Gln Leu Ala Ser Ser Pro Pro

85                      90                      95
Thr Phe Gly Gly Gly Thr Lys Val Glu Ile Lys
            100                 105

<210>  35
<211>  107
<212>  PRT
<213>  artificial sequence

<220>
<221>  Domain
<223>  Hu303_VL.1B


<400>  35
Asp Ile Gln Met Thr Gln Ser Pro Ser Ser Leu Ser Ala Ser Val Gly
1               5                   10                  15
Asp Arg Val Thr Ile Thr Cys Arg Ala Ser Gln Asp Ile Gly Ser Arg
            20                  25                  30
Leu Asn Trp Tyr Gln Gln Lys Pro Gly Lys Ala Pro Lys Arg Leu Ile
        35                  40                  45
Tyr Ala Thr Ser Thr Leu Asp Ser Gly Val Pro Lys Arg Phe Ser Gly
    50                  55                  60
Ser Arg Ser Gly Thr Asp Phe Thr Leu Thr Ile Ser Ser Leu Gln Pro
65                  70                  75                  80
Glu Asp Phe Ala Thr Tyr Tyr Cys Leu Gln Leu Ala Ser Ser Pro Pro
                85                  90                  95
Thr Phe Gly Gly Gly Thr Lys Val Glu Ile Lys
            100                 105

<210>  36
<211>  107
<212>  PRT
<213>  artificial sequence

<220>
<221>  Domain
<223>  Hu303_VL.1C


<400>  36
Asp Ile Gln Met Thr Gln Ser Pro Ser Ser Leu Ser Ala Ser Val Gly
1               5                   10                  15
Asp Arg Val Thr Ile Thr Cys Arg Ala Ser Gln Asp Ile Gly Ser Arg
            20                  25                  30
Leu Asn Trp Leu Gln Gln Lys Pro Gly Lys Ala Phe Lys Arg Leu Ile
        35                  40                  45
Tyr Ala Thr Ser Thr Leu Asp Ser Gly Val Pro Lys Arg Phe Ser Gly
    50                  55                  60
Ser Arg Ser Gly Thr Asp Phe Thr Leu Thr Ile Ser Ser Leu Gln Pro
65                  70                  75                  80
Glu Asp Phe Ala Thr Tyr Tyr Cys Leu Gln Leu Ala Ser Ser Pro Pro
                85                  90                  95
Thr Phe Gly Gly Gly Thr Lys Val Glu Ile Lys
            100                 105

<210>  37
<211>  107
<212>  PRT
<213>  artificial sequence

<220>
<221>  Domain

<223>  Hu303_VL.1D

<400>  37
Asp Ile Gln Met Thr Gln Ser Pro Ser Ser Leu Ser Ala Ser Val Gly
1               5                   10                  15
Asp Arg Val Thr Leu Thr Cys Arg Ala Ser Gln Asp Ile Gly Ser Arg
            20                  25                  30
Leu Asn Trp Leu Gln Gln Lys Pro Gly Gly Ala Phe Lys Arg Leu Ile
        35                  40                  45
Tyr Ala Thr Ser Thr Leu Asp Ser Gly Val Pro Lys Arg Phe Ser Gly
    50                  55                  60
Ser Arg Ser Gly Thr Asp Phe Thr Leu Thr Ile Ser Ser Leu Gln Pro
65                  70                  75                  80
Glu Asp Phe Ala Asp Tyr Tyr Cys Leu Gln Leu Ala Ser Ser Pro Pro
                85                  90                  95
Thr Phe Gly Gly Gly Thr Lys Val Glu Ile Lys
            100                 105

<210>  38
<211>  327
<212>  PRT
<213>  artificial sequence

<220>
<221>  Domain
<223>  Heavy chain constant region, S228P mutation.

<400>  38
Ala Ser Thr Lys Gly Pro Ser Val Phe Pro Leu Ala Pro Cys Ser Arg
1               5                   10                  15
Ser Thr Ser Glu Ser Thr Ala Ala Leu Gly Cys Leu Val Lys Asp Tyr
            20                  25                  30
Phe Pro Glu Pro Val Thr Val Ser Trp Asn Ser Gly Ala Leu Thr Ser
        35                  40                  45
Gly Val His Thr Phe Pro Ala Val Leu Gln Ser Ser Gly Leu Tyr Ser
    50                  55                  60
Leu Ser Ser Val Val Thr Val Pro Ser Ser Ser Leu Gly Thr Lys Thr
65                  70                  75                  80
Tyr Thr Cys Asn Val Asp His Lys Pro Ser Asn Thr Lys Val Asp Lys
                85                  90                  95
Arg Val Glu Ser Lys Tyr Gly Pro Pro Cys Pro Pro Cys Pro Ala Pro
            100                 105                 110
Glu Phe Leu Gly Gly Pro Ser Val Phe Leu Phe Pro Pro Lys Pro Lys
        115                 120                 125
Asp Thr Leu Met Ile Ser Arg Thr Pro Glu Val Thr Cys Val Val Val
    130                 135                 140
Asp Val Ser Gln Glu Asp Pro Glu Val Gln Phe Asn Trp Tyr Val Asp
145                 150                 155                 160
Gly Val Glu Val His Asn Ala Lys Thr Lys Pro Arg Glu Glu Gln Phe
                165                 170                 175
Asn Ser Thr Tyr Arg Val Val Ser Val Leu Thr Val Leu His Gln Asp
            180                 185                 190
Trp Leu Asn Gly Lys Glu Tyr Lys Cys Lys Val Ser Asn Lys Gly Leu
        195                 200                 205
Pro Ser Ser Ile Glu Lys Thr Ile Ser Lys Ala Lys Gly Gln Pro Arg
    210                 215                 220
Glu Pro Gln Val Tyr Thr Leu Pro Pro Ser Gln Glu Glu Met Thr Lys
225                 230                 235                 240
Asn Gln Val Ser Leu Thr Cys Leu Val Lys Gly Phe Tyr Pro Ser Asp
                245                 250                 255
Ile Ala Val Glu Trp Glu Ser Asn Gly Gln Pro Glu Asn Asn Tyr Lys

260                 265                 270
Thr Thr Pro Pro Val Leu Asp Ser Asp Gly Ser Phe Phe Leu Tyr Ser
        275                 280                 285
Arg Leu Thr Val Asp Lys Ser Arg Trp Gln Glu Gly Asn Val Phe Ser
    290                 295                 300
Cys Ser Val Met His Glu Ala Leu His Asn His Tyr Thr Gln Lys Ser
305                 310                 315                 320
Leu Ser Leu Ser Leu Gly Lys
                325

<210>  39
<211>  107
<212>  PRT
<213>  artificial sequence

<220>
<221>  Domain
<223>  kappa light chain constant region


<400>  39

<210>  40
<211>  451
<212>  PRT
<213>  artificial sequence

<220>
<221>  CHAIN
<223>  heavy chain amino acid sequence of Hu229-013


<400>  40
Gln Val Gln Leu Val Gln Ser Gly Ser Glu Leu Lys Lys Pro Gly Ala
1               5                   10                  15
Ser Val Lys Val Ser Cys Lys Ala Ser Gly Tyr Thr Phe Thr Thr Ser
            20                  25                  30
Gly Met Ser Trp Val Lys Gln Ala Pro Gly Gln Gly Leu Lys Trp Met
        35                  40                  45
Gly Trp Ile Asn Thr Tyr Ser Gly Val Pro Thr Tyr Ala Asp Asp Phe
    50                  55                  60
Lys Gly Arg Phe Val Phe Ser Leu Asp Thr Ser Val Ser Thr Ala Tyr
65                  70                  75                  80
Leu Gln Ile Ser Ser Leu Lys Ala Glu Asp Thr Ala Thr Tyr Tyr Cys
                85                  90                  95
Ala Arg Asp Asn Tyr Asp Ala Arg Asp Val Tyr Tyr Tyr Ala Met Asp
            100                 105                 110
Tyr Trp Gly Gln Gly Thr Thr Val Thr Val Ser Ser Ala Ser Thr Lys
        115                 120                 125

Gly Pro Ser Val Phe Pro Leu Ala Pro Cys Ser Arg Ser Thr Ser Glu
    130                 135                 140
Ser Thr Ala Ala Leu Gly Cys Leu Val Lys Asp Tyr Phe Pro Glu Pro
145                 150                 155                 160
Val Thr Val Ser Trp Asn Ser Gly Ala Leu Thr Ser Gly Val His Thr
                165                 170                 175
Phe Pro Ala Val Leu Gln Ser Ser Gly Leu Tyr Ser Leu Ser Ser Val
            180                 185                 190
Val Thr Val Pro Ser Ser Ser Leu Gly Thr Lys Thr Tyr Thr Cys Asn
        195                 200                 205
Val Asp His Lys Pro Ser Asn Thr Lys Val Asp Lys Arg Val Glu Ser
    210                 215                 220
Lys Tyr Gly Pro Pro Cys Pro Pro Cys Pro Ala Pro Glu Phe Leu Gly
225                 230                 235                 240
Gly Pro Ser Val Phe Leu Phe Pro Pro Lys Pro Lys Asp Thr Leu Met
                245                 250                 255
Ile Ser Arg Thr Pro Glu Val Thr Cys Val Val Val Asp Val Ser Gln
            260                 265                 270
Glu Asp Pro Glu Val Gln Phe Asn Trp Tyr Val Asp Gly Val Glu Val
        275                 280                 285
His Asn Ala Lys Thr Lys Pro Arg Glu Glu Gln Phe Asn Ser Thr Tyr
    290                 295                 300
Arg Val Val Ser Val Leu Thr Val Leu His Gln Asp Trp Leu Asn Gly
305                 310                 315                 320
Lys Glu Tyr Lys Cys Lys Val Ser Asn Lys Gly Leu Pro Ser Ser Ile
                325                 330                 335
Glu Lys Thr Ile Ser Lys Ala Lys Gly Gln Pro Arg Glu Pro Gln Val
            340                 345                 350
Tyr Thr Leu Pro Pro Ser Gln Glu Glu Met Thr Lys Asn Gln Val Ser
        355                 360                 365
Leu Thr Cys Leu Val Lys Gly Phe Tyr Pro Ser Asp Ile Ala Val Glu
    370                 375                 380
Trp Glu Ser Asn Gly Gln Pro Glu Asn Asn Tyr Lys Thr Thr Pro Pro
385                 390                 395                 400
Val Leu Asp Ser Asp Gly Ser Phe Phe Leu Tyr Ser Arg Leu Thr Val
                405                 410                 415
Asp Lys Ser Arg Trp Gln Glu Gly Asn Val Phe Ser Cys Ser Val Met
            420                 425                 430
His Glu Ala Leu His Asn His Tyr Thr Gln Lys Ser Leu Ser Leu Ser
        435                 440                 445
Leu Gly Lys
    450

<210>  41
<211>  214
<212>  PRT
<213>  artificial sequence

<220>
<221>  CHAIN
<223>  light chain amino acid sequence of Hu229-013


<400>  41
Asp Ile Gln Met Thr Gln Ser Pro Ser Ser Leu Ser Ala Ser Val Gly
1               5                   10                  15
Asp Arg Val Thr Ile Thr Cys Arg Ala Ser Glu Asn Ile Tyr Ser Asn
            20                  25                  30
Leu Ala Trp Tyr Gln Gln Lys Pro Gly Lys Ala Pro Lys Leu Leu Val
        35                  40                  45
Tyr Ala Ala Thr Asn Leu Ala Asp Gly Val Pro Ser Arg Phe Ser Gly
    50                  55                  60
Ser Gly Ser Gly Thr Asp Tyr Thr Leu Thr Ile Ser Ser Leu Gln Pro

65                  70                  75                  80
Glu Asp Phe Ala Thr Tyr Tyr Cys Gln His Phe Trp Ile Thr Pro Trp
                85                  90                  95
Thr Phe Gly Gly Gly Thr Lys Val Glu Ile Lys Arg Thr Val Ala Ala
            100                 105                 110
Pro Ser Val Phe Ile Phe Pro Pro Ser Asp Glu Gln Leu Lys Ser Gly
        115                 120                 125
Thr Ala Ser Val Val Cys Leu Leu Asn Asn Phe Tyr Pro Arg Glu Ala
    130                 135                 140
Lys Val Gln Trp Lys Val Asp Asn Ala Leu Gln Ser Gly Asn Ser Gln
145                 150                 155                 160
Glu Ser Val Thr Glu Gln Asp Ser Lys Asp Ser Thr Tyr Ser Leu Ser
                165                 170                 175
Ser Thr Leu Thr Leu Ser Lys Ala Asp Tyr Glu Lys His Lys Val Tyr
            180                 185                 190
Ala Cys Glu Val Thr His Gln Gly Leu Ser Ser Pro Val Thr Lys Ser
        195                 200                 205
Phe Asn Arg Gly Glu Cys
    210

<210>  42
<211>  447
<212>  PRT
<213>  artificial sequence

<220>
<221>  CHAIN
<223>  heavy chain amino acid sequence of Hu303-005


<400>  42
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Asp Tyr
            20                  25                  30
Tyr Met Asn Trp Val Arg Gln Ala Pro Gly Gln Arg Leu Glu Trp Met
        35                  40                  45
Gly Val Ile Asn Pro Tyr Asn Gly Asp Thr Ala Tyr Asn Gln Lys Phe
    50                  55                  60
Lys Gly Arg Val Thr Ile Thr Arg Asp Thr Ser Ala Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Asp Asp Gly Tyr Tyr Asp Tyr Tyr Phe Asp Val Trp Gly Gln
            100                 105                 110
Gly Thr Thr Val Thr Val Ser Ser Ala Ser Thr Lys Gly Pro Ser Val
        115                 120                 125
Phe Pro Leu Ala Pro Cys Ser Arg Ser Thr Ser Glu Ser Thr Ala Ala
    130                 135                 140
Leu Gly Cys Leu Val Lys Asp Tyr Phe Pro Glu Pro Val Thr Val Ser
145                 150                 155                 160
Trp Asn Ser Gly Ala Leu Thr Ser Gly Val His Thr Phe Pro Ala Val
                165                 170                 175
Leu Gln Ser Ser Gly Leu Tyr Ser Leu Ser Ser Val Val Thr Val Pro
            180                 185                 190
Ser Ser Ser Leu Gly Thr Lys Thr Tyr Thr Cys Asn Val Asp His Lys
        195                 200                 205
Pro Ser Asn Thr Lys Val Asp Lys Arg Val Glu Ser Lys Tyr Gly Pro
    210                 215                 220
Pro Cys Pro Pro Cys Pro Ala Pro Glu Phe Leu Gly Gly Pro Ser Val
225                 230                 235                 240
Phe Leu Phe Pro Pro Lys Pro Lys Asp Thr Leu Met Ile Ser Arg Thr
                245                 250                 255

Pro Glu Val Thr Cys Val Val Val Asp Val Ser Gln Glu Asp Pro Glu
            260                 265                 270
Val Gln Phe Asn Trp Tyr Val Asp Gly Val Glu Val His Asn Ala Lys
        275                 280                 285
Thr Lys Pro Arg Glu Glu Gln Phe Asn Ser Thr Tyr Arg Val Val Ser
    290                 295                 300
Val Leu Thr Val Leu His Gln Asp Trp Leu Asn Gly Lys Glu Tyr Lys
305                 310                 315                 320
Cys Lys Val Ser Asn Lys Gly Leu Pro Ser Ser Ile Glu Lys Thr Ile
                325                 330                 335
Ser Lys Ala Lys Gly Gln Pro Arg Glu Pro Gln Val Tyr Thr Leu Pro
            340                 345                 350
Pro Ser Gln Glu Glu Met Thr Lys Asn Gln Val Ser Leu Thr Cys Leu
        355                 360                 365
Val Lys Gly Phe Tyr Pro Ser Asp Ile Ala Val Glu Trp Glu Ser Asn
    370                 375                 380
Gly Gln Pro Glu Asn Asn Tyr Lys Thr Thr Pro Pro Val Leu Asp Ser
385                 390                 395                 400
Asp Gly Ser Phe Phe Leu Tyr Ser Arg Leu Thr Val Asp Lys Ser Arg
                405                 410                 415
Trp Gln Glu Gly Asn Val Phe Ser Cys Ser Val Met His Glu Ala Leu
            420                 425                 430
His Asn His Tyr Thr Gln Lys Ser Leu Ser Leu Ser Leu Gly Lys
        435                 440                 445

<210>  43
<211>  214
<212>  PRT
<213>  artificial sequence

<220>
<221>  CHAIN
<223>  light chain amino acid sequence of Hu303-005


<400>  43
Asp Ile Gln Met Thr Gln Ser Pro Ser Ser Leu Ser Ala Ser Val Gly
1               5                   10                  15
Asp Arg Val Thr Ile Thr Cys Arg Ala Ser Gln Asp Ile Gly Ser Arg
            20                  25                  30
Leu Asn Trp Tyr Gln Gln Lys Pro Gly Lys Ala Pro Lys Arg Leu Ile
        35                  40                  45
Tyr Ala Thr Ser Thr Leu Asp Ser Gly Val Pro Ser Arg Phe Ser Gly
    50                  55                  60
Ser Arg Ser Gly Thr Asp Phe Thr Leu Thr Ile Ser Ser Leu Gln Pro
65                  70                  75                  80
Glu Asp Phe Ala Thr Tyr Tyr Cys Leu Gln Leu Ala Ser Ser Pro Pro
                85                  90                  95
Thr Phe Gly Gly Gly Thr Lys Val Glu Ile Lys Arg Thr Val Ala Ala
            100                 105                 110
Pro Ser Val Phe Ile Phe Pro Pro Ser Asp Glu Gln Leu Lys Ser Gly
        115                 120                 125
Thr Ala Ser Val Val Cys Leu Leu Asn Asn Phe Tyr Pro Arg Glu Ala
    130                 135                 140
Lys Val Gln Trp Lys Val Asp Asn Ala Leu Gln Ser Gly Asn Ser Gln
145                 150                 155                 160
Glu Ser Val Thr Glu Gln Asp Ser Lys Asp Ser Thr Tyr Ser Leu Ser
                165                 170                 175
Ser Thr Leu Thr Leu Ser Lys Ala Asp Tyr Glu Lys His Lys Val Tyr
            180                 185                 190
Ala Cys Glu Val Thr His Gln Gly Leu Ser Ser Pro Val Thr Lys Ser
        195                 200                 205
Phe Asn Arg Gly Glu Cys

    210

## Description

### FIELD OF THE INVENTION

The present invention belongs to the field of pharmaceutical preparations, in particular, the present invention relates to a pharmaceutical composition comprising a LAG-3 antibody and antigen-binding fragment thereof, and the use thereof as a medicament.

### BACKGROUND OF THE INVENTION

Lymphocyte Activation Gene-3, also known as LAG-3 or CD215, is a member of the immunoglobulin superfamily, which can negatively regulate various functions and survival cycles of immune cells. Studies have shown that LAG-3 plays an important role in viral infection, autoimmune diseases and tumor-induced immune system dysfunction. Influencing the function of LAG-3 can improve the status of immune dysfunction during the development of these diseases, so as to improve the prognosis of the diseases.

As a member of the immunoglobulin superfamily, LAG-3 is composed of three regions: extracellular domain, transmembrane region and the cytoplasmic domain. Mature LAG-3 molecule, which was first discovered by Triebel et al., in 1990 (J Exp Med, 1990, 171 (5): 1393-405), consists of 470 amino acids with a relative molecular weight of 70kDa. Researchers have found that LAG-3, like CTLA-4 and PD-1, is a negative co-stimulatory molecule, the activation of which can negatively regulate function of lymphocytes. Structurally, LAG-3 is closely related to CD4, but the function thereof is opposite to that of CD4. Specifically, LAG-3 molecule has high similarity to CD4 molecule, and both can bind to MHC-II (Major Histocompatibility Complex) class molecules. However, the binding avidity of LAG-3 to MHC-II molecules is higher than that of CD4. Thus, LAG-3 intervenes in TCR activation induced by CD4+T lymphocytes and inhibits the activation of T lymphocytes (Curr Opin Immunol, 2009, 21(2):179-86; Eur J Immunol, 2003, 33 (4): 970-9). In vitro studies, it has been shown that LAG-3 can inhibit antigen-induced proliferation of T lymphocytes. Blocking LAG-3 will improve activation and proliferation of T lymphocytes, and improve the cytokines secreted by type 1 T helper cells (Th1). Huang et al., have showed that the level of LAG-3 was significantly increased on the activated CD4+ Treg cell surface, and LAG-3 was a necessary condition enabling CD4+ Tregs to exert the greatest immunosuppressive effect (Immunity, 2004, 21 (4): 503-13). In addition, anti-LAG-3 antibodies also maintain the homeostasis of CD4+ and CD8+T lymphocytes, blocking LAG-3 will significantly enhance the ability of CD8+T lymphocytes to kill tumor cells (J Clin Invest, 2007, 117 (11): 3383-92). It has also been found in some studies on diseases that LAG-3 plays an important role in the regulating development and progression of diseases. Gandhi et al., verified that the expression level of LAG-3 in T lymphocytes of human lymphoma tissue is associated with T lymphocyte dysfunction, and clearance of LAG-3⁺T lymphocytes can significantly enhance the ability of eliminating tumor cells by lymphocytes (Blood, 2006, 108 (7): 2280-9). The results show that LAG-3 is an important inhibitory molecule on the surface of immune cells and has a significant negative regulatory effect on T lymphocytes.

LAG-3 is mainly expressed on T lymphocytes, B lymphocytes, NK cells, Treg cells and DC cells (Proc Natl Acad Sci USA, 1997, 94 (11): 5744-9. Eur J Immunol, 2005, 35 (7): 2081-8; J Immunol, 2009, 182 (4): 1885-91). LAG-3 is a class of immunosuppressive molecules, and is one of the components constituting the co-receptor of TCR. It intervenes in TCR activation induced by T lymphocytes, and plays a negatively regulatory role in the activation of T lymphocytes. In some diseases, the expression of LAG-3 was increased, and the corresponding immunosuppression was observed. Gandhi et al., found that the LAG-3 was highly expressed in lymphocytes from the blood and tumor tissues of patients with Hoggkin's lymphoma; and the function of specific CD8+T cells was obviously impaired in tumor tissues, if the LAG-3-positive T cell was removed, the anti-tumor function was restored and cytokine secretion was increased. The authors speculated that the expression of LAG-3 is associated with the negative regulation of the immune function by specific T cells, inhibiting the function of LAG-3 molecule can enhance the anti-tumor effect of T cells, so that LAG-3 molecule may be a potential target for tumor immunotherapy (Blood, 2006, 108 (7): 2280-9).

Currently there are several multinational pharmaceutical companies, such as BMS and Novartis, engaging in the study of monoclonal antibodies against LAG-3, which enhance the anti-tumor effect of T cells and maximize the patients' own immune response to the tumor by stimulating antigen-specific T cell responses, and subsequently achieve the purpose of killing tumor cells.

However, antibody drugs are unstable due to their large molecular weight, complex structure, being susceptible to degradation, being polymerized, or undesired chemical modification. Studies on stable formulations of antibody drugs are particularly important in order to make antibodies suitable for administration, and to maintain stability during the storage and the subsequent use.

Although a number of companies are currently developing LAG3 antibodies and their formulations, for example, WO2018204374, WO2010019570, WO2014008218, WO9530750, WO2004078928, WO2008132601, WO2014140180, WO2015138920, and so forth, there are little studies focusing on the new LAG3 antibody formulation. There is still a need to develop pharmaceutical compositions (formulations) comprising LAG3 which are more suitable for administration.

### SUMMARY OF THE INVENTION

The present invention provides a pharmaceutical composition comprising a LAG3 antibody or an antigen-binding fragment thereof and a buffer, the buffer is selected from the group consisting of acetate buffer, histidine buffer, citrate buffer, succinate buffer or Tris buffer.

In an alternative embodiment, the acetate buffer comprised in the pharmaceutical composition is selected from the group consisting of acetic acid-sodium acetate buffer, acetic acid-potassium acetate buffer, acetic acid-histidine salt buffer, acetic acid-calcium acetate buffer and acetic acid-magnesium acetate buffer, preferably is acetic acid-sodium acetate buffer.

In an alternative embodiment, the histidine buffer comprised in the pharmaceutical composition is selected from the group consisting of histidine-hydrochloric acid buffer, histidine-acetic acid buffer, histidine-phosphoric acid buffer, histidine-sulfuric acid buffer, preferably is histidine-hydrochloric acid buffer.

In an alternative embodiment, the citrate buffer comprised in the pharmaceutical composition is citric acid-sodium citrate buffer; the succinate buffer is succinic acid-sodium succinate buffer.

In an alternative embodiment, the concentration of the LAG-3 antibody or an antigen-binding fragment thereof comprised in the pharmaceutical composition is from about 1 mg/ml to 90 mg/ml, preferably from about 10 mg/ml to 90 mg/ml, preferably from about 20 mg/ml to 90 mg/ml, preferably from about 30 mg/ml to 90 mg/ml, preferably from about 40 mg/ml to 90 mg/ml, preferably from about 50 mg/ml to 90 mg/ml, preferably from about 60 mg/ml to 90 mg/ml, preferably from about 70 mg/ml to 90 mg/ml, preferably from about 80 mg/ml to 90 mg/ml, preferably from about 10 mg/ml to 80 mg/ml, preferably from about 20 mg/ml to 80 mg/ml, preferably from about 30 mg/ml to 80 mg/ml, preferably from about 40 mg/ml to 80 mg/ml, preferably from about 50 mg/ml to 80 mg/ml, preferably from about 60 mg/ml to 80 mg/ml, preferably from about 70 mg/ml to 80 mg/ml, preferably from about 10 mg/ml to 70 mg/ml, preferably from about 20 mg/ml to 70 mg/ml, preferably from about 30 mg/ml to 70 mg/ml, preferably from about 40 mg/ml to 70 mg/ml, preferably from about 50 mg/ml to 70 mg/ml, preferably from about 60 mg/ml to 70 mg/ml, preferably about 10 mg/ml to 60 mg/ml, preferably from about 20 mg/ml to 60 mg/ml, preferably from about 30 mg/ml to 60 mg/ml, preferably from about 40 mg/ml to 60 mg/ml, preferably from about 50 mg/ml to 60 mg/ml, preferably from about 10 mg/ml to 50 mg/ml, preferably from about 20 mg/ml to 50 mg/ml, preferably from about 30 mg/ml to 50 mg/ml, preferably from about 40 mg/ml to 50 mg/ml, preferably about from 10 mg/ml to 40 mg/ml, preferably from about 20 mg/ml to 40 mg/ml, preferably from about 30 mg/ml to 40 mg/ml, preferably from about 10 mg/ml to 30 mg/ml, preferably from about 20 mg/ml to 30 mg/ml. As a non-limiting example, the concentration of the LAG-3 antibody or antigen-binding fragment thereof is about 40 mg/ml, 41 mg/ml, 42 mg/ml, 43 mg/ml, 44 mg/ml, 45 mg/ml, 46 mg/ml, 47 mg/ml, 48mg/ml, 49mg/ml, 50mg/ml, 51mg/ml, 52mg/ml, 53mg/ml, 54mg/ml, 55mg/ml, 56mg/ml, 57mg/ml, 58mg/ml, 59mg/ml or 60 mg/ml, most preferably is 50 mg/ml.

In an alternative embodiment, the concentration of the buffer is from about 5 mM to 30 mM, preferably from about 10 mM to 30 mM, preferably from about 15 mM to 30 mM, preferably from about 20 mM to 30 mM, preferably from about 25 mM to 30 mM, preferably from about 5 mM to 25 mM, preferably from about 10 mM to 25 mM, preferably from about 15 mM to 25 mM, preferably from about 20 mM to 25 mM, preferably from about 5 mM to 20 mM, preferably from about 10 mM to 15 mM; As a non-limiting example, the concentration of the buffer is about 10 mM, 12 mM, 14 mM, 16 mM, 18 mM, 20 mM, 22 mM, 24 mM, 26 mM, 28 mM or 30 mM, most preferably is 10 mM.

In an alternative embodiment, the pH value of the buffer comprised in the pharmaceutical composition is from about 5.0 to 7.5, preferably from about 5.5 to 7.5, preferably from about 6.0 to 7.5, preferably from about 6.5 to 7.5, preferably from about 7.0 to 7.5, preferably from about 5.0 to 7.0, preferably from about 5.5 to 7.0, preferably from about 6.0 to 7.0, preferably from about 6.5 to 7.0, preferably from about 5.0 to 6.5, preferably from about 5.5 to 6.5, preferably from about 6.0 to 6.5, preferably from about 5.0 to 6.0, preferably from about 5.5 to 6.0, preferably from about 5.0 to 5.5. As a non-limiting example, pH value of the buffer can alternatively be about 5.0, 5.1, 5.2, 5.3, 5.4, 5.5, 5.6, 5.7, 5.8, 5.9, 6.0, 6.0, 6.1, 6.2, 6.3, 6.4, 6.5, more preferably from about 5.5 to 6.0, still more preferably 5.5 or 6.0.

Further, in an alternative embodiment, the pharmaceutical composition further comprises an adjuvant selected from one or more of a saccharide and a surfactant.

In an alternative embodiment, wherein the saccharide is disaccharide, preferably is trehalose, sucrose, mannitol or sorbitol, more preferably is sucrose. In an alternative embodiment, the concentration of the saccharide comprised in the pharmaceutical composition is from about 30 mg/ml to 90 mg/ml, preferably from about 60 mg/ml to 90 mg/ml, from 35 mg/ml to 90 mg/ml, preferably from about 40 mg/ml to 90 mg/ml, preferably from about 45 mg/ml to 90 mg/ml, preferably from about 50 mg/ml to 90 mg/ml, preferably from about 55 mg/ml to 90 mg/ml, preferably from about 60 mg/ml to 90 mg/ml, preferably from about 65 mg/ml to 90 mg/ml, preferably from about 70 mg/ml to 90 mg/ml, preferably from about 75 mg/ml to 90 mg/ml, preferably from about 80 mg/ml to 90 mg/ml, preferably from about 85 mg/ml to 90 mg/ml, preferably from about 30 mg/ml to 85 mg/ml, preferably from about 35 mg/ml to 85 mg/ml, preferably from about 40 mg/ml to 85 mg/ml, preferably from about 45 mg/ml to 85 mg/ml, preferably from about 50 mg/ml to 85 mg/ml, preferably from about 55 mg/ml to 85 mg/ml, preferably from about 60 mg/ml to 85 mg/ml, preferably from about 65 mg/ml to 85 mg/ml, preferably from about 70 mg/ml to 85 mg /ml, preferably from about 75 mg/ml to 85 mg/ml, preferably from about 80 mg/ml to 85 mg/ml, preferably from about 30 mg/ml to 80 mg/ml, preferably from about 35 mg/ml to 80 mg/ml, preferably from about 40 mg/ml to 80 mg/ml, preferably from about 45 mg/ml to 80 mg/ml, preferably from about 50 mg/ml to 80 mg/ml, preferably from about 55 mg/ml to 80 mg/ml, preferably from about 60 mg/ml to 80 mg/ml, preferably from about 65 mg/ml to 80 mg/ml, preferably from about 70 mg/ml to 80 mg/ml, preferably from about 75 mg/ml to 80 mg/ml, preferably from about 30 mg/ ml to 75 mg/ml, preferably from about 35 mg/ml to 75 mg/ml, preferably from about 40 mg/ml to 75 mg/ml, preferably from about 45 mg/ml to 75 mg/ml, preferably from about 50 mg/ml to 75 mg/ml, preferably from about 55 mg/ml to 75 mg/ml, preferably from about 60 mg/ml to 75 mg/ml, preferably from about 65 mg/ml to 75 mg/ml, preferably from about 70 mg/ml to 75 mg/ml, preferably from about 30 mg/ml to 70 mg/ml, preferably from about 35 mg/ml to 70 mg/ml, preferably from about 40 mg/ml to 70 mg/ml, preferably from about 45 mg/ml to 70 mg/ml, preferably from about 50 mg/ml to 70 mg/ml, preferably from about 55 mg/ml to 70 mg/ml, preferably from about 60 mg/ml to 70 mg/ml, preferably from about 65 mg/ml to 70 mg/ml, preferably from about 30 mg/ml to 65 mg/ml, preferably from about 35 mg/ml to 65 mg/ml, preferably from about 40 mg/ml to 65 mg/ml, preferably from about 45 mg/ml to 65 mg/ml, preferably from about 50 mg/ml to 65 mg/ml, preferably from about 55 mg/ml to 65 mg/ml, preferably from about 60 mg/ml to 65 mg/ml, preferably from about 30 mg/ml to 60 mg/ml, preferably from about 35 mg/ml to 60 mg/ml, preferably from about 40 mg/ml to 60 mg/ml, preferably from about 45 mg/ml to 60 mg/ml, preferably from about 50 mg/ml to 60 mg/ml, preferably from about 55 mg/ml to 60 mg/ml, preferably from about 30 mg/ml to 55 mg/ml, preferably from about 35 mg/ml to 55 mg/ml, preferably from about 40 mg/ml to 55 mg/ml, preferably from about 45 mg/ml to 55 mg/ml, preferably from about 50 mg/ml to 55 mg/ml, preferably from about 30 mg/ml to 50 mg/ml, preferably from about 35 mg/ml to 50 mg/ml, preferably from about 40 mg/ml to 50 mg/ml, preferably from about 45 mg/ml to 50 mg/ml, preferably from about 30 mg/ml to 45 mg/ml, preferably from about 35 mg/ml to 45 mg/ml, preferably from about 40 mg/ml to 45 mg/ml, preferably from about 30 mg/ml to 40 mg/ml, preferably from about 35 mg/ml to 40 mg/ml, preferably from about 30 mg/ml to 35 mg/ml; As a non-limiting example, the concentration of the saccharide comprised in the pharmaceutical composition is about 60 mg/ml, 65 mg/ml, 70 mg/ml, 75 mg/ml, 80 mg/ml, 85 mg/ml or 90 mg/ml, more preferably is from about 70 mg/ml to 80 mg/ml, most preferably is 75 mg/ml.

In an alternative embodiment, the pharmaceutical composition further comprises a surfactant. The surfactant can be selected from the group consisting of polysorbate 20, polysorbate 80, polyhydroxyl hydrocarbon, Triton, sodium dodecyl sulfonate, sodium lauryl sulfonate, sodium octyl glycoside, lauryl-sulfobetaine, myristyl-sulfobetaine, linoleyl-sulfobetaine, stearyl-sulfobetaine, lauryl-sarcosine, myristyl-sarcosine, linoleyl-sarcosine, stearyl-sarcosine, linoleyl-betaine, myristyl-betaine, cetyl-betaine, lauramidopropyl-betaine, cocaamidopropyl-betaine, linoleamidopropyl-betaine, myristamidopropyl-betaine, palmitoylpropyl-betaine, isostearamidopropyl-betaine, myristamidopropyl-dimethylamine, palmitoylpropyl-dimethylamine, isostearamidopropyl-dimethylamine, sodium methylcocoacyl, sodium methyloleyl taurine, polyethylene glycol, polypropylene glycol, copolymer of ethylene and propylene glycol, and so forth. The preferred surfactant is polysorbate 80 or polysorbate 20, more preferably is polysorbate 80.

In an alternative embodiment, the concentration of the surfactant comprised in the pharmaceutical composition is from about 0.02 mg/ml to 0.8 mg/ml, preferably from about 0.1 mg/ml to 0.8 mg/ml, preferably from about 0.2 mg/ml to 0.8 mg/ml, preferably from about 0.3 mg/ml to 0.8 mg/ml, preferably from about 0.4 mg/ml to 0.8 mg/ml, preferably from about 0.5 mg/ml to 0.8 mg/ml, preferably from about 0.6 mg/ml to 0.8 mg/ml, preferably from about 0.7 mg/ml to 0.8 mg/ml, preferably from about 0.02 mg/ml to 0.7 mg/ml, preferably from about 0.1 mg/ml to 0.7 mg/ml, preferably from about 0.2 mg/ml to 0.7 mg/ml, preferably from about 0.3 mg/ml to 0.7 mg/ml, preferably from about 0.4 mg/ml to 0.7 mg/ml, preferably from about 0.5 mg/ml to 0.7 mg/ml, preferably from about 0.6 mg/ml to 0.7 mg/ml, preferably from about 0.02 mg/ml to 0.6 mg/ml, preferably from about 0.1 mg/ml to 0.6 mg/ml, preferably from about 0.2 mg/ml to 0.6 mg/ml, preferably from about 0.3 mg/ml to 0.6 mg/ml, preferably from about 0.4 mg/ml to 0.6 mg/ml, preferably from about 0.5 mg/ml to 0.6 mg/ml, preferably from about 0.02 mg/ml to 0.5 mg/ml, preferably from about 0.1 mg/ml to 0.5 mg/ml, preferably from about 0.2 mg/ml to 0.5 mg/ml, preferably from about 0.3 mg/ml to 0.5 mg/ml, preferably from about 0.4 mg/ml to 0.5 mg/ml, preferably from about 0.02 mg/ml to 0.4 mg/ml, preferably from about 0.1 mg/ml to 0.4 mg/ml, preferably from about 0.2 mg/ml to 0.4 mg/ml, preferably from about 0.3 mg/ml to 0.4 mg/ml, preferably from about 0.02 mg/ml to 0.3 mg/ml, preferably from about 0.1 mg/ml to 0.3 mg/ml, preferably from about 0.2 mg/ml to 0.3 mg/ml, preferably from about 0.02 mg/ml to 0.2 mg/ml, preferably from about 0.1 mg/ml to 0.2 mg/ml, preferably from about 0.02 mg/ml to 0.1 mg/ml. As a non-limiting example, the concentration of the surfactant comprised in the pharmaceutical composition is about 0.2 mg/ml, 0.25 mg/ml, 0.3 mg/ml, 0.35 mg/ml, 0.4 mg/ml, 0.45 mg/ml or 0.5 mg/ml, more preferably is from about 0.3 mg/ml to 0.5 mg/ml.

In one embodiment, the pharmaceutical composition comprises the components as shown in i) or) ii) below:
i) (a) 1 mg/ml to 90 mg/ml LAG-3 antibody or antigen-binding fragment thereof, (b) 5 mM to 30 mM acetic acid-sodium acetate buffer, pH is about 5.0-6.5, (c) 30 mg/ml to 90 mg/ml sucrose, and (d) 0.02 mg/ml to 0.8 mg/ml polysorbate 80; preferably, the pharmaceutical composition comprises:
   (e) 40 mg/ml to 80 mg/ml LAG-3 antibody or antigen-binding fragment thereof, (f) 10 mM to 30 mM acetic acid-sodium acetate buffer, pH is about 5.2-5.8, (g) 70 mg/ml to 80 mg /ml sucrose, and (h) 0.4 mg/ml to 0.5 mg/ml polysorbate 80; or
ii) (a) 1 mg/ml to 90 mg/ml LAG-3 antibody or antigen-binding fragment thereof, (b) 5 mM to 30 mM histidine-hydrochloric acid buffer, pH is about 5.0-6.5, (c) 30 mg/ml to 90 mg/ml sucrose or trehalose, and (d) 0.05 mg/ml to 0.6 mg/ml polysorbate 80; preferably, the pharmaceutical composition comprises:
   (e) 45 mg/ml to 60 mg/ml LAG-3 antibody or antigen-binding fragment thereof, (f) 10 mM to 30 mM histidine-hydrochloric acid buffer, pH is about 5.5-6.0, (g) 60 mg/ml to 90 mg/ml sucrose, and (h) 0.2 mg/ml to 0.6 mg/ml polysorbate 80.

In one embodiment, the LAG3 antibody or antigen-binding fragment thereof comprised in the pharmaceutical composition comprises heavy chain HCDR1, HCDR2 and HCDR3 as shown in SEQ ID NO: 9, SEQ ID NO: 10 and SEQ ID NO: 11, respectively; and light chain LCDR1, LCDR2 and LCDR3 as shown in SEQ ID NO: 15, SEQ ID NO: 16 and SEQ ID NO: 17, respectively.

In an alternative embodiment, the pharmaceutical composition comprises:
(a) 1 mg/ml to 90 mg/ml LAG-3 antibody or antigen-binding fragment thereof, (b) 5 mM to 30 mM acetate buffer, preferably pH is 5.0 to 6.5, (c) 30 mg/ml to 90 mg/ml sucrose, and (d) 0.02 mg/ml to 0.8 mg/ml polysorbate 80.

In an alternative embodiment, the pharmaceutical composition comprises:
(a) 40 mg/ml to 80 mg/ml LAG-3 antibody or antigen-binding fragment thereof, (b) 10 mM to 30 mM acetate buffer, pH is about 5.0-6.0, (c) 60 mg/ml to 90 mg/ml sucrose, and (d) 0.1 mg/ml to 0.5 mg/ml polysorbate 80.

In an alternative embodiment, the pharmaceutical composition comprises:
(a) 50 mg/ml to 60 mg/ml LAG-3 antibody or antigen-binding fragment thereof, (b) 10 mM to 30 mM acetic acid-sodium acetate buffer, and pH is about 5.2-5.8, (c) 70 mg to 80 mg/ml sucrose, and (d) 0.4 mg/ml to 0.5 mg/ml polysorbate 80.

In an alternative embodiment, the pharmaceutical composition comprises:
(a) about 50 mg/ml LAG-3 antibody or antigen-binding fragment thereof, (b) 10 mM acetic acid-sodium acetate buffer, pH is about 5.5, (c) about 75 mg/ml sucrose, and (d) about 0.4 mg/ml polysorbate 80.

In an alternative embodiment, the LAG-3 antibody or antigen-binding fragment thereof comprised in the above pharmaceutical composition is a murine antibody or antigen-binding fragment thereof, a chimeric antibody or antigen-binding fragment thereof, a humanized antibody or antigen-binding fragment thereof.

In an alternative embodiment, the murine LAG3 antibody comprised in the above pharmaceutical composition comprises a heavy chain variable region set forth in SEQ ID NO: 5 and a light chain variable region set forth in SEQ ID NO:6.

In an alternative embodiment, the humanized LAG-3 antibody comprised in the above pharmaceutical composition comprises a heavy chain variable region and a light chain variable region, wherein the amino acid sequence of the heavy chain variable region is set forth in any one of SEQ ID NO: 21, SEQ ID NO: 23, SEQ ID NO: 24 or SEQ ID NO: 25, or has at least 85% sequence identity thereto; and
wherein the light chain variable region is set forth in any one of SEQ ID NO: 22, SEQ ID NO: 26, SEQ ID NO: 27 or SEQ ID NO: 28, or has at least 85% sequence identity thereto.

In an alternative embodiment, the light chain variable region of the LAG-3 antibody comprised in the pharmaceutical composition has at least 85%, 86%, 87%, 88%, 89%, 90%, 91%, 92%, 93%, 94%, 95%, 96%, 97%, 98 %, 99% or 100% sequence identity to the light chain variable region amino acid sequence set forth in SEQ ID NO:22, SEQ ID NO:26, SEQ ID NO:27 or SEQ ID NO:28, and the heavy chain variable region amino acid sequence of the LAG-3 antibody has at least 85%, 86%, 87%, 88%, 89%, 90%, 91%, 92%, 93%, 94%, 95%, 96%, 97 %, 98%, 99% or 100% sequence identity to the heavy chain variable region amino acid sequence set forth in SEQ ID NO: 21, SEQ ID NO: 23, SEQ ID NO: 24 or SEQ ID NO: 25.

In an alternative embodiment, the LAG3 humanized antibody comprised in the above pharmaceutical composition comprises a combination of a heavy chain variable region and a light chain variable region selected from the group consisting of:
1) a heavy chain variable region of SEQ ID NO: 21 and a light chain variable region of SEQ ID NO: 22;
2) a heavy chain variable region of SEQ ID NO: 21 and a light chain variable region of SEQ ID NO: 26;
3) a heavy chain variable region of SEQ ID NO: 21 and a light chain variable region of SEQ ID NO: 27;
4) a heavy chain variable region of SEQ ID NO: 21 and a light chain variable region of SEQ ID NO: 28;
5) a heavy chain variable region of SEQ ID NO: 23 and a light chain variable region of SEQ ID NO: 22;
6) a heavy chain variable region of SEQ ID NO: 23 and a light chain variable region of SEQ ID NO: 26;
7) a heavy chain variable region of SEQ ID NO: 23 and a light chain variable region of SEQ ID NO: 27;
8) a heavy chain variable region of SEQ ID NO: 23 and a light chain variable region of SEQ ID NO: 28;
9) a heavy chain variable region of SEQ ID NO: 24 and a light chain variable region of SEQ ID NO: 22;
10) a heavy chain variable region of SEQ ID NO: 24 and a light chain variable region of SEQ ID NO: 26;
11) a heavy chain variable region of SEQ ID NO: 24 and a light chain variable region of SEQ ID NO: 27;
12) a heavy chain variable region of SEQ ID NO: 24 and a light chain variable region of SEQ ID NO: 28;
13) a heavy chain variable region of SEQ ID NO: 25 and a light chain variable region of SEQ ID NO: 22;
14) a heavy chain variable region of SEQ ID NO: 25 and a light chain variable region of SEQ ID NO: 26;
15) a heavy chain variable region of SEQ ID NO: 25 and a light chain variable region of SEQ ID NO: 27; and
16) a heavy chain variable region of SEQ ID NO: 25 and a light chain variable region of SEQ ID NO: 28.

In an alternative embodiment, the humanized LAG-3 antibody comprised in the above pharmaceutical composition comprises a heavy chain constant region and a light chain constant region, wherein the heavy chain constant region is preferably set forth in SEQ ID NO:38, the light chain constant region is preferably set forth in SEQ ID NO:39.

In an alternative embodiment, the heavy chain of the humanized LAG-3 antibody comprised in the above pharmaceutical composition is set forth in SEQ ID NO: 40 or has at least 95% sequence identity to SEQ ID NO: 40, and the light chain is set forth in SEQ ID NO: 41 or has at least 95% sequence identity to SEQ ID NO: 41.

In an alternative embodiment, the heavy chain of the LAG-3 antibody comprised in the pharmaceutical composition has at least 95%, 96%, 97%, 98%, 99% or 100% sequence identity to the heavy chain amino acid sequence set forth in SEQ ID NO:40, and the light chain amino acid sequence of the LAG-3 antibody has at least 95%, 96%, 97%, 98%, 99% or 100% sequence identity to the antibody light chain set forth in SEQ ID NO:41.

In one embodiment, the pharmaceutical composition comprises 50 mg/ml LAG3 antibody Hu229-013 and 10 mM acetic acid-sodium acetate buffer, pH 5.5.

In yet another embodiment, the pharmaceutical composition comprises 50 mg/ml LAG3 antibody Hu229-013 and 10 mM succinic acid-sodium succinate buffer, pH 6.0.

In yet another embodiment, the pharmaceutical composition comprises 50 mg/ml LAG3 antibody Hu229-013 and 10 mM histidine-hydrochloric acid buffer, pH 6.0.

In yet another embodiment, the pharmaceutical composition comprises 50 mg/ml LAG3 antibody Hu229-013, 10 mM succinic acid-sodium succinate, pH 6.0 and 0.1 mg/ml polysorbate 80.

In yet another embodiment, the pharmaceutical composition comprises 50 mg/ml LAG3 antibody Hu229-013, 10 mM succinic acid-sodium succinate, pH 6.0 and 70 mg/ml sucrose.

In yet another embodiment, the pharmaceutical composition comprises 50 mg/ml LAG3 antibody Hu229-013, 10 mM acetic acid-sodium acetate, pH 5.5, 60 mg/ml sucrose and 0.4 mg/mL polysorbate 80.

In yet another embodiment, the pharmaceutical composition comprises 50 mg/ml LAG3 antibody Hu229-013, 10 mM histidine-acetic acid, pH 6.0, 60 mg/ml sucrose and 0.4 mg/mL polysorbate 80.

In some embodiments, the pharmaceutical composition comprises 1 mg/ml LAG3 antibody Hu229-013, 10-30 mM histidine-acetic acid, pH 5.5, 75 mg/ml sucrose and 0.2 mg/mL polysorbate 80.

In still other embodiments, the pharmaceutical composition comprises 50 mg/ml LAG3 antibody Hu229-013, 10-30 mM acetic acid-sodium acetate, pH 5.2-5.8, 75 mg/ml sucrose and 0.2 mg/mL polysorbate 80.

In yet another embodiment, the pharmaceutical composition comprises 60 mg/ml LAG3 antibody Hu229-013, 10 mM acetic acid-sodium acetate, pH 5.5, 60 mg/ml sucrose and 0.4 mg/mL polysorbate 80.

In still another embodiment, the pharmaceutical composition comprises 50-60 mg/ml LAG3 antibody Hu229-013, 10 mM acetic acid-sodium acetate, pH 5.5, 30-90 mg/ml sucrose and 0.4-0.5 mg/mL polysorbate 80.

In yet another embodiment, the pharmaceutical composition comprises 50 mg/ml LAG3 antibody Hu229-013, 10 mM acetic acid-sodium acetate, pH 5.5, 75 mg/ml sucrose and 0.4 mg/mL polysorbate 80.

In another embodiments, the LAG-3 antibody or antigen-binding fragment thereof comprised in the pharmaceutical composition comprises HCDR1, HCDR2 and HCDR3 set forth in SEQ ID NO: 12, SEQ ID NO: 13 and SEQ ID NO: 14, respectively; and LCDR1, LCDR2 and LCDR3 set forth in SEQ ID NO: 18, SEQ ID NO: 19 and SEQ ID NO: 20, respectively.

In an alternative embodiment, the pharmaceutical composition comprises:
(a) 1 mg/ml to 90 mg/ml LAG-3 antibody or antigen-binding fragment thereof, (b) 5 mM to 30 mM histidine-hydrochloric acid buffer, pH is about 5.0-6.5, (c) 30 mg/ml to 90 mg/ml sucrose or trehalose, and (d) 0.05 mg/ml to 0.6 mg/ml polysorbate 80.

In an alternative embodiment, the pharmaceutical composition comprises:
(a) 45 mg/ml to 60 mg/ml LAG-3 antibody or antigen-binding fragment thereof, (b) about 10 mM to 30 mM of histidine-sodium hydrochloride buffer, pH is about 5.0-6.0, (c) 60 mg to 90 mg/ml sucrose or trehalose, and (d) 0.2 mg/ml to 0.6 mg/ml polysorbate 80.

In an alternative embodiment, the pharmaceutical composition comprises:
(a) about 50 mg/ml LAG-3 antibody or antigen-binding fragment thereof, (b) 10 mM histidine-hydrochloric acid buffer, pH is about 5.0, (c) about 75 mg/ml sucrose or trehalose, and (d) about 0.3 mg/ml polysorbate 80.

In an alternative embodiment, the LAG-3 antibody or antigen-binding fragment thereof comprised in the above pharmaceutical composition is a murine antibody or an antigen-binding fragment thereof, a chimeric antibody or an antigen-binding fragment thereof, or a humanized antibody or an antigen-binding fragment thereof.

In an alternative embodiment, the murine LAG3 antibody comprised in the above pharmaceutical composition comprises a heavy chain variable region set forth in SEQ ID NO: 7 and a light chain variable region set forth in SEQ ID NO:8.

In an alternative embodiment, the humanized LAG-3 antibody comprised in the above pharmaceutical composition comprises:
a heavy chain variable region sequence selected from any one of SEQ ID NO: 29, SEQ ID NO: 31, SEQ ID NO: 32 or SEQ ID NO: 33 or an amino acid sequence having at least 85% sequence identity thereto, and a light chain variable region sequence selected from any one of SEQ ID NO: 30, SEQ ID NO: 34, SEQ ID NO: 35, SEQ ID NO: 36 or SEQ ID NO: 37 or an amino acid sequence having at least 85% sequence identity thereto.

In an alternative embodiment, the light chain variable region of the LAG-3 antibody comprised in the pharmaceutical composition has at least 85%, 86%, 87%, 88%, 89%, 90%, 91%, 92%, 93%, 94%, 95%, 96%, 97 %, 98%, 99% or 100% sequence identity to the light chain variable region amino acid sequence set forth in SEQ ID NO: 30, SEQ ID NO: 34, SEQ ID NO: 35, SEQ ID NO: 36 or SEQ ID NO:37, and the heavy chain variable region amino acid sequence of the LAG-3 antibody has at least 85%, 86%, 87%, 88%, 89%, 90%, 91%, 92%, 93%, 94%, 95%, 96%, 97%, 98%, 99% or 100% sequence identity to the heavy chain variable region set forth in SEQ ID NO: 29, SEQ ID NO: 31, SEQ ID NO: 32 or SEQ ID NO: 33.

In an alternative embodiment, the humanized LAG3 antibody comprised in the above pharmaceutical composition comprises a combination of a heavy chain variable region and a light chain variable region selected from the group consisting of:
1) a heavy chain variable region of SEQ ID NO: 29 and a light chain variable region of SEQ ID NO: 30;
2) a heavy chain variable region of SEQ ID NO: 29 and a light chain variable region of SEQ ID NO: 34;
3) a heavy chain variable region of SEQ ID NO: 29 and a light chain variable region of SEQ ID NO: 35;
4) a heavy chain variable region of SEQ ID NO: 29 and a light chain variable region of SEQ ID NO: 36;
5) a heavy chain variable region of SEQ ID NO: 29 and a light chain variable region of SEQ ID NO: 37;
6) a heavy chain variable region of SEQ ID NO: 31 and a light chain variable region of SEQ ID NO: 30;
7) a heavy chain variable region of SEQ ID NO: 31 and a light chain variable region of SEQ ID NO: 34;
8) a heavy chain variable region of SEQ ID NO: 31 and a light chain variable region of SEQ ID NO: 35;
9) a heavy chain variable region of SEQ ID NO: 31 and a light chain variable region of SEQ ID NO: 36;
10) a heavy chain variable region of SEQ ID NO: 31 and a light chain variable region of SEQ ID NO: 37;
11) a heavy chain variable region of SEQ ID NO: 32 and a light chain variable region of SEQ ID NO: 30;
12) a heavy chain variable region of SEQ ID NO: 32 and a light chain variable region of SEQ ID NO: 34;
13) a heavy chain variable region of SEQ ID NO: 32 and a light chain variable region of SEQ ID NO: 35;
14) a heavy chain variable region of SEQ ID NO: 32 and a light chain variable region of SEQ ID NO: 36;
15) a heavy chain variable region of SEQ ID NO: 32 and a light chain variable region of SEQ ID NO: 37;
16) a heavy chain variable region of SEQ ID NO: 33 and a light chain variable region of SEQ ID NO: 30;
17) a heavy chain variable region of SEQ ID NO: 33 and a light chain variable region of SEQ ID NO: 34;
18) a heavy chain variable region of SEQ ID NO: 33 and a light chain variable region of SEQ ID NO: 35;
19) a heavy chain variable region of SEQ ID NO: 33 and a light chain variable region of SEQ ID NO: 36; and
20) a heavy chain variable region of SEQ ID NO: 33 and a light chain variable region of SEQ ID NO: 37.

In an alternative embodiment, the heavy chain of the chimeric antibody or humanized antibody comprised in the above pharmaceutical composition further comprises a heavy chain constant region derived from human IgG1, IgG2, IgG3 or IgG4 or a variant thereof, preferably comprises a heavy chain constant region derived from human IgG4 or a variant thereof, most preferably comprised a heavy chain constant region set forth in SEQ ID NO: 38; and the light chain of the chimeric antibody or humanized antibody further comprises a light chain constant region derived from human κ, λ chain or a variant thereof, preferably comprised a light chain constant region set forth in SEQ ID NO:39.

In an alternative embodiment, the heavy chain of the humanized LAG-3 antibody comprised in the above pharmaceutical composition is set forth in SEQ ID NO: 42 or has at least 95% sequence identity thereto, and the light chain is set forth in SEQ ID NO: 43 or has at least 95% sequence identity thereto.

In an alternative embodiment, the heavy chain of the LAG-3 antibody comprised in the pharmaceutical composition has at least 95%, 96%, 97%, 98%, 99% or 100% sequence identity to the heavy chain amino acid sequence set forth in SEQ ID NO:42, and the light chain amino acid sequence of the LAG-3 antibody has at least 95%, 96%, 97%, 98%, 99% or 100% sequence identity to the antibody light chain set forth in SEQ ID NO:43.

In one embodiment, the pharmaceutical composition comprises 50 mg/ml LAG3 antibody Hu303-005, and 10 mM histidine-hydrochloric acid buffer, pH 6.0.

In one embodiment, the pharmaceutical composition comprises 50 mg/ml LAG3 antibody Hu303-005, and 10 mM histidine-hydrochloric acid buffer, pH 5.0.

In one embodiment, the pharmaceutical composition comprises 50 mg/ml LAG3 antibody Hu303-005, and 10 mM histidine-hydrochloric acid buffer, pH 6.5.

In yet another embodiment, the pharmaceutical composition comprises 50 mg/ml LAG3 antibody Hu303-005, and 10 mM acetic acid-sodium acetate buffer, pH 5.5.

In yet another embodiment, the pharmaceutical composition comprises 50 mg/ml LAG3 antibody Hu303-005, 10 mM acetic acid-sodium acetate buffer, pH 5.5, 75 mg/ml sucrose, and 0.2 mg/ml polysorbate 80.

In yet another embodiment, the pharmaceutical composition comprises 50 mg/ml LAG3 antibody Hu303-005, 10 mM acetic acid-sodium acetate buffer, pH 5.5, 75 mg/ml trehalose, and 0.2 mg/ml polysorbate 80.

In yet another embodiment, the pharmaceutical composition comprises 50 mg/ml LAG3 antibody Hu303-005, 10 mM acetic acid-sodium acetate buffer, pH 5.5, and 0.4 mg/ml polysorbate 80.

In yet another embodiment, the pharmaceutical composition comprises 50 mg/ml LAG3 antibody Hu303-005, 10 mM histidine-hydrochloric acid buffer, pH 6.0, 75 mg/ml sucrose, and 0.4 mg/ml polysorbate 80.

In yet another embodiment, the pharmaceutical composition comprises 50 mg/ml LAG3 antibody Hu303-005, 10 mM histidine- hydrochloric acid buffer, pH 6.5, 75 mg/ml sucrose, and 0.4 mg/ml polysorbate 80.

In yet another embodiment, the pharmaceutical composition comprises 50 mg/ml LAG3 antibody Hu303-005, 10 mM histidine-hydrochloric acid buffer, pH 5.5, 75 mg/ml sucrose, and 0.4 mg/ml polysorbate 80.

In some embodiments, the pharmaceutical composition comprises 45-60 mg/ml LAG3 antibody Hu303-005, 10 mM histidine-hydrochloric acid buffer, pH 5.5, 75 mg/ml sucrose, and 0.2-0.6 mg/ml polysorbate 80.

In yet another embodiment, the pharmaceutical composition comprises about 50 mg/ml LAG3 antibody Hu303-005, about 10 mM histidine-hydrochloric acid buffer, about pH 6.0, about 75 mg/ml sucrose, and about 0.3 mg/ml polysorbate 80.

The present invention also provides a method of preparing a pharmaceutical composition comprising a LAG-3 antibody, comprising mixing the LAG-3 antibody or antigen-binding fragment with a pharmaceutically acceptable excipient.

The present invention also provides a method of preparing a lyophilized formulation comprising a LAG-3 antibody, which comprises the step of freeze-drying the aforementioned pharmaceutical composition.

In an alternative embodiment of the method of preparing a lyophilized formulation comprising a LAG-3 antibody, the freeze-drying includes the steps of pre-freezing, primary drying, and secondary drying.

In an alternative embodiment of the method of preparing a lyophilized formulation comprising a LAG-3 antibody, primary drying is performed at the temperature of from -5 °C to -20 °C, preferably -10 °C.

The present invention also provides a lyophilized formulation comprising a LAG-3 antibody prepared by the aforementioned method of preparing a lyophilized formulation comprising a LAG-3 antibody.

In some embodiments, the lyophilized formulation maintains its stability at 2-8 °C for at least 3 months, at least 6 months, at least 12 months, at least 18 months, or at least 24 months. In some embodiments, the lyophilized formulation retains its stability at 40 °C for at least 7 days, at least 14 days or at least 28 days.

The present invention also provides a lyophilized formulation comprising a LAG-3 antibody prepared by the above lyophilization method.

The present invention also provides a lyophilized formulation comprising a LAG3 antibody, characterized in that the lyophilized formulation can be reconstituted to obtain the above pharmaceutical composition.

The present invention also provides a method for preparing a reconstituted solution from the lyophilized formulation comprising a LAG-3 antibody, which includes the step of reconstituting the aforementioned lyophilized formulation, wherein the solvent for reconstitution is selected from, but not limited to, water for injection, physiological saline or glucose solution.

The present invention also provides a reconstituted solution obtainable from the lyophilized formulation comprising the LAG-3 antibody, which is prepared by the method for preparing a reconstituted solution from the lyophilized formulation comprising the LAG-3 antibody.

The present invention also provides a reconstituted solution comprising a LAG3 antibody, which further comprises the following components:
(a) 1 to 90 mg/ml LAG-3 antibody or antigen-binding fragment thereof, (b) 5 to 30 mM acetic acid-sodium acetate buffer, pH is about 5.0-6.5, (c) 30 to 90 mg/ml sucrose, and (d) 0.02 to 0.8 mg/ml polysorbate 80.

The present invention also provides a reconstituted solution comprising a LAG3 antibody, which further comprises the following components:
(a) 40 to 80 mg/ml LAG-3 antibody or antigen-binding fragment thereof, (b) 10 to 30 mM acetic acid-sodium acetate buffer, pH is about 5.2-5.8, (c) 70 to 80 mg/ml sucrose, and (d) 0.4 to 0.5 mg/ml polysorbate 80.

The present invention also provides a reconstituted solution comprising a LAG3 antibody, which further comprises the following components:
(a) 50 mg/ml LAG-3 antibody or antigen-binding fragment thereof, (b) 10 mM acetic acid-sodium acetate buffer, pH is about 5.5, (c) 75 mg/ml sucrose, and (d) 0.4 mg/ml polysorbate 80.
(a) 1 to 90 mg/ml LAG-3 antibody or antigen-binding fragment thereof, (b) 5 to 30 mM histidine-hydrochloric acid buffer, pH is about 5.0-6.5, (c) 30 to 90 mg/ml sucrose, and (d) 0.05 to 0.6 mg/ml polysorbate 80.

The present invention also provides a reconstituted solution comprising a LAG3 antibody, which further comprises the following components:
(a) 45 to 60 mg/ml LAG-3 antibody or antigen-binding fragment thereof, (b) 10 to 30 mM histidine-hydrochloric acid buffer, pH is about 5.5-6.0, (c) 60 to 80 mg/ml sucrose, and (d) 0.2 to 0.6 mg/ml polysorbate 80.

The present invention also provides a reconstituted solution comprising a LAG3 antibody, which further comprises the following components:
(a) 50 mg/ml LAG-3 antibody or antigen-binding fragment thereof, (b) 10 mM histidine-hydrochloric acid buffer, pH is about 6.0, (c) 75 mg/ml sucrose, and (d) 0.3 mg/ml polysorbate 80.

The invention further provides an article or kit comprising a container containing any of the stable pharmaceutical compositions described herein. In some embodiments, the vial is an injection vial made of neutral borosilicate glass.

The aforementioned pharmaceutical composition or lyophilized formulation or reconstituted solution of the lyophilized formulation of the present invention can be used as a medicament.

The present invention also provides use of the aforementioned pharmaceutical composition or the lyophilized formulation or the reconstituted solution of the lyophilized formulation for the preparation of a medicament for treating a disease or condition associated with LAG-3, wherein the disease or condition is a disease or condition involving pathogenic T cells, preferably is a cancer. The cancer includes, but not limited to, ovarian cancer, melanoma, prostate cancer, intestinal cancer, gastric cancer, esophageal cancer, breast cancer, lung cancer, kidney cancer, pancreatic cancer, uterine cancer, liver cancer, bladder cancer, cervical cancer, oral cancer, brain cancer, testicular cancer, skin cancer, thyroid cancer, and hematological malignancies, including myeloma and chronic and acute leukemia.

The invention also provides a method of treating and preventing a disease or condition associated with LAG-3, comprising administering to a subject in need thereof a therapeutically effective amount of the aforementioned pharmaceutical composition or the lyophilized formulation or the reconstituted solution of the lyophilized formulation, wherein the disease or condition is a disease or condition involving pathogenic T cells, preferably is a cancer. The cancer includes, but not limited to, ovarian cancer, melanoma, prostate cancer, intestinal cancer, gastric cancer, esophageal cancer, breast cancer, lung cancer, kidney cancer, pancreatic cancer, uterine cancer, liver cancer, bladder cancer, cervical cancer, oral cancer, brain cancer, testicular cancer, skin cancer, thyroid cancer, and hematological malignancies, including myeloma and chronic and acute leukemia.

The present invention also provides an article comprising a container containing the aforementioned pharmaceutical composition or the lyophilized formulation or the reconstituted solution of the lyophilized formulation.

One, some, or all features of the various embodiments described in this disclosure can be further combined to form further embodiments of the invention, as is well known to those skilled in the art. The above embodiments of the invention and other embodiments obtained by combination are further illustrated by the following detailed description.

### DESCRIPTION OF THE DRAWINGS

Figure 1: Humanized anti-LAG-3 antibodies enhance the secretion of IL-2 cytokine from T lymphocytes activated by SEB. The results show that humanized LAG-3 antibody candidates, Hu229-013 and Hu303-005, can enhance the secretion of cytokine IL-2 from the activated T lymphocytes to varying degrees, showing dose-effect dependent on drug concentration.
Figure 2: Effect of humanized anti-LAG-3 antibodies on tumor volume in U-87MG tumor-bearing mice. The results show that, on day 14 after administration, both LAG-3 antibody Hu229-013 6mpk and Hu303-005 6mpk have certain effects on inhibiting tumor, and the tumor inhibition rates were 27.25% (p <0.05) and 34.94% (p <0.01), respectively, and there were significant differences compared to the control group (p<0.001 vs hIGg).
Figure 3: Tendency chart showing the CE purity of antibody Hu229-013 at 40 °C.
Figure 4: Tendency chart showing the IEC neutral peak of antibody Hu229-013 at 40 °C.
Figure 5: Tendency chart showing non-reducing CE of antibody Hu303-005 at 40 °C.
Figure 6: Tendency chart showing iCE main peak of antibody Hu303-005 at 40 °C.
Figure 7: Tendency chart showing shaking SEC results of Hu303-005.
Figure 8: Fitting results showing the difference value between IEC of antibody Hu303-005 at 0°C and IEC of the same at 40°C.
Figure 9: Fitting graphs showing the difference value between CE purity of antibody Hu303-005 at 0°C and CE purity of the same at 40°C.
Figure 10: Fitting results showing iCE/CE/DLS of antibody Hu303-005 formulation at 25°C and at 40°C.

### Terminology

In order to make the invention more readily understood, certain technical and scientific terms are specifically defined below. Unless specifically defined otherwise in this document, all other technical and scientific terms used herein shall be taken to have the same meaning as commonly understood by one of ordinary skill in the art to which this disclosure belongs.

"Buffer" refers to a buffer that is resistant to changes in pH due to its conjugate acid-base component. Examples of the buffer which controls the pH in appropriate range include acetate buffer, succinate buffer, gluconate buffer, histidine buffer, oxalate buffer, lactate buffer, phosphate buffer, citrate buffer, tartrate buffer, fumarate buffer, glycylglycine and other organic acid buffers.

"Histidine buffer" refers to a buffer comprising histidine ions. Examples of histidine buffers include histidine-hydrochloride buffer, histidine-acetate buffer, histidine-phosphate buffer, histidine-sulfate buffer, etc., preferably histidine-hydrochloride buffer. Histidine-hydrochloride buffer is prepared by histidine and hydrochloric acid or by histidine and histidine hydrochloride.

"Citrate buffer" refers to a buffer that includes citrate ions. Examples of the citrate buffer include citric acid-sodium citrate buffer, citric acid-potassium citrate buffer, citric acid-calcium citrate buffer, citric acid-magnesium citrate buffer, etc. A preferred citrate buffer is citric acid-sodium citrate.

"Succinate buffer" refers a buffer that includes succinate ions. Examples of the succinate buffer include succinic acid-sodium succinate buffer, succinic acid-potassium succinate buffer, succinic acid-calcium succinate buffer, etc. A preferred succinate buffer is succinic acid-sodium succinate buffer.

"Phosphate buffer" refers a buffer that includes phosphate ions. Examples of the phosphate buffer include disodium hydrogen phosphate-sodium dihydrogen phosphate buffer, and disodium hydrogen phosphate-potassium dihydrogen phosphate buffer, etc. A preferred phosphate buffer is disodium hydrogen phosphate-sodium dihydrogen phosphate buffer.

"Acetate buffer" refers a buffer that includes acetate ions. Examples of the acetate buffer include acetic acid-sodium acetate buffer, acetic acid-histidine buffer, acetic acid-potassium acetate buffer, acetic acid-calcium acetate buffer, acetic acid-magnesium acetate buffer, etc. A preferred acetate buffer is acetic acid-sodium acetate buffer.

"Tris buffer" refers to a buffer solution comprising tris(hydroxymethyl)aminomethane, also known as Tris base, Trizma, Trisamine, THAM, tromethamine, and trometamol. The effective buffering range of the Tris buffer is between pH 7.0 and 9.2, and the pH of the Tris base aqueous solution is about 10.5. Generally, hydrochloric acid is added to adjust the pH to a desired value to obtain a buffer with said pH.

The "saccharide" of the present invention comprises a conventional composition (CH₂O)ₙ and derivatives thereof, including monosaccharides, disaccharides, trisaccharides, polysaccharides, saccharide alcohols, reducing saccharides, non-reducing saccharides and so forth. It can be selected from the group consisting of glucose, sucrose, trehalose, lactose, fructose, maltose, dextran, glycerol, erythritol, glycerol, arabitol, sylitol, sorbitol, mannitol, melidiose, melezitose, melitriose, mannotriose, stachyose, maltose, lactulose, maltulose, sorbitol, maltitol, lactitol, iso-maltulose, and the like. Preferably, saccharides are non-reducing disaccharides, more preferably sucrose.

"Viscosity modifier" is a conventional pharmaceutical material added to adjust the viscosity of the formulation. The viscosity modifier mentioned herein mainly refers to an inorganic salt and an amino acid salt, wherein the inorganic salt is preferably selected from the group consisting of sodium chloride, calcium chloride, magnesium chloride and calcium acetate, and the amino acid salt is preferably selected from the group consisting of arginine hydrochloride, histidine hydrochloride, glycine hydrochloride, and histidine acetate and the like.

"Pharmaceutical composition" refers to a mixture comprising one or more compounds described herein or the physiologically/pharmaceutically acceptable salt thereof or the prodrug thereof and other chemical components. wherein the other chemical components are, for example, physiological/pharmaceutically acceptable carriers and excipients. The purpose of the pharmaceutical composition is to promote the administration to the organism, which facilitates the absorption of the active ingredient, thereby exerting biological activity. As used herein, "pharmaceutical composition" and "formulation" are not mutually exclusive.

With respect to the solution form of the pharmaceutical composition in the present invention, unless otherwise specified, the solvent included therein is water.

"Lyophilized formulation" refers to a formulation or pharmaceutical composition obtained by vacuum freeze-drying the liquid form of or the solution form of pharmaceutical composition or formulation.

The freeze-drying of the present disclosure includes pre-freezing, primary drying, and secondary drying. The purpose of pre-freezing is to freeze the product to obtain a crystalline solid. The temperature and speed for the pre-freezing are two important process parameters. In the present invention, the temperature for pre-freezing is set as -45°C, and the speed for pre-freezing is set as 1°C/min. The primary drying is also known as main drying, which is the main stage of freeze-drying. The purpose is to remove the ice from the product while maintaining the shape of the product, minimizing damage to the product. If the temperature and vacuum degree for the primary freezing are not appropriate, it will cause the product to collapse. Higher temperature and vacuum degree will accelerate the efficiency of lyophilization, but at the same time increase the risk of product collapse. The temperature for the primary drying of the present invention can be a conventional temperature in the art, for example, from -30°C to 0°C. Secondary drying is also known as analytical drying, which is the primary step to remove bound water from the product by ultimate vacuum (0.01 mbar) and increasing the temperature (20-40°C). Since most biological products are sensitive to temperature, temperature for the secondary drying is chosen to be at the lower point of the temperature range, i.e. 25°C. The duration for freeze-drying is related to the freezer, the dose of the lyophilized formulation, and the container comprising the lyophilized agent. Those skilled in the art well know how to adjust the duration for the freeze-drying.

As used herein, the term "about" refers to a value that is within an acceptable error range for a particular value as determined by one of ordinary skill in the art, which will depend partially on how the value is measured or determined (i.e., the limitation of the measurement system). For example, "about" can indicate a standard deviation within 1 or more than 1 for each practice in the art. Alternatively, "about" or "comprising essentially of" can mean a range of up to 20%. For example, pH of about 5.5 means pH 5.5±1.1. Furthermore, particularly with respect to biological systems or processes, the term can refer to up to an order of magnitude or up to 5-fold of a value. When particular values are mentioned in the application and claims, unless otherwise stated, the meaning of "about" or "comprising essentially of" should be assumed to be within an acceptable error range for that particular value.

The pharmaceutical composition of the present invention is capable of achieving a stable effect: the antibody can substantially maintain its physical stability and/or chemical stability and/or biological activity after storage; preferably, the pharmaceutical composition substantially maintains its physical stability, chemical stability and biological activity after storage. The shelf life is generally selected based on the predetermined shelf life of the pharmaceutical composition. There are currently a number of analytical techniques for measuring protein stability, which can measure the stability after storage for a selected period of time at a selected temperature.

A stable antibody pharmaceutical formulation is the one in which no significant change is observed in the following conditions: storage at a refrigerated temperature (2-8°C) for at least 3 months, preferably 6 months, more preferably 1 year, and even more preferably up to 2 years. In addition, the stable liquid formulation includes a liquid formulation which exhibits a desired characteristics upon storage for example, at a temperature of 25°C for a period of 1 month, 3 months, and 6 months, or at 40°C for 1 month. Typically, acceptable criteria for the stability are as follows: typically, no more than about 5%, preferably no more than about 5% of antibody monomer is degraded, as assessed by SEC-HPLC. The pharmaceutical antibody formulation is colorless or clear to slightly opalescent white by visual analysis. The concentration, pH and osmolality of the formulation have no more than ±5% change. Typically, no more than about 5%, preferably no more than about 5% of truncate is observed. Typically, no more than about 5%, preferably no more than about 5% of aggregation is formed.

An antibody is considered to "maintain its physical stability" in a pharmaceutical formulation, if it shows no significant increase of aggregation, precipitation and/or denaturation upon visual examination of color and/or clarity, or as measured by UV light scattering, size exclusion chromatography (SEC) and dynamic light scattering (DLS). The change of protein conformation can be evaluated by fluorescence spectroscopy (which determines the protein tertiary structure), and by FTIR spectroscopy (which determines the protein secondary structure).

An antibody is considered to "retain its chemical stability" in a pharmaceutical formulation, if it shows no significant chemical alteration. Chemical stability can be assessed by detecting and quantifying chemically altered forms of the protein. Degradation processes that often alter the chemical structure of a protein include hydrolysis or truncation (evaluated by methods such as size exclusion chromatography and SDS-PAGE), oxidation (evaluated by methods such as peptide mapping in conjunction with mass spectroscopy or MALDI/TOF/MS), deamidation (evaluated by methods such as ion-exchange chromatography, capillary isoelectric focusing, peptide mapping, isoaspartic acid measurement), and isomerization (evaluated by measuring the isoaspartic acid content, peptide mapping, etc.).

An antibody is considered to "retain its biological activity" in a pharmaceutical formulation, if the biological activity of the antibody at a given time is within a predetermined range of the biological activity exhibited at the time when the pharmaceutical preparation was prepared. The biological activity of an antibody can be determined, for example, by an antigen binding assay.

The term "LAG-3" refers to Lymphocyte Activation Gene-3. The term "LAG-3" includes variants, isoforms, homologs, orthologs and paralogs. The term "human LAG-3" refers to the sequence of human LAG-3, such as the complete amino acid sequence of human LAG-3 with Uniprot No. P18627. LAG-3 is also known as in the art, for example, CD215. The human LAG-3 sequence can differ from human LAG-3 of Uniprot No. P18627, e.g., the human LAG-3 has conserved mutations or mutations in non-conserved regions and it has substantially the same biological function as that of human LAG-3 of Uniprot No. P18627. For example, a biological function of human LAG-3 consists in that it has an epitope in the extracellular domain of LAG-3, wherein the epitope is specifically bound by the antibodies disclosed herein, or a biological function of human LAG-3 consists in its binding to MHC Class II molecules.

A particular human LAG-3 sequence will generally have at least 90% identity in amino acid sequence to human LAG-3 of Uniprot No. P18627 and contains amino acid residues which are identified as being human amino acid sequences when compared to LAG-3 amino acid sequences from other species (e.g., murine). In certain cases, a human LAG-3 can have at least 85%, or even at least 95%, 96%, 97%, 98%, or 99% identity in amino acid sequence to LAG-3 of Uniprot No. P18627. In certain embodiments, a human LAG-3 sequence will display no more than 10 amino acid differences from the LAG-3 sequence of Uniprot No. P18627. In certain embodiments, the human LAG-3 can display no more than 5, or even no more than 4, 3, 2, or 1 amino acid difference from human LAG-3 sequence of Uniprot No. P18627. Percent identity can be determined as described herein.

The three letter codes and single-letter codes for the amino acid residues used herein are described in J. Biol. Chem. 243, p. 3558 (1968).

The "antibody" as used in the present invention refers to an immunoglobulin, which is a tetra-peptide chain structure connected together by inter-chain disulfide bonds between two identical heavy chains and two identical light chains.

In the present invention, the antibody light chain of the present invention can further comprise a light chain constant region comprising human or murine κ, λ chain or variant thereof.

In the present invention, the antibody heavy chain of the present invention can further comprise a heavy chain constant region comprising human or murine IgG1, IgG2, IgG3, IgG4 or variant thereof.

About 15 amino acid sequences adjacent to the N-terminus of the antibody heavy and light chains are highly variable, known as variable region (Fv region); the rest of amino acid sequences close to the C-terminus are relatively stable, known as constant regions. The variable region includes three hypervariable regions (HVRs) and four relatively conserved framework regions (FRs). The three hypervariable regions which determine the specificity of the antibody are also known as the complementarity determining regions (CDRs). Each light chain variable region (LCVR) and each heavy chain variable region (HCVR) consists of three CDR regions and four FR regions, with sequential order from the amino terminus to carboxyl terminus in the following order: FR1, CDR1, FR2, CDR2, FR3, CDR3, and FR4. The three CDR regions of the light chain refer to LCDR1, LCDR2, and LCDR3, and the three CDR regions of the heavy chain refer to HCDR1, HCDR2, and HCDR3.

The antibody of the present invention includes murine antibody, chimeric antibody or humanized antibody, preferably humanized antibody.

The term "murine antibody" in the present invention refers to a monoclonal antibody against human LAG-3 prepared according to the knowledge and skills of the field. During the preparation, a test subject is injected with LAG-3 antigen, and then a hybridoma expressing the antibody having the desired sequence or functional properties is separated.

The term "chimeric antibody" is an antibody which is formed by fusing the variable region of a murine antibody with the constant region of a human antibody, and the chimeric antibody can alleviate the immune response that is induced by murine antibody. To construct a chimeric antibody, a hybridoma that secretes a specific murine monoclonal antibody is constructed, and then variable region genes are cloned from the mouse hybridoma cells. Subsequently, constant region genes of human antibody are cloned as desired. The murine variable region gene is ligated with the human constant region gene to form a chimeric gene which can be inserted into a human vector, and finally a chimeric antibody molecule is expressed in the eukaryotic or prokaryotic industrial system. In a preferred embodiment of the present invention, the light chain of the LAG-3 chimeric antibody further comprises the light chain constant regions of human κ, λ chain, or variant thereof. The heavy chain of the LAG-3 chimeric antibody further comprises the heavy chain constant regions of human IgG1, IgG2, IgG3, or IgG4, or variant thereof.

The term "humanized antibody", also known as CDR-grafted antibody, refers to an antibody generated by grafting murine CDR sequences into a variable region framework of human antibody, namely, an antibody produced from different types of human germline antibody framework sequences. A humanized antibody overcomes disadvantage of the strong antibody response induced by the chimeric antibody, which carries a lots of murine protein components. Such framework sequences can be obtained from a public DNA database covering germline antibody gene sequences or published references. For example, germline DNA sequences of human heavy and light chain variable region genes can be found in "VBase" human germline sequence database (available on web www.mrccpe.com.ac.uk/vbase), as well as can be found in Kabat, E A, et al, 1991 Sequences of Proteins of Immunological Interest, 5th Ed. To avoid the decrease in activity along with the decrease in immunogenicity, the framework sequences in the variable region of human antibody are subjected to minimal reverse mutations or back mutations to maintain the activity. The humanized antibody of the present invention also comprises humanized antibody on which CDR affinity maturation is performed by phage display.

The terms "anti-LAG-3 antibody", "anti-LAG-3", "LAG-3 antibody" and "antibody binding to LAG-3" in the present invention refer to an antibody that is capable of binding to LAG-3 with sufficient affinity, so that the antibody can be used as a diagnostic agent and/or a therapeutic agent for targeting LAG-3.

The term "binding to LAG-3" in the present invention refers to being capable of interacting with human LAG-3.

The term "specifically binding to" is determined by techniques available in the art, such as competitive ELISA, BIACORE® assay, or KINEXA® assay. For example, the term is also applicable for the case in which the antigen binding domain of the antibody of the invention is specific for a particular epitope carried by many antigens. In such case, the antibody carrying the antigen binding domain can specifically bind to a variety of antigens carrying such epitope.

The term "competitive binding" refers to that an antibody which recognizes the same human LAG-3 extracellular region epitope (also referred to as an antigenic determinant) or a portion thereof as that is recognized by the antibody of the invention, and binds to the antigen. An antibody that binds to the same epitope as that is recognized by the monoclonal antibody of the present invention refers to, an antibody that recognizes and binds to the amino acid sequence of human LAG-3 recognized by the monoclonal antibody of the present invention.

The term "KD" of "Kd" refers to the dissociation equilibrium constant of a particular antibody-antigen interaction. Typically, the antibodies of the invention bind to LAG-3 with a dissociation equilibrium constant (KD) of less than approximately 10⁻⁷ M, for example less than approximately 10⁻⁸ M, 10⁻⁹ M or 10⁻¹⁰ M or even lower, for example, as determined using surface plasmon resonance (SPR) technology in a BIACORE instrument.

"Antigen-binding fragment" mentioned in the present invention refers to Fab fragment, Fab' fragment, or F(ab')2 fragment having antigen-binding activity, as well as scFv fragment binding to human LAG-3, and other fragments capable of binding to human LAG-3 formed by the anti-LAG-3 antibody VH and VL; it comprises one or more CDR regions of antibodies described in the present invention, selected from the group consisting of SEQ ID NO: 9, 10, 11, 15, 16, 17 and 12, 13, 14, 18, 19 and 20. Fv fragment comprises heavy chain variable region and light chain variable region, without constant region, and it is a minimal antibody fragment possessing all antigen-binding sites. Generally, Fv antibody further comprises a polypeptide linker between the VH and VL domains, and is capable of forming a structure necessary for antigen binding. Also, different linkers can be used to connect the variable regions of two antibodies to form a polypeptide chain, referred to as single chain antibody or single chain Fv (scFv).

The term "epitope" refers to a portion located in the antigen that can be recognized and bound by one or more antibodies.

"Conservative modifications" or "conservative replacement or substitution" refers to substitutions of amino acids in a protein with other amino acids having similar characteristics (e.g. charge, side-chain size, hydrophobicity/hydrophilicity, backbone conformation and rigidity, etc.), such that the changes can frequently be made without altering the biological activity of the protein. Those of skilled in this art recognize that, in general, a single amino acid substitution in non-essential regions of a polypeptide does not substantially alter biological activity (see, e.g., Watson et al., (1987) Molecular Biology of the Gene, The Benjamin/Cummings Pub. Co., p. 224 (4th Ed.)). In addition, substitutions for structurally or functionally similar amino acids are less likely to disrupt biological activity.

"Amino acid identity" refers to sequence similarity between two proteins or between two polypeptides. When a position in both of the two sequences to be compared is occupied by the same amino acid residue, e.g., if a position in each of two polypeptides is occupied by identical amino acid residue, the molecules are identical at that position. Examples of algorithms suitable for determining percent sequence identity and percent sequence similarity are the BLAST and BLAST 2.0 algorithms, which are described in Altschul et al., (1990) J. Mol. Biol. 215: 403-45 and Altschul et al., (1977) Nucleic Acids Res. 25:3389-3402, respectively. Software for performing BLAST analyses is publicly available at the National Center of Biotechnology Information (www.ncbi.nlm.nih.gov/).

Methods for producing and purifying antibodies and antigen-binding fragments are well known in the art and can be found, for example, in Antibody Experimental Technology Guide of Cold Spring Harbor, Chapters 5-8 and 15. The antibodies or the antigen-binding fragments of the present invention are genetically engineered to introduce one or more human framework regions (FRs) into a non-human derived CDR region. Human FR germline sequences can be obtained by comparing the IMGT human antibody variable region germline gene database and by using MOE software, from ImMunoGeneTics (IMGT) via their website http://imgt.cines.fr, or from The Immunoglobulin FactsBook, 2001ISBN012441351.

The engineered antibodies or antigen-binding fragments of the present invention can be prepared and purified by conventional methods. For example, cDNA sequences encoding a heavy chain and a light chain can be cloned and recombined into a GS expression vector. The recombined immunoglobulin expression vector can then be stably transfected into CHO cells. As a more recommended method well known in the art, mammalian expression systems will result in glycosylation of antibodies, typically at the highly conserved N-terminus in the Fc region. Stable clones can be obtained through expression of an antibody specifically binding to human LAG-3. Positive clones can be expanded in serum-free culture medium for antibody production in bioreactors. Culture medium, into which an antibody has been secreted, can be purified by conventional techniques. For example, the purification can be conveniently performed by a Protein A or G Sepharose FF column that has been equilibrated with adjusted buffer. The column is washed to remove nonspecific binding components. The bound antibody is eluted by pH gradient and antibody fragments are detected by SDS-PAGE, and then collected. The antibody can be filtered and concentrated using common techniques. Soluble mixture and multimers can also be effectively removed by common techniques, including molecular sieve or ion exchange. The obtained product can be immediately frozen, for example at -70°C, or can be lyophilized.

"Administration" and "treatment", when applying to an animal, human, experimental subject, cell, tissue, organ, or biological fluid, refers to contacting an exogenous pharmaceutical, therapeutic, diagnostic agent, or composition with the animal, human, subject, cell, tissue, organ, or biological fluid. "Administration" and "treatment" can refer, e.g., to therapeutic, pharmacokinetic, diagnostic, research, and experimental methods. Treatment of a cell encompasses contacting a reagent with the cell, as well as contacting a reagent with a fluid, where the fluid is in contact with the cell. "Administration" and "treatment" also mean in vitro and ex vivo treatments, e.g., of a cell, by a reagent, diagnostic, binding compound, or by another cell. "Treatment", as it applies to a human, veterinary, or a research subject, refers to therapeutic treatment, prophylactic or preventative measures, research and diagnostic applications.

"Treat" means to administer a therapeutic agent, such as a composition comprising any of the binding compounds of the present invention, internally or externally to a patient having one or more disease symptoms for which the agent has known therapeutic activity. Typically, the therapeutic agent is administered in an amount effective to alleviate one or more disease symptoms in the treated patient or population, so as to induce the regression or inhibit the progression of such symptom(s) to any clinically measurable degree. The amount of a therapeutic agent that is effective to alleviate any particular disease symptom (also referred to "therapeutically effective amount") can vary according to factors such as the disease state, age, and weight of the patient, health status, behavior, diet of the patient, administration time, administration method, excretion rate, drug combination, and forth on, and the ability of the drug to elicit a desired response in the patient. Whether a disease symptom has been alleviated can be assessed by any clinical measurement typically used by physicians or other skilled healthcare providers to assess the severity or progression status of that symptom. While an embodiment of the present invention (e.g., a treatment method or article of manufacture) can not be effective in alleviating each disease symptom of interest, it should alleviate the target disease symptom(s) of interest in a statistically significant number of patients as determined by any statistical test known in the art such as the Student's t-test, the chi-square test, the U-test according to Mann and Whitney, the Kruskal-Wallis test (H-test), Jonckheere-Terpstra-test and the Wilcoxon-test.

"Effective amount" encompasses an amount sufficient to ameliorate or prevent a symptom or sign of a medical condition. Effective amount also means an amount sufficient to allow or facilitate diagnosis. An effective amount for a particular patient or veterinary subject can vary depending on factors such as the condition being treated, the general health of the patient, the route and dose of administration and the severity of side effects. An effective amount can be the maximal dose or dosing protocol that avoids significant side effects or toxic effects.

"Tm value" refers to the thermal denaturation temperature of the protein, namely, a temperature at which half of the proteins are unfolded and the spatial structure of the protein is destroyed. Therefore, the higher the Tm value is, the higher the thermal stability of the protein will be.

### DETAILED DESCRIPTION OF THE DISCLOSURE

The present invention provides a stable pharmaceutical composition (formulation) comprising a LAG3 antibody or an antigen-binding fragment thereof, acetate buffer or histidine salt, sucrose and polysorbate 80, and the pharmaceutical composition (formulation) is more suitable for administration.

### EXAMPLES

The invention is further illustrated in detail by the following examples. These examples are only provided for illustrative purposes, and are not intended to limit the scope of the invention.

In the examples of the present invention, where specific conditions are not described, the experiments are generally conducted under conventional conditions, or under conditions proposed by the material or product manufacturers. Where the source of the reagents is not specifically given, the reagents are commercially available conventional reagents.

### Example 1. Preparation of LAG-3 Antigen and Antibody

### 1. Protein Design and Expression

UniProt Lymphocyte activation gene 3 protein (human LAG-3, Uniprot: P18627) was used as the template of the LAG-3 herein, and the amino acid sequences of the antigen and the protein used for detection were designed, optionally different labels were fused to the LAG-3 protein and then cloned into pHr vector (produced in-house) or pTT5 vector (Biovector, Cat#: 102762) or pTargeT vector (Promega, A1410). The antigen protein and the detection protein of the present invention were transiently expressed in 293 cells or stably expressed in CHO-S, purified and obtained. The following LAG-3 antigens are referred to human LAG-3 if not specifically indicated.

LAG-3 extracellular domain with a Flag tag: LAG-3-Flag, for immunization of mice. 
MWEAQFLGLLFLQPLWVAPVKPLQPGAEVPVVWAQEGAPAQLPCSPTIPLQDL
SLLRRAGVTWQHQPDSGPPAAAPGHPLAPGPHPAAPSSWGPRPRRYTVLSVGP
GGLRSGRLPLQPRVQLDERGRQRGDFSLWLRPARRADAGEYRAAVHLRDRAL
SCRLRLRLGQASMTASPPGSLRASDWVILNCSFSRPDRPASVHWFRNRGQGRVP
VRESPHHHLAESFLFLPQVSPMDSGPWGCILTYRDGFNVSIMYNLTVLGLEPPTP
LTVYAGAGSRVGLPCRLPAGVGTRSFLTAKWTPPGGGPDLLVTGDNGDFTLRL
EDVSQAQAGTYTCHIHLQEQQLNATVTLAIITVTPKSFGSPGSLGKLLCEVTPVS
GQERFVWSSLDTPSQRSFSGPWLEAQEAQLLSQPWQCQLYQGERLLGAAVYFT
ELSSPGDYKDDDDK
SEQ ID NO:1
 NOTE: Underlined portion represents a signal peptide, and italic portion refers to the Flag-tag sequence.

Full length of LAG-3: used to construct LAG-3 overexpressing cell line, for immunization of mice and detection 
MWEAQFLGLLFLQPLWVAPVKPLQPGAEVPVVWAQEGAPAQLPCSPTIPLQDL
SLLRRAGVTWQHQPDSGPPAAAPGHPLAPGPHPAAPSSWGPRPRRYTVLSVGP
GGLRSGRLPLQPRVQLDERGRQRGDFSLWLRPARRADAGEYRAAVHLRDRAL
SCRLRLRLGQASMTASPPGSLRASDWVILNCSFSRPDRPASVHWFRNRGQGRVP
VRESPHHHLAESFLFLPQVSPMDSGPWGCILTYRDGFNVSIMYNLTVLGLEPPTP
LTVYAGAGSRVGLPCRLPAGVGTRSFLTAKWTPPGGGPDLLVTGDNGDFTLRL
EDVSQAQAGTYTCHIHLQEQQLNATVTLAIITVTPKSFGSPGSLGKLLCEVTPVS
GQERFVWSSLDTPSQRSFSGPWLEAQEAQLLSQPWQCQLYQGERLLGAAVYFT
ELSSPGAQRSGRAPGALPAGHLLLFLILGVLSLLLLVTGAFGFHLWRRQWRPRRF
SALEQGIHPPQAQSKIEELEQEPEPEPEPEPEPEPEPEPEQL
 SEQ ID NO:2 NOTE: Signal peptide + extracellular region + transmembrane region + *intracellular region*

Fusion protein of LAG-3 extracellular region and hIgG1 Fc: LAG-3-Fc, for detection 
MWEAQFLGLLFLQPLWVAPVKPLQPGAEVPVVWAQEGAPAQLPCSPTIPLQDL
SLLRRAGVTWQHQPDSGPPAAAPGHPLAPGPHPAAPSSWGPRPRRYTVLSVGPG
GLRSGRLPLQPRVQLDERGRQRGDFSLWLRPARRADAGEYRAAVHLRDRALSC
RLRLRLGQASMTASPPGSLRASDWVILNCSFSRPDRPASVHWFRNRGQGRVPVR
ESPHHHLAESFLFLPQVSPMDSGPWGCILTYRDGFNVSIMYNLTVLGLEPPTPLT
VYAGAGSRVGLPCRLPAGVGTRSFLTAKWTPPGGGPDLLVTGDNGDFTLRLEDV
SQAQAGTYTCHIHLQEQQLNATVTLAIITVTPKSFGSPGSLGKLLCEVTPVSGQE
RFVWSSLDTPSQRSFSGPWLEAQEAQLLSQPWQCQLYQGERLLGAAVYFTELSS
PGDDDDKGSGSGEPKSCDKTHTCPPCPAPELLGGPSVFLFPPKPKDTLMISRTPEV
TCVVVDVSHEDPEVKFNWYVDGVEVHNAKTKPREEQYNSTYRVVSVLTVLHQDWLN
GKEYKCKVSNKALPAPIEKTISKAKGQPREPQVYTLPPSREEMTKNQVSLTCLVKGFY
PSDIAVEWESNGQPENNYKTTPPVLDSDGSFFLYSKLTVDKSRWQQGNVFSCSVMH
EALHNHYTQKSLSLSPG
SEQ ID NO:3
 NOTE: Underlined portion represents a signal peptide, double underlined portion represents a linker, and the italic portion represents Fc.

Fusion protein of LAG-3 extracellular region and mIgG2a Fc: LAG-3-mFc, for detection 
MWEAQFLGLLFLQPLWVAPVKPLQPGAEVPVVWAQEGAPAQLPCSPTIPLQDL
SLLRRAGVTWQHQPDSGPPAAAPGHPLAPGPHPAAPSSWGPRPRRYTVLSVGPG
GLRSGRLPLQPRVQLDERGRQRGDFSLWLRPARRADAGEYRAAVHLRDRALSC
RLRLRLGQASMTASPPGSLRASDWVILNCSFSRPDRPASVHWFRNRGQGRVPVR
ESPHHHLAESFLFLPQVSPMDSGPWGCILTYRDGFNVSIMYNLTVLGLEPPTPLT
VYAGAGSRVGLPCRLPAGVGTRSFLTAKWTPPGGGPDLLVTGDNGDFTLRLEDV
SQAQAGTYTCHIHLQEQQLNATVTLAIITVTPKSFGSPGSLGKLLCEVTPVSGQE
RFVWSSLDTPSQRSFSGPWLEAQEAQLLSQPWQCQLYQGERLLGAAVYFTELSS
PGDDDDKGSGSGEPRGPTIKPCPPCKCPAPNLLGGPSVFIFPPKIKDVLMISLSPIVT
CVVVDVSEDDPDVQISWFVNNVEVHTAQTQTHREDYNSTLRVVSALPIQHQDWMSG
KEFKCKVNNKDLPAPIERTISKPKGSVRAPQVYVLPPPEEEMTKKQVTLTCMVTDFM
PEDIYVEWTNNGKTELNYKNTEPVLDSDGSYFMYSKLRVEKKNWVERNSYSCSVVHE
GLHNHHTTKSFSRTPGK
 SEQ ID NO:4 NOTE: Underlined portion represents a signal peptide, double underlined portion represents a linker, and the italic portion represents mFc.

### 2. Purification of LAG-3-related recombinant protein, as well as hybridoma antibody, and recombinant antibody

### 1). Purification steps for LAG-3-Flag recombinant protein with a Flag tag

The samples were centrifuged at a high speed to remove impurities and concentrated to an appropriate volume. The flag affinity column was equilibrated with 0.5 × PBS and washed with 2-5 column volumes. The supernatants expressed by cells were loaded onto the column after removing the impurities. The column was washed with 0.5 × PBS until the A280 reading was reduced to the baseline. The column was washed with PBS, and the impurity proteins were washed off and then the target protein was collected. The target protein was eluted with 100mM glycine, pH 3.0 and collected for further activation and purification *in vitro.*

### 2). Purification for hybridoma, recombinant antibody and Fc fusion protein

The supernatants expressed by cells were centrifuged at a high speed to remove impurities, supernatant expressed by hybridoma was purified by Protein G column, recombinant antibody and Fc fusion protein expressing supernatants were purified by Protein A column. The column was washed with PBS, until the A280 reading was reduced to baseline. The target protein was eluted with 100 mM acetic acid (pH 3.0) and neutralized with 1 M Tris-HCl, pH 8.0. The eluted sample was properly concentrated and further purified using gel chromatography Superdex200 (GE), which has been equilibrated with PBS, the peaks representing the aggregate were excluded, and the samples were collected and aliquoted for use.

### Example 2. Preparation of Anti-human LAG-3 hybridoma monoclonal antibody

### 1. Immunization

The anti-human LAG-3 monoclonal antibodies were produced by immunizing mice. Experimental SJL white mice, female, 6-week old (Beijing Charles River Lab Animal Technology Co., Ltd., animal production license number: SCXK (Beijing) 2012-0001). Feeding environment: SPF level. After the mice were purchased, the animals were kept in the laboratory for 1 week, with 12/12-hour light/dark cycle, at temperature of 20-25°C, and with a humidity of 40-60%. The mice that had been adapted to the environment were immunized according to the following schemes. Immune antigen was extracellular region of LAG-3 with Flag tag (SEQ ID NO: 1).

Scheme A: Mice were cross-immunized with TiterMax® Gold Adjuvant (Sigma Cat No: T2684) and Thermo Imject® Alum (Thremo Cat No: 77161). The ratio of antigen to adjuvant (TiterMax® Gold Adjuvant) was 1:1, and the ratio of antigen to adjuvant (Thermo Imject® Alum) was 3:1, with a dose of 50µg/mouse (first immunization) and 25µg/mouse (booster immunization). After the antigen was emulsified, the mice were inoculated on day 0, 7, 14, 21, 28, 35 and 42. On day 0, the mice were, on several sites, subcutaneously (s.c.) injected with emulsified antigen, 50µg/mouse. On day 7, the mice were intraperitoneally (i.p.) injected with 25µg/mouse. On days 14, 28, 35 and 42, either back or intraperitoneal injection of antigen was selected according to the lumps on the back and the swelling conditions in abdomen. Blood samples were collected on days 21, 35, 49, and antibody titers in mouse serum were determined by ELISA. After 7 immunizations, mice with higher serum antibody titer which was tending to be a platform were selected for splenocyte fusion. A booster immunization was performed by i.p. injection of antigen solution formulated with saline, 50µg/mouse, 3 days prior to splenocyte fusion.

Scheme B: Mice were immunized with QuickAntibody-Mouse5W (KX0210041). The ratio of antigen to adjuvant was 1:1, 25µg/mouse once (first immunization/booster immunization). The antigen and adjuvant were rapidly mixed and used for inoculation on days 0, 21 and 35. On day 0, mice were injected with antigens via posterior calf muscles (i.m.), 25µg/mouse, On days 21 and 35, injection was repeated in the same way, 25µg/mouse (whether the third immunization was performed or not is dependent on antibody titer). Blood samples were collected on days 28 and 42. The antibody titer in mouse serum was determined by ELISA. Mice with higher serum antibody titer which was tending to a platform were selected for splenocyte fusion. A booster immunization was performed by i.p. injection of antigen solution formulated with saline, 50µg/mouse, 3 days prior to splenocyte fusion.

### 2. Splenocyte Fusion

Hybridoma cells were obtained by fusing splenic lymphocytes with myeloma Sp2/0 cells (ATCC® CRL-8287™) by using an optimized PEG-mediated fusion procedure. The fused hybridoma cells obtained were re-suspended in a complete medium (DMEM medium containing 20% FBS, 1 x HAT and 1 x OPI) at a density of 0.5-1 x 10⁶/ml, and seeded in 96-well cell culture plates, 100µl/well. After incubation at 37°C, 5%CO₂, for 3-4 days, 100µl/well of the HAT complete medium was supplemented and the culture was maintained for 3-4 days to form needle-like clones. The supernatants were removed and 200µl/well of HT complete medium (RPMI-1640 medium containing 20% FBS, 1×HAT, 1×OPI) was added, cultured at 5%CO₂, 37°C for three days and then detected by ELISA assay.

### 3. Screening Hybridoma Cells

Hybridoma culture supernatants were detected by binding ELISA according to the growth density of hybridoma cells. Cell-blocking experiments were performed on cell supernatants in positive wells detected by binding ELISA. Cells which were positive both for binding and blocking experiments were expanded and cryopreserved quickly, and the cells were subcloned twice to three times until a single cell clone was obtained.

After each subcloning procedure, the cells were subjected to LAG-3 binding ELISA and cell blocking assay. The hybridoma clones were obtained by the above screening experiments, and the antibodies were further prepared by serum-free cell culture method, and then purified according to purification example for use in the test example.

### 4. Sequencing of the positive hybridoma clone

The process of cloning sequences of the positive hybridoma was as follows: Collecting the hybridoma cells at logarithmic growth phase, and extracting RNA with Trizol (Invitrogen Cat No. 15596-018) according to the manufacturer's instructions, and then performing reverse transcription with the PrimeScript™ Reverse Transcriptase kit (Takara, Cat No. 2680A). The cDNAs obtained by reverse transcription were amplified by PCR using the mouse Ig-Primer Set (Novagen, TB326 Rev.B 0503) and sequencing was performed by a sequencing company. The heavy chain and light chain amino acid sequences corresponding to DNA sequences of hybridoma clone mAb229 are shown in SEQ ID NOs: 5, 6 and SEQ ID NOs: 7, 8, respectively.
mAb229-VH 
QIQLVQSGPELKKPGETVKISCKASGYTFTTSGMSWVKQAPGKGLKWMGWINT
YSGVPTYADDFKGRFAFSLETSASTAYLQINNLKNEDTATYFCARDNYDARDVY
YYAMDYWGQGTSVTVSS
SEQ ID NO: 5
mAb229-VL 
DIQMTQSPASLSVSVGETVTITCRASENIYSNLAWYQQKQGKSPQLLVYAATNL
ADGVPSRFSGSGSGTQYSLKINSLQSEDFGSYYCQHFWITPWTFGGGTKLEIK
SEQ ID NO: 6
mAb303-VH 
EVQLQQSGPVLVKPGASVKMSCKASGYTLTDYYMNWVKQSHGKSLEWIGVIN
PYNGDTAYNQKFKGKATLTVDKSSNTAYMEINSLTSEDSAVYYCTRDDGYYDY
YFDVWGTGTTVTVSS
SEQ ID NO: 7
mAb303-VL

DIQMTQSPSSLSASLGERVILTCRASQDIGSRLNWLQQGPDGTFKRLIYATSTLDS
GVPKRFSGSRSGSDFSLTISSLESEDFVDYYCLQLASSPPTFGGGTKLEIK
SEQ ID NO: 8.

**Table 1. CDR region sequence of each heavy and light chain**

| | Heavy chain | | Light chain | |
|---|---|---|---|---|
| mAb229 | HCDR1 | TSGMS SEQ ID NO: 9 | LCDR1 | RASENIYSNLA SEQ ID NO: 15 |
| | HCDR2 | WINTYSGVPTYADDFKG SEQ ID NO: 10 | LCDR2 | AATNLAD SEQ ID NO: 16 |
| | HCDR3 | DNYDARDVYYYAMDY | LCDR3 | QHFWITPWT |
| | | SEQ ID NO: 11 | | SEQ ID NO: 17 |
| mAb303 | HCDR1 | DYYMN SEQ ID NO: 12 | LCDR1 | RASQDIGSRLN SEQ ID NO: 18 |
| | HCDR2 | VINPYNGDTAYNQKFKG SEQ ID NO: 13 | LCDR2 | ATSTLDS SEQ ID NO: 19 |
| | HCDR3 | DDGYYDYYFDV SEQ ID NO: 14 | LCDR3 | LQLASSPPT SEQ ID NO: 20 |

The obtained positive clones were subjected to ELISA assay for the binding to human LAG-3 (the results of EC50 value for the protein binding activity are shown in Table 2), ELISA assay for the binding to human LAG-3 overexpressing CHO-s cells (the results of EC50 values for the cell binding activity are shown in Table 2), and an assay for the blocking of the binding between LAG-3 antigen and Daudi cells (the results of EC50 value for blocking activity are shown in Table 2), and assay for the affinity to human LAG-3 protein (results are shown in Table 3).

**Table 2. In vitro activity of murine LAG-3 antibody**

| **Candidate antibody** | **Protein binding activity EC50(nM)** | **Cell binding activity EC50(nM)** | **Blocking activity IC50 (nM)** |
|---|---|---|---|
| mAb229 | 0.129 | 0.191 | 1.327 |
| mAb303 | 0.172 | 0.279 | 0.596 |

**Table 3. Affinity of murine LAG-3 antibody**

| Stationary phase | Mobile phase | Affinity(M) |
|---|---|---|
| mAb229 | LAG-3-Flag | 4.26E-10 |
| mAb303 | LAG-3-Flag | 4.70E-10 |

The results shown in Table 2 demonstrate that both LAG-3 antibody mAb229 and mAb303 showed excellent binding activity to human LAG-3 protein. LAG-3 antibody mAb229 and mAb303 also showed excellent binding activity to CHO-S cells overexpressing full-length of human LAG-3 protein. Both LAG-3 antibody mAb229 and mAb303 significantly blocked the binding of human LAG-3 antigen with Daudi cells.

The results shown in table 3 demonstrate that LAG-3 antibody mAb229 and mAb303 of the present invention showed a stronger binding activity and affinity to human LAG-3 protein.

### Example 3. Humanization of murine anti-human LAG-3 hybridoma monoclonal antibody mAb229

Through aligning IMGT human antibody heavy and light chain variable region germline gene database against MOE software, the heavy and light chain variable region germline genes with high homology to mAb229 were selected as templates, the CDRs derived from murine antibodies were grafted into the corresponding human source template to form a variable region sequence in an order of FR1-CDR1-FR2-CDR2-FR3-CDR3-FR4. The amino acid residues were identified and annotated according to Kabat Numbering System.

### 1. Selection of a framework for humanization of hybridoma clone mAb229

The light chain template for humanizing murine antibody mAb229 is IGKV1-39*01 and hjk4.1, and the heavy chain template for humanization is IGHV7-4-1*01 and hjh6.1, the sequences of humanized variable region are as follows:
Hu229VH-CDR graft 
QVQLVQSGSELKKPGASVKVSCKASGYTFTTSGMSWVRQAPGQGLEWMGWINTYS
GVPTYADDFKGRFVFSLDTSVSTAYLQISSLKAEDTAVYYCARDNYDARDVYYYAM
DYWGQGTTVTVSS
SEQ ID NO: 21
Hu229VL-CDR graft 
DIQMTQSPSSLSASVGDRVTITCRASENIYSNLAWYQQKPGKAPKLLIYAATNLADG
VPSRFSGSGSGTDFTLTISSLQPEDFATYYCQHFWITPWTFGGGTKVEIK
SEQ ID NO: 22
 NOTE: The order is FR1-CDR1-FR2-CDR2-FR3-CDR3-FR4, italic sequence represents FR sequence, and underlined sequence represents CDR sequence.

### 2. Template selection and back-mutation design for hybridoma clone mAb229, see Table 4 below:

**Table 4. Template selection and back mutation design for mAb229**

| **Hu229_VL** | | **Hu229_VH** | |
|---|---|---|---|
| Hu229_VL.1 | **Grafted** | Hu229_VH.1 | **Grafted** |
| Hu229_VL.1A | 148V, F71Y | Hu229_VH.1A | E46K |
| Hu229_VL.1B | D70Q, F71Y, I48V | Hu229_VH.1B | E46K, R38K, V93T |
| Hu229_VL.1C | D70Q, F71Y, I48V, A43S | Hu229_VH.1C | E46K, R38K, V93T, Y95F |

NOTE: For example, I48V denotes a back mutation from I to V at position 48 according to Kabat numbering system. Grafted indicates that the murine antibody CDR was implanted into human germline FR sequences.

**Table 5: Sequence combination for humanization of murine antibody mAb229**

| | **Hu229_VL.1** | **Hu229_VL.1A** | **Hu229_VL.1B** | **Hu229_VL.1C** |
|---|---|---|---|---|
| Hu229_VH.1 | Hu229-004 | LF 229-005 | Hu229-006 | Hu229-007 |
| Hu229_VH.1A | Hu229-008 | Hu229-009 | Hu229-010 | Hu229-011 |
| Hu229_VH.1B | Hu229-012 | Hu229-013 | Hu229-014 | Hu229-015 |
| Hu229_VH.1C | Hu229-016 | Hu229-017 | Hu229-018 | Hu229-019 |

NOTE: This table shows various sequence combinations of different mutations. For example, Hu229-005 indicates that two mutations (light chain HumAb229_VL.1A and heavy chain HumAb229_VH.1) are present in the humanized murine antibody Hu229-005, and the rest can be explained in the same manner.

Sequences of humanized antibody mAb229 are as follows:
Hu229VH.1 (identical to Hu229VH-CDR graft) 
QVQLVQSGSELKKPGASVKVSCKASGYTFTTSGMSWVRQAPGQGLEWMGWIN
TYSGVPTYADDFKGRFVFSLDTSVSTAYLQISSLKAEDTAVYYCARDNYDARDV
YYYAMDYWGQGTTVTVSS SEQ ID NO: 21
Hu229VH.1A 
QVQLVQSGSELKKPGASVKVSCKASGYTFTTSGMSWVRQAPGQGLKWMGWIN
TYSGVPTYADDFKGRFVFSLDTSVSTAYLQISSLKAEDTAVYYCARDNYDARDV
YYYAMDYWGQGTTVTVSS SEQ ID NO: 23
Hu229VH.1B 
QVQLVQSGSELKKPGASVKVSCKASGYTFTTSGMSWVKQAPGQGLKWMGWI
NTYSGVPTYADDFKGRFVFSLDTSVSTAYLQISSLKAEDTATYYCARDNYDARD
VYYYAMDYWGQGTTVTVSS SEQ ID NO: 24
Hu229VH.1C 
QVQLVQSGSELKKPGASVKVSCKASGYTFTTSGMSWVKQAPGQGLKWMGWI
NTYSGVPTYADDFKGRFVFSLDTSVSTAYLQISSLKAEDTATYFCARDNYDARD
VYYYAMDYWGQGTTVTVSS SEQ ID NO: 25
Hu229VL.1 (identical to Hu229VL-CDR graft) 
DIQMTQSPSSLSASVGDRVTITCRASENIYSNLAWYQQKPGKAPKLLIYAATNLA
DGVPSRFSGSGSGTDFTLTISSLQPEDFATYYCQHFWITPWTFGGGTKVEIK
SEQ ID NO: 22
Hu229VL.1A 
DIQMTQSPSSLSASVGDRVTITCRASENIYSNLAWYQQKPGKAPKLLVYAATNLA
DGVPSRFSGSGSGTDYTLTISSLQPEDFATYYCQHFWITPWTFGGGTKVEIK
SEQ ID NO: 26
Hu229VL.1B 
DIQMTQSPSSLSASVGDRVTITCRASENIYSNLAWYQQKPGKAPKLLVYAATNLA
DGVPSRFSGSGSGTQYTLTISSLQPEDFATYYCQHFWITPWTFGGGTKVEIK
SEQ ID NO: 27
Hu229VL.1C

DIQMTQSPSSLSASVGDRVTITCRASENIYSNLAWYQQKPGKSPKLLVYAATNLA
DGVPSRFSGSGSGTQYTLTISSLQPEDFATYYCQHFWITPWTFGGGTKVEIK
SEQ ID NO: 28

### Example 4. Humanization of murine anti-human LAG-3 hybridoma monoclonal antibody mAb303

Through aligning IMGT human antibody heavy and light chain variable region germline gene database against MOE software, the heavy and light chain variable region germline genes with high homology to mAb303 were selected as templates, the CDRs derived from murine antibodies were grafted into the corresponding human source template to form a variable region sequence in an order of FR1-CDR1-FR2-CDR2-FR3-CDR3-FR4. The amino acid residues were identified and annotated according to the Kabat Numbering System.

### 1. Selection of a framework for humanization of hybridoma clone mAb303

The light chain template for humanizing murine antibody mAb303 is IGKV1-39*01 and hjk4.1, and the heavy chain template for humanization is IGHV1-3*01 and hjh6.1, the sequences of humanized variable region are as follows:
Hu303VH-CDR graft 
QVQLVQSGAEVKKPGASVKVSCKASGYTFTDYYMNWVRQAPGQRLEWMGVINPY
NGDTAYNQKFKGRVTITRDTSASTAYMELSSLRSEDTAVYYCARDDGYYDYYFDVW
GQGTTVTVSS
SEQ ID NO: 29
Hu303VL-CDR graft 
DIQMTQSPSSLSASVGDRVTITCRASQDIGSRLNWYQQKPGKAPKLLIYATSTLDSGV
PSRFSGSGSGTDFTLTISSLQPEDFATYYCLQLASSPPTFGGGTKVEIK
SEQ ID NO: 30
NOTE: The order is FR1-CDR1-FR2-CDR2-FR3-CDR3-FR4, italic sequence represents FR sequence, and the underlined sequence represents CDR sequence. 2. Template selection and back-mutation design of hybridoma clone mAb303, see Table 6 below:

**Table 6. Back mutations for humanization of hybridoma clone mAb303**

| **Hu303_VL** | | **Hu303_VH** | |
|---|---|---|---|
| Hu303_VL.1 | **Grafted** | Hu303_VH.1 | **Grafted** |
| Hu303_VL.1A | L46R, G66R | Hu303_VH.1A | R72V, T74K, A97T |
| Hu303_VL.1B | L46R, G66R, S60K | Hu303_VH.1B | R72V, T74K, A97T, F29L |
| Hu303_VL.1C | L46R, G66R, S60K, P44F, Y36L | Hu303_VH.1C | R72V, T74K, F29L, A97T, M48I, V68A, I70L |
| Hu303_VL.1D | L46R, G66R, S60K, P44F, Y36L, K42G, I21L, T85D | | |

NOTE: For example, L46R denotes a back mutation from L to R at position 46 according to Kabat numbering system. Grafted indicates that the murine antibody CDR was implanted into human germline FR sequences.

Sequence combinations of different mutations are as follows:

**Table 7: Sequence combinations for humanization of murine antibody mAb303**

| | Hu303_VL. 1 | Hu303_VL.1 A | Hu303_VL.1 B | Hu303_VL.1 C | Hu303_VL.1 D |
|---|---|---|---|---|---|
| Hu303_VH.1 | Hu303-004 | Hu303-005 | Hu303-006 | Hu303-007 | Hu303-008 |
| Hu303_VH.1A | Hu303-009 | Hu303-010 | Hu303-011 | Hu303-012 | Hu303-013 |
| Hu303_VH.1B | Hu303-014 | Hu303-015 | Hu303-016 | Hu303-017 | Hu303-018 |
| Hu303_VH.1C | Hu303-019 | Hu303-020 | Hu303-021 | Hu303-022 | Hu303-023 |

NOTE: This table shows various sequence combinations of different mutations. For example, Hu303-005 indicates that two mutations (light chain HumAb303_VL.1A and heavy chain HumAb303_VH.1) are present on the humanized murine antibody Hu303-005, and the rest can be explained in the same manner.

Sequences of humanized antibody mAb303 are as follows:
Hu303_VH.1 (identical to Hu303VH-CDR graft) 
QVQLVQSGAEVKKPGASVKVSCKASGYTFTDYYMNWVRQAPGQRLEWMGVI
NPYNGDTAYNQKFKGRVTITRDTSASTAYMELSSLRSEDTAVYYCARDDGYYD
YYFDVWGQGTTVTVSS SEQ ID NO: 29
Hu303_VH.1A 
QVQLVQSGAEVKKPGASVKVSCKASGYTFTDYYMNWVRQAPGQRLEWMGVI
NPYNGDTAYNQKFKGRVTITVDKSASTAYMELSSLRSEDTAVYYCTRDDGYYD
 YYFDVWGQGTTVTVSS SEQ ID NO: 31
Hu303_VH.1B 
QVQLVQSGAEVKKPGASVKVSCKASGYTLTDYYMNWVRQAPGQRLEWMGVI
NPYNGDTAYNQKFKGRVTITVDKSASTAYMELSSLRSEDTAVYYCTRDDGYYD
YYFDVWGQGTTVTVSS SEQ ID NO: 32
Hu303_VH.1C 
QVQLVQSGAEVKKPGASVKVSCKASGYTLTDYYMNWVRQAPGQRLEWIGVIN
PYNGDTAYNQKFKGRATLTVDKSASTAYMELSSLRSEDTAVYYCTRDDGYYDY
YFDVWGQGTTVTVSS SEQ ID NO: 33
Hu303_VL.1 (identical to Hu303VL-CDR graft) 
DIQMTQSPSSLSASVGDRVTITCRASQDIGSRLNWYQQKPGKAPKLLIYATSTLD
SGVPSRFSGSGSGTDFTLTISSLQPEDFATYYCLQLASSPPTFGGGTKVEIK
SEQ ID NO: 30
Hu303_VL.1A 
DIQMTQSPSSLSASVGDRVTITCRASQDIGSRLNWYQQKPGKAPKRLIYATSTLD
SGVPSRFSGSRSGTDFTLTISSLQPEDFATYYCLQLASSPPTFGGGTKVEIK
SEQ ID NO: 34
Hu303_VL.1B 
DIQMTQSPSSLSASVGDRVTITCRASQDIGSRLNWYQQKPGKAPKRLIYATSTLD
SGVPKRFSGSRSGTDFTLTISSLQPEDFATYYCLQLASSPPTFGGGTKVEIK
SEQ ID NO: 35
Hu303_VL.1C 
DIQMTQSPSSLSASVGDRVTITCRASQDIGSRLNWLQQKPGKAFKRLIYATSTLD
SGVPKRFSGSRSGTDFTLTISSLQPEDFATYYCLQLASSPPTFGGGTKVEIK
SEQ ID NO: 36
Hu303_VL.1D

DIQMTQSPSSLSASVGDRVTLTCRASQDIGSRLNWLQQKPGGAFKRLIYATSTLD
SGVPKRFSGSRSGTDFTLTISSLQPEDFADYYCLQLASSPPTFGGGTKVEIK
SEQ ID NO: 37

### Example 5. Recombination and Preparation of humanized antibody

The antibody was constructed with constant region derived from human heavy chain IgG4/light chain kappa in combination with each variable region, and a S228P mutation was made in Fc to increase the stability of the IgG4 antibody. The other mutations known in the art can also be used to increase its performance.

Heavy chain constant region:

ASTKGPSVFPLAPCSRSTSESTAALGCLVKDYFPEPVTVSWNSGALTSGVHTFPA
VLQSSGLYSLSSVVTVPSSSLGTKTYTCNVDHKPSNTKVDKRVESKYGPPCPPCP
APEFLGGPSVFLFPPKPKDTLMISRTPEVTCVVVDVSQEDPEVQFNWYVDGVEV
HNAKTKPREEQFNSTYRVVSVLTVLHQDWLNGKEYKCKVSNKGLPSSIEKTISK
AKGQPREPQVYTLPPSQEEMTKNQVSLTCLVKGFYPSDIAVEWESNGQPENNYK
TTPPVLDSDGSFFLYSRLTVDKSRWQEGNVFSCSVMHEALHNHYTQKSLSLSLG
K SEQ ID NO: 38

Light chain constant region:



SEQ ID NO: 39

The heavy chain amino acid sequences of Hu229-013:

QVQLVQSGSELKKPGASVKVSCKASGYTFTTSGMSWVKQAPGQGLKWMGWI
NTYSGVPTYADDFKGRFVFSLDTSVSTAYLQISSLKAEDTATYYCARDNYDARD
VYYYAMDYWGQGTTVTVSSASTKGPSVFPLAPCSRSTSESTAALGCLVKDYFPE
PVTVSWNSGALTSGVHTFPAVLQSSGLYSLSSVVTVPSSSLGTKTYTCNVDHKPS
NTKVDKRVESKYGPPCPPCPAPEFLGGPSVFLFPPKPKDTLMISRTPEVTCVVVD
VSQEDPEVQFNWYVDGVEVHNAKTKPREEQFNSTYRVVSVLTVLHQDWLNGK
EYKCKVSNKGLPSSIEKTISKAKGQPREPQVYTLPPSQEEMTKNQVSLTCLVKGF
YPSDIAVEWESNGQPENNYKTTPPVLDSDGSFFLYSRLTVDKSRWQEGNVFSCS
VMHEALHNHYTQKSLSLSLGK SEQ ID NO: 40

The light chain amino acid sequences of Hu229-013:

DIQMTQSPSSLSASVGDRVTITCRASENIYSNLAWYQQKPGKAPKLLVYAATNLA
DGVPSRFSGSGSGTDYTLTISSLQPEDFATYYCQHFWITPWTFGGGTKVEIKRTV
AAPSVFIFPPSDEQLKSGTASVVCLLNNFYPREAKVQWKVDNALQSGNSQESVT
EQDSKDSTYSLSSTLTLSKADYEKHKVYACEVTHQGLSSPVTKSFNRGEC
SEQ ID NO: 41

The heavy chain amino acid sequences of Hu303-005:

QVQLVQSGAEVKKPGASVKVSCKASGYTFTDYYMNWVRQAPGQRLEWMGVI
NPYNGDTAYNQKFKGRVTITRDTSASTAYMELSSLRSEDTAVYYCARDDGYYD

YYFDVWGQGTTVTVSSASTKGPSVFPLAPCSRSTSESTAALGCLVKDYFPEPVT
VSWNSGALTSGVHTFPAVLQSSGLYSLSSVVTVPSSSLGTKTYTCNVDHKPSNTK
VDKRVESKYGPPCPPCPAPEFLGGPSVFLFPPKPKDTLMISRTPEVTCVVVDVSQ
EDPEVQFNWYVDGVEVHNAKTKPREEQFNSTYRVVSVLTVLHQDWLNGKEYK
CKVSNKGLPSSIEKTISKAKGQPREPQVYTLPPSQEEMTKNQVSLTCLVKGFYPS
DIAVEWESNGQPENNYKTTPPVLDSDGSFFLYSRLTVDKSRWQEGNVFSCSVMH
EALHNHYTQKSLSLSLGK SEQ ID NO: 42

The light chain amino acid sequences of Hu303-005:

DIQMTQSPSSLSASVGDRVTITCRASQDIGSRLNWYQQKPGKAPKRLIYATSTLD
SGVPSRFSGSRSGTDFTLTISSLQPEDFATYYCLQLASSPPTFGGGTKVEIKRTVAA
PSVFIFPPSDEQLKSGTASVVCLLNNFYPREAKVQWKVDNALQSGNSQESVTEQ
DSKDSTYSLSSTLTLSKADYEKHKVYACEVTHQGLSSPVTKSFNRGEC
SEQ ID NO: 43

### 1. Molecular cloning of the recombinant antibody

The sequences of variable region coding gene were obtained by sequencing the positive antibody molecules obtained from hybridoma screening. The primers were designed according to the obtained sequence, the sequencing gene was used as template, and various antibody VH/VK gene fragments were constructed by PCR, and then reconstituted with the expression vector pHr (with a signal peptide and hIgG4/hkappa constant region (CH1-FC/CL) fragment) by homologous recombination, to construct an expression plasmid VH-CH1-FC-pHr/VL-CL-pHr for full-length recombinant antibody.

### 2. Molecular cloning of humanized antibody

The designed humanized antibody sequence was subjected to codon optimization, and a coding sequence having human codon preference was generated. Primers were designed and various VH/VK gene fragments of the antibodies were constructed by PCR, and reconstituted with the expression vector pHr (with a signal peptide and hIgG4/hkappa constant region (CH1-FC/CL) fragment) by homologous recombination, to construct an expression plasmid VH-CH1-FC-pHr/VL-CL-pHr for full-length humanized antibody.

### 3. Expression and purification of recombination and humanized antibody

The plasmids for separate expression of antibody light chain and heavy chain were co-transfected into HEK293E cell at a ratio of 1: 1.2. The expression supernatant was collected after 6 days and impurities were removed by high-speed centrifugation and then purified by Protein A column. The column was washed with PBS until the A280 reading was reduced to the baseline. The target protein was eluted with acidic elution buffer, pH 3.0-pH 3.5, and neutralized with 1 M Tris-HCl, pH 8.0-9.0. The eluent was properly concentrated and further purified by gel chromatography Superdex200 (GE) which had been equilibrated with PBS. The peaks representing the aggregate were excluded, and the single peak was collected and aliquoted for use.

The performance and benefits of the antibodies of the present invention were verified by biochemical test methods as below.

### Example 6. ELISA assay for the binding of LAG-3 antibody to human LAG-3 protein

The binding ability of anti-LAG-3 antibody to human LAG-3 protein was detected by ELISA assay. LAG-3 fusion protein with Fc or mFc tag was immobilized into 96-well microtiter plate by binding to anti-Fc or anti-mFc antibody coated on the microtiter plate, the strength of the signal after the addition of the antibody was used to determine the binding activity of the antibody to LAG-3, the specific experimental method is as follows.

The goat anti-human Fc antibody (Jackson Immuno Research, Cat No. 109-005-008) or goat anti-mouse Fc antibody (Sigma, Cat No. M3534-1ML) was diluted to a concentration of 2µg/ml with PBS buffer at pH 7.4 (Sigma, Cat No. P4417-100TAB), and added to a 96-well plate at a volume of 50µl/well and then, the plate was incubated in the incubator at 37°C for 2 hours. After discarding the liquid, the plates were blocked with 200µl/well of blocking solution containing 5% skim milk (Bright Dairy, skim milk powder) in PBS, and incubated in the incubator at 37°C for 2.5 hours or overnight at 4°C (16-18 hours). After blocking, the blocking solution was discarded and the plate was washed 5 times with PBST buffer (PH7.4 PBS containing 0.05% tween-20). LAG-3-Fc fusion protein (SEQ ID NO:3, produced in-house) or LAG-3-mFc fusion protein (SEQ ID NO: 4, produced in-house) was diluted with sample diluent (PH7.4 PBS containing 1%BSA) to 1µg/ml and was added to each well, 50µl/well. Then the plate was incubated in the incubator at 37°C for 1h or overnight at 4°C. After incubation, the reaction solution in the plate was discarded, and the plate was washed with PBST for 6 times, and then was added with 50µl/well of various concentrations of antibodies to be tested (hybridoma purified antibody or humanized antibody) which have been diluted with sample diluent, and the plate was incubated at 37°C for 1h. The plates was washed 5 times with PBST after incubation, and was added with 100µl/well of goat anti-mouse (Jackson Immuno Research, Cat No. 115-035-003) or goat anti-human secondary antibody (Jackson Immuno Research, Cat No. 109-035-003) labeled with HRP, diluted in sample diluent, and the plate was incubated at 37°C for 1h. After washing the plates 6 times with PBST, 50µl/well of TMB chromogenic substrate (KPL, Cat No. 52-00-03) was added to each well, and incubated at room temperature for 5-15 min, the reaction was stopped by the addition of 50µl/well 1M H2SO4 to each well. The OD value at a wavelength of 450nm was read on NOVOStar microplate reader, and then EC50 values of the binding of LAG-3 antibody to human LAG-3 were calculated. The results are shown in Table 8. The data showed that all the humanized antibodies obtained by the screening method in the present invention showed excellent binding activities to human LAG-3 protein.

**Table 8. Determination of EC50 value of candidate antibody in Binding Assay**

| **Candidate Antibody** | **Binding ELISA EC50(nM)** |
|---|---|
| mAb229 | 0.129 |
| Hu229-008 | 0.506 |
| Hu229-009 | 0.152 |
| Hu229-010 | 0.174 |
| Hu229-011 | 0.201 |
| Hu229-012 | 0.268 |
| Hu229-013 | 0.106 |
| Hu229-014 | 0.153 |
| Hu229-015 | 0.156 |
| Hu229-016 | 0.154 |
| Hu229-017 | 0.048 |
| Hu229-019 | 0.068 |
| mAb303 | 0.172 |
| Hu303-004 | 0.278 |
| Hu303-005 | 0.309 |
| Hu303-006 | 0.288 |
| Hu303-007 | 0.135 |
| Hu303-008 | 0.140 |
| Hu303-009 | 0.316 |
| Hu303-010 | 0.137 |
| Hu303-011 | 0.314 |
| Hu303-012 | 0.164 |
| Hu303-013 | 0.166 |
| Hu303-014 | 0.232 |
| Hu303-015 | 0.172 |
| Hu303-016 | 0.161 |
| Hu303-017 | 0.168 |
| Hu303-018 | 0.244 |
| Hu303-019 | 0.277 |
| Hu303-020 | 0.140 |
| Hu303-021 | 0.170 |
| Hu303-022 | 0.145 |
| Hu303-023 | 0.152 |

### Example 7. Binding assay of LAG-3 antibody with human LAG-3 over-expressing CHO-S cells

The binding ability of anti-LAG-3 antibody to LAG-3 protein over-expressing CHO-S cells was detected by binding assay. The full-length LAG-3 plasmid (produced in-house, SEQ ID NO: 2) was transfected into CHO-S cells by electroporation, and the expression level of LAG-3 was detected after two weeks of screening under stress. The LAG-3 over-expressing cells were fixed to the bottom of the 96-well plate, and the strength of the signal after the addition of the antibody was used to determine the binding activity of the antibody to LAG-3 over-expressing CHO-S cells, the specific experimental method is as follows.

100µl/well of cells were seeded into 96-well plate at a density of 4 x 10⁵/ml and incubated overnight. The supernatant was discarded, and the plate was washed three times with PBS, and was fixed with 4% PFA (100µl/well) for half an hour at room temperature, and then the plate was washed three times with PBS. After discarding the liquid, the plate was blocked with 200µl/well of blocking solution containing 5% skim milk (Bright Dairy, skim milk powder) diluted in PBS, and incubated at 37°C for 2.5 hours. After blocking, the blocking solution was discarded and the plate was washed 5 times with PBST buffer (PH7.4 PBS containing 0.05% tween-20), added with 50µl/well of various concentrations of antibodies to be tested (Hybridoma purified antibody or humanized antibody) which have been diluted with sample diluent, and then incubated in incubator at 37°C for 1h. The plate was washed 5 times with PBST after incubation, added with 100µl/well of goat anti-mouse (Jackson Immuno Research, Cat No. 115-035-003) or goat anti-human secondary antibody (Jackson Immuno Research, Cat No. 109-035-003) labeled with HRP, diluted in sample diluent, and the plate was incubated at 37°C for 1h. After washing the plates 6 times with PBST, 50µl of TMB chromogenic substrate (KPL, Cat No. 52-00-03) was added to each well, and incubated at room temperature for 5-15 min, the reaction was stopped by the addition of 50µl 1M H2SO4 to each well. The OD value at a wavelength of 450nm was read on NOVOStar microplate reader, and then the EC50 values of the binding of LAG-3 antibody to LAG-3 over-expressing CHO-S cell were calculated.

### Example 8. Assay for the anti-LAG-3 antibody in blocking the binding of LAG-3 antigen to Daudi cells

Daudi cells (human leukemia cells, purchased from the cell bank in Chinese Academy of Sciences) were seeded in 96-well plate with a density of 3 x 10⁵/well. After centrifugation at 1000 rpm, the supernatant was discarded and then the plate was fixed with 4% PFA for 30min at room temperature. The plate was washed 4 times with PBS after discarding the fixed solution, and the plate was blocked with 200µl/well of blocking solution containing 5% skim milk (Bright Dairy, skim milk powder) diluted in PBS, and incubated at 37°C for 2.5 hours. After blocking, the blocking solution was discarded and the plate was washed 5 times with PBST buffer (pH7.4 PBS containing 0.05% tween-20), added with 50µl/well mixture of biotin-labeled (Biotin labeling kit, Dojindo Chemical, Cat No. LK03) LAG-3-Fc fusion protein (produced in-house, SEQ ID NO: 3) and gradient concentrations of the antibody to be tested, wherein the biotin-labeled LAG-3-Fc fusion protein has been diluted with sample diluent (PH7.4 PBS containing 1% BSA) at a final concentration of 0.4µg/ml, and pre-mixed for an hour, then the plate was incubated at 37°C for 1h. The reaction solution was discarded and the plate was washed 5 times with PBST after incubation, 50µl/well of HRP-labeled Streptavidin (Sigma,Cat No. S2438) diluted with sample diluent was added, and the plate was incubated at 37°C for 1h. After washing the plate 5 times with PBST, 50µl/well of TMB chromogenic substrate (KPL, Cat No. 52-00-03) was added to each well, and incubated at room temperature for 5-15 min, the reaction was stopped by the addition of 50µl 1M H₂SO₄ to each well. The OD value at a wavelength of 450nm was read on NOVOStar microplate reader, and then the activity of the LAG-3 antibody in blocking the binding of the antigen to Daudi cells was calculated. The results are shown in Table 9. The data shows that all of the humanized antibodies obtained by the screening method in the present invention significantly blocked the binding of human LAG-3 antigen to Daudi cells.

**Table 9 Determination of IC50 value of candidate antibodies in Blocking Assay**

| **Candidate Antibody** | **Binding assay IC50 (nM)** |
|---|---|
| mAb229 | 1.327 |
| Hu229-009 | 0.559 |
| Hu229-010 | 0.453 |
| Hu229-011 | 0.566 |
| Hu229-013 | 0.39 |
| Hu229-014 | 0.718 |
| Hu229-015 | 0.808 |
| Hu229-016 | 0.875 |
| Hu229-017 | 0.239 |
| Hu229-019 | 0.289 |
| mAb303 | 0.596 |
| Hu303-004 | 0.502 |
| Hu303-005 | 0.622 |
| Hu303-006 | 0.821 |
| Hu303-007 | 0.343 |
| Hu303-008 | 0.346 |
| Hu303-009 | 0.417 |
| Hu303-010 | 0.346 |
| Hu303-011 | 0.728 |
| Hu303-012 | 0.361 |
| Hu303-013 | 0.347 |
| Hu303-014 | 0.467 |
| Hu303-015 | 0.398 |
| Hu303-016 | 0.395 |
| Hu303-017 | 0.398 |
| Hu303-018 | 0.608 |
| Hu303-019 | 0.471 |
| Hu303-020 | 0.345 |
| Hu303-021 | 0.456 |
| Hu303-022 | 0.360 |
| Hu303-023 | 0.369 |

### Example 9. BIAcore assay for the affinity of LAG-3 antibody

1. The mouse anti-capture antibody was covalently linked to the CM5 biochip (Cat. # BR-1000-12, GE) according to the method suggested in the instruction of the mouse anti-capture kit (Cat. #BR-1008-38, GE), so that the antibodies to be tested were captured via affinity. Then, the LAG-3-Flag antigen (produced in-house, SEQ ID NO:1) was flowed through the surface of the biochip, and the reaction signal was detected in real time by using a Biacore instrument to obtain the binding and dissociation curves, the value of affinity was obtained by fitting, see above table 2. After each cycle of dissociation was finished in the experiment, the biochip was washed and regenerated with a regeneration solution provided in the mouse anti-capture kit. The results demonstrate that the LAG-3 antibody mAb229 and mAb303 showed excellent binding activity and affinity to human LAG-3 protein.
2. The human anti-capture antibody was covalently linked to the CM5 biochip (Cat. # BR-1000-12, GE) according to the method suggested in the instruction of the human anti-capture kit (Cat. # BR-1008-39, GE), so that the antibodies to be tested were captured via affinity. Then, the LAG-3-Flag antigen (produced in-house, SEQ ID NO:1) was flowed through the surface of the biochip, and the reaction signal was detected real time using a Biacore instrument to obtain the binding and dissociation curves, the value of affinity was obtained by fitting, see table 10 below. After each cycle of dissociation was finished in the experiment, the biochip was washed and regenerated with a regeneration solution provided in the human anti-capture kit. The results demonstrate that the antibodies obtained by the screening method of present invention showed excellent binding activity and affinity to human LAG-3 protein.

**Table10. Affinity of anti-LAG-3 antibody**

| Stationary phase | Mobile phase | Affinity(M) |
|---|---|---|
| mAb229 | LAG-3-Flag | 1.72E-11 |
| Hu229-009 | | 4.88E-11 |
| Hu229-010 | | 3.82E-11 |
| Hu229-013 | | 2.81E-11 |
| Hu229-014 | | 3.74E-11 |
| Hu229-015 | | 4.59E-11 |
| Hu229-017 | | 6.71E-11 |
| Hu229-019 | | 7.29E-11 |
| mAb303 | | 7.49E-11 |
| Hu303-004 | | 1.06E-09 |
| Hu303-005 | | 7.15E-11 |
| Hu303-006 | | 7.53E-11 |
| Hu303-009 | | 9.43E-10 |
| Hu303-010 | | 1.47E-10 |
| Hu303-014 | | 4.91E-10 |
| Hu303-016 | | 7.48E-11 |

### Example 10. Activation of PBMC-T lymphocytes

In order to study the effect of LAG-3 antibody on activating T lymphocytes, human peripheral blood mononuclear cells (PBMCs) were collected and purified. The secretion level of IL-2 cytokines was measured after stimulating with super-antigen of Staphylococcus aureus enterotoxin B (SEB) in vitro for 72h. The experimental process is briefly described below:
Freshly isolated and purified PBMCs were seeded into 96-well cell culture plate at a cell density of about 1 x 10⁵/well, and 100ng/ml SEB super-antigen stimulus was added, and gradiently diluted antibody samples (diluted with medium) or medium as a blank control were added at the same time. The plate was incubated at 37°C, 5% CO₂ for 72h, the cell culture supernatant was collected. The level of the secreted IL-2 in the culture supernatant was measured by ELISA (BD, CAT # 550611). Detailed procedures are indicated in the manufacturers' manual.

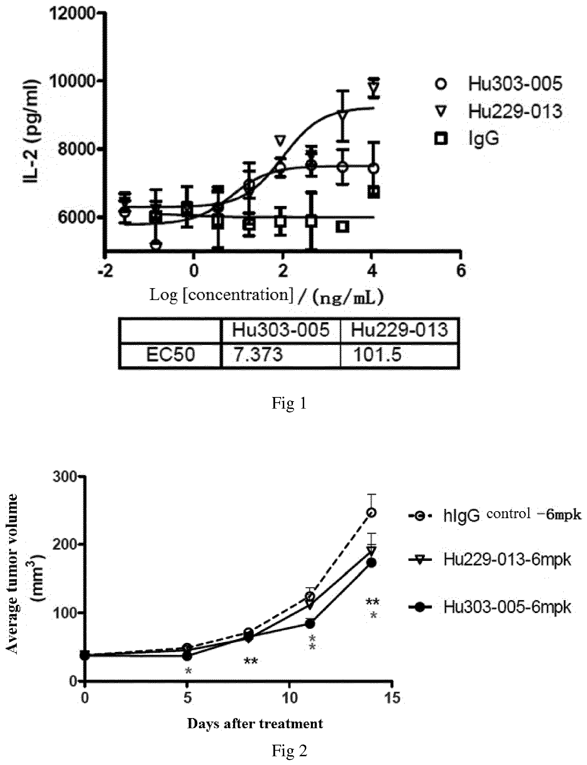

The result was shown in Figure 1. Both humanized LAG-3 antibodies Hu229-013 and Hu303-005 can enhance the levels of cytokine IL-2 secreted by the activated T lymphocytes to different degree, with dose-effect dependent on drug concentration.

### Example 11. Inhibition of subcutaneously inoculated U-87MG tumor by LAG-3 antibody

In this study, the effect of humanized anti LAG-3 antibody on the tumor volume of U-87 MG tumor bearing mice was measured.

100µl of human glioma U87 MG cells (3.5×10⁶ cells) were inoculated subcutaneously in right ribs of NOD-SCID mice (Purchased from Changzhou Cavion Experimental Animal Co., Ltd.). When the tumor grew to 40 mm³ after 10 to 14 days, the mice, excluding those with too large or too small body weight or tumor volume, were randomly divided into three groups: a control group of Isotype matched hIgG, a group of humanized LAG-3 candidate antibody Hu229-013, and a group of humanized LAG-3 candidate antibody Hu303-005, according to the tumor volume (Grouping and dosage are indicated in Table 11), each group of 8 mice (D0). The PBMCs stimulated by CD3 antibody were injected into the tumor tissues at 5×10⁵ cells/60 µl, and injection of antibodies to be tested was started via i.p. injection, three times a week for total of 6 times. Mice were measured for tumor volume twice a week, data were recorded. Tumor volume (V) was calculated as: $Tumor volume \left(TV\right) = 1 / 2 \times {L}_{long} \times {{L}_{short}}^{2} ,$

The tumor volume of each group was expressed as mean ± standard error (Mean ± SEM), and plotted with Graphpad Prism 5 software, analyzed with two way ANOVA statistical analysis, and the tumor inhibition rate was calculated according to the following formula: $Tumor proliferation rate \left(T/C%\right) = \left(T-{T}_{0}/C-{C}_{0}\right) \times 100 %$ $Tumor inhibition rate % TGI = 1 - T / C %$

The results were shown in table 11 and figure 2. Both LAG-3 antibody Hu229-013 6mpk and Hu303-005 6mpk have certain anti-tumor effect 14 days after administration, and the tumor inhibition rates were 27.25% (p <0.05) and 34.94% (p <0.01), respectively. There was a significant difference compared to control group (p <0.001 vs hIGg).

**Table11. Effect of humanized anti-LAG-3 antibody on subcutaneously inoculated U-87MG tumor in Mice.**

| **Group** | **Dose(mpk)** | **Day 0 Mean ± SEM (mm³)** | **Day 14 Mean ± SEM (mm³)** | **P (vs hIgG)** | **% TGI at Day14** |
|---|---|---|---|---|---|
| hIgG control | 6 | 37.9±2.6 | 247.1±26.5 | - | - |
| Hu229-013 | 6 | 37.9±2.5 | 190.1±26.2* | <0.05 | 27.25% |
| Hu303-005 | 6 | 37.7±2.4 | 173.5±26.5** | <0.01 | 34.94% |

| | | | | | |
|---|---|---|---|---|---|
| Note: D0: First administration; * p<0.05, ** p<0.01, ***p<0.001 vs hIGg by two way ANOVA. | | | | | |

### Example 12. PK assay of humanized anti-LAG-3 antibody Hu229-013 and Hu303-005 in mouse

Eighteen ICR male mice, weighing from 18 to 22g, were purchased from the Sippr-BK Lab Animal Co., Ltd. During the feeding period, the mice were access to water and diet *ad libitum,* the mice were adapted to the laboratory environment for no less than 3 days, with 12/12 hour light/dark cycle regulation, at the temperature of 16-26°C and relative humidity of 40-70%. ICR mice were numbered and randomly divided into different groups one day before the experiment, each group of 3 mice. On the day of the experiment, two groups of mice were injected intravenously with humanized candidate antibody (Hu229-013) at dose of 3 mg/kg and 10 mg/kg, respectively; The other two groups of mice were injected intravenously with humanized candidate antibody (Hu303-005) at dose of 3 mg/kg and 10 mg/kg, respectively. The volume for intravenous injection is of 20 ml/kg.

The blood samples were collected at time point of 15min, 8h, 1d, 2d, 4d, 7d, 10d, 14d, 21d, 28d, and 35d after administration. Each time about 0.1ml of whole blood was taken into the centrifuge tube without anticoagulant, placed at 4°C for 30min, and then centrifuged at 1000g for 15 min. The supernatant was pipetted into EP tube and stored at -80°C.

The serum concentration of drug was measured by ELISA, and the T1/2 and other main parameters were calculated by Winnolin software. The main pharmacokinetic parameters are shown in Table 12:

**Table 12. Pharmacokinetic parameters of Hu229-013 and Hu303-005 in mice**

| | Hu229-013 | | Hu303-005 | |
|---|---|---|---|---|
| Dosage (mg/kg) | 3mg/kg | 10mg/kg | 3mg/kg | 10mg/kg |
| tₘₐₓ(hour) | 0.25 | 0.25 | 0.25 | 0.25 |
| Cₘₐₓ(ug/ml) | 51.6±1.2 | 130±20.2 | 68.2±8.4 | 243.2±19.9 |
| AUC ₀₋ₜ (ug/ml*h) | 5556±891 | 17120±4177 | 6386±453 | 22609±1567 |
| AUC _{0- ∞} (ug/ml*h) | 5871±1036 | 19736±6142 | 7124±581 | 27061±5154 |
| t_{1/2}(h) | 183±54 | 276±193 | 232±24 | 330±194 |
| CLz/F(ml/min/kg) | 0.0087±0.0015 | 0.0092±0.0034 | 0.007±0.0006 | 0.0063±0.0011 |
| Vz/F(ml/kg) | 134±16 | 186±107 | 141±14 | 168±66 |
| MRT _{0-∞} (h) | 241±59 | 353±191 | 324±37 | 411±181 |

The *in vivo* exposure of humanized LAG-3 antibodies Hu229-013 and Hu303-005 in mice were similar, and the exposure amount and peak concentrations of these two antibodies at the dose of 3 mg/kg and 10mg/kg were linearly correlated with the increasing dose, showing linear pharmacokinetic characteristic.

### Exemplary process for preparation of antibody pharmaceutical compositions (formulations)

Step 1: Passing stock solution of a formulation comprising LAG-3 antibody through a 0.22 µm PVDF filter, sampling the filtrate for sterility test, and collecting the filtrate.

Step 2: Adjusting loading volume to 5.3 ml, loading the filtrate into a 6 ml stoppered vial; and detecting the volume differences by sampling at the beginning of, during and at the end of the loading procedure, respectively.

Step 3: Capping an aluminum cap by using a capping machine.

Step 4: Performing visual inspection to confirm whether there is any defect such as inaccurate loading. Printing and pasting a label onto the vial. Printing a label for a paper tray, folding a paper tray and placing the vials into the paper tray, and pasting the label onto the paper tray.

### Exemplary process for preparation of antibody pharmaceutical compositions

Step 1: Passing stock solution of a formulation comprising Hu303-005 through a 0.22 µm PVDF filter, sampling the filtrate for sterility test, and collecting the filtrate.

Step 2: Adjusting loading volume to 5.3 ml, loading the filtrate into a 20 ml vial, pressing a plug half into the vial and freeze-dying the stock solution, and sealing the vial with the rubber plug.

Step 3: Capping an aluminum cap by using a capping machine.

Step 4: Performing visual inspection to confirm whether there is any defect such collapse during freezing. Printing and pasting a label onto the vial. Printing a label for a paper tray, folding a paper tray and placing the vials into the paper tray, and pasting the label onto the paper tray.

### Example 13. Screening buffer system for LAG-3 antibody formulation

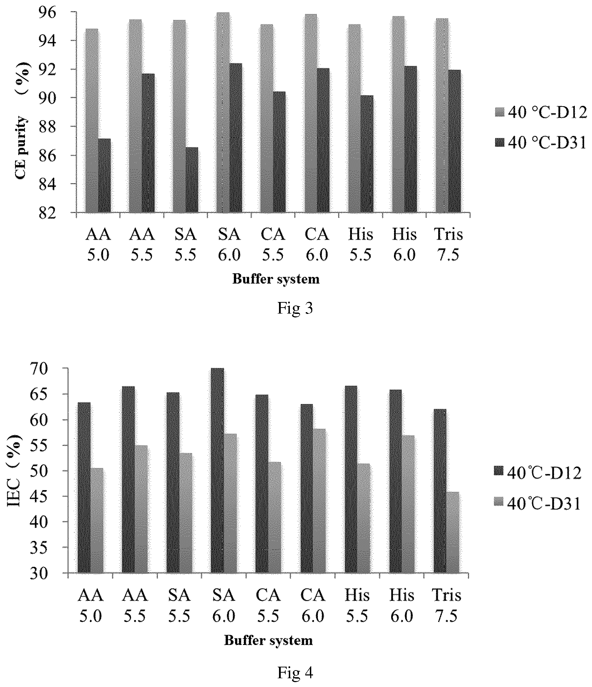

LAG-3 antibody Hu229-013 or Hu303-005 formulations were prepared in a series of lOmM buffers, pH 5.0-7.5, at a protein concentration of 50 mg/mL, and each formulation was filtered and added into a stoppered vial, and the vial was capped and sealed. The samples were subjected to forced degradation testing such as at 40°C high temperature, shaking, and were evaluated by appearance, size exclusion chromatography (SEC), non-reducing sodium dodecyl sulfate (CE-SDS)-capillary electrophoresis and ion exchange chromatography (IEC), or capillary isoelectric focusing electrophoresis-whole column imaging detection (iCIEF). The results are shown in Table 13-1 and Table 13-2, and the results of statistical analysis are shown in Figure 3 to Figure 6.

**Table 13-1. Screening results for Hu229-013 buffer system**

| buffer/pH | condition | appearance | SEC% monomer | Non-re ducing CE% | IEC% | | |
|---|---|---|---|---|---|---|---|
| | | | | | acid | neutr al | alkali ne |
| Acetic acid-sodiu m acetate (AA)/5.0 | D0 | clear, containing a few particles | 99.3 | 97.1 | 10.4 | 78.2 | 11.4 |
| | 40°C D12 | clear, containing a few filamentous particles | 99.1 | 94.9 | 13.9 | 63.4 | 22.8 |
| | 40°C D31 | N/A | 98.5 | 87.2 | 20.6 | 50.5 | 28.9 |
| | Shaking D6 | cloudy | 99.0 | 96.7 | 10.9 | 75.8 | 13.3 |
| | Shaking D12 | clear, containing a few small particles | 98.8 | 97.0 | 11.0 | 75.0 | 13.9 |
| Acetic acid-sodiu m acetate / 5.5 | D0 | clear, containing a few particles | 99.3 | 97.2 | 10.5 | 78.4 | 11.1 |
| | 40°C D12 | clear, containing a few filamentous particles | 99.1 | 95.5 | 15.4 | 66.5 | 18.1 |
| | 40°C D31 | N/A | 98.6 | 91.7 | 24.6 | 55.0 | 20.4 |
| | Shaking D6 | cloudy | 99.0 | 96.6 | 10.9 | 76.2 | 12.8 |
| | Shaking D12 | opalescent, containing a few small particles | 99.0 | 97.0 | 11.2 | 75.7 | 13.1 |
| Succinic acid-sodiu m succinate (SA)/ 5.5 | D0 | clear, containing a few particles | 99.3 | 97.1 | 12.6 | 76.3 | 11.2 |
| | 40°C D12 | much more filamentous particles | 99.0 | 95.5 | 15.5 | 65.2 | 19.3 |
| | 40°C D31 | N/A | 98.5 | 86.6 | 24.1 | 53.4 | 22.5 |
| | Shaking D6 | cloudy, containing filamentous large particles or precipitate | 99.1 | 96.6 | 11.0 | 76.1 | 12.8 |
| | Shaking D12 | clear, containing a few small particles | 99.1 | 97.0 | 11.3 | 75.1 | 13.7 |
| Succinic acid-sodiu m succinate /6.0 | D0 | clear, containing a few particles | 99.3 | 97.2 | 13.0 | 76.2 | 10.8 |
| | 40°C D12 | clear, containing a few filamentous particles | 99.0 | 96.0 | 15.6 | 70.2 | 14.3 |
| | 40°C D31 | N/A | 98.4 | 92.4 | 27.5 | 57.2 | 15.3 |
| | Shaking D6 | cloudy, containing filamentous large particles or precipitate | 99.0 | 96.6 | 11.2 | 76.3 | 12.6 |
| | Shaking D12 | much more small particles | 99.2 | 97.0 | 11.3 | 76.4 | 12.3 |
| Citric acid-sodiu m citrate (CA)/ 5.5 | D0 | clear, containing a few particles | 99.3 | 97.1 | 13.1 | 76.3 | 10.6 |
| | 40°C D12 | much more filamentous particles | 99.0 | 95.2 | 15.4 | 64.9 | 19.7 |
| | 40°C D31 | N/A | 98.2 | 90.4 | 24.2 | 51.7 | 24.1 |
| | Shaking D6 | cloudy, containing filamentous large particles or precipitate | 99.2 | 96.7 | 11.0 | 76.0 | 13.0 |
| | Shaking D12 | cloudy, small particle precipitate | 99.2 | 96.9 | 11.2 | 75.1 | 13.7 |
| Citric acid-sodiu m citrate / 6.0 | D0 | clear, containing a few particles | 99.3 | 97.3 | 12.8 | 76.8 | 10.4 |
| | 40°C D12 | much more filamentous particles | 99.0 | 95.9 | 14.6 | 63.0 | 22.3 |
| | 40°C D31 | N/A | 98.7 | 92.1 | 25.7 | 58.2 | 16.0 |
| | Shaking D6 | cloudy, containing filamentous large particles or precipitate | 99.0 | 96.8 | 11.1 | 76.3 | 12.5 |
| | Shaking D12 | cloudy, small particle precipitate | 99.0 | 97.0 | 14.0 | 73.8 | 12.1 |
| Histidine-hydrochlo ric acid (His-HCl) /5.5 | D0 | clear, containing a few particles | 99.3 | 96.7 | 10.5 | 78.3 | 11.1 |
| | 40°C D12 | much more filamentous particles | 99.1 | 95.1 | 16.9 | 66.6 | 16.5 |
| | 40°C D31 | N/A | 98.7 | 90.2 | 21.7 | 51.4 | 26.9 |
| | Shaking D6 | cloudy | 99.0 | 96.7 | 10.7 | 76.3 | 12.9 |
| | Shaking D12 | much more small particles | 99.2 | 96.9 | 11.0 | 75.7 | 13.3 |
| Histidine-hydrochlo ric acid/6.0 | D0 | clear, containing a few particles | 99.3 | 96.4 | 10.3 | 79.2 | 10.5 |
| | 40°C D12 | much more filamentous particles | 99.1 | 95.7 | 16.6 | 65.8 | 17.6 |
| | 40°C D31 | N/A | 98.8 | 92.0 | 25.7 | 57.0 | 17.3 |
| | Shaking D6 | cloudy, containing filamentous large particles or precipitate | 99.1 | 96.8 | 10.9 | 77.2 | 11.9 |
| | Shaking D12 | much more small particles | 99.2 | 96.9 | 11.5 | 76.3 | 12.2 |
| Tris 7.5 | D0 | clear, containing a few particles | 99.1 | 96.4 | 11.0 | 79.8 | 9.2 |
| | 40°C D12 | much more filamentous particles | 98.8 | 95.6 | 27.9 | 62.0 | 10.1 |
| | 40°C D31 | N/A | 98.0 | 92.0 | 45.9 | 45.9 | 8.3 |
| | Shaking D6 | cloudy, containing filamentous large particles or precipitate | 99.1 | 96.7 | 12.7 | 77.6 | 9.7 |
| | Shaking D12 | cloudy, small particle precipitate | 99.0 | 97.0 | 14.0 | 76.7 | 9.3 |

Note: 0.01 mg/ml polysorbate 80 was added to prepare Shaking D12 sample, and the other samples did not contain polysorbate 80; D indicates day.

**Table 13-2. Screening results for Hu303-005 buffer system**

| buffer/pH | condition | appearance | iCIEF neutral peak% | Non-reducin g CE-SDS% |
|---|---|---|---|---|
| Acetic acid-sodium acetate(AA) 5.0 | D0 | clear | 55.7 | 97.28 |
| | 40°C D11 | N/A | 26.0 | 92.57 |
| | Shaking D3 | clear | N/A | N/A |
| Acetic acid-sodium acetate5.5 | D0 | clear | 57.0 | 97.63 |
| | 40°C D11 | N/A | 32.9 | 94.27 |
| | Shaking D3 | a few particles | N/A | N/A |
| Succinic acid-sodium succinate (SA) 5.0 | D0 | clear | 56.9 | 97.48 |
| | 40°C D11 | N/A | 19.6 | 90.11 |
| | Shaking D3 | clear, opalescent | N/A | N/A |
| Succinic acid-sodium succinate 5.5 | D0 | clear, opalescent | 55.2 | 97.23 |
| | 40°C D11 | N/A | 29.0 | 92.11 |
| | Shaking D3 | lots of particles | N/A | N/A |
| Succinic acid-sodium succinate 6.0 | D0 | clear | 59.1 | 97.41 |
| | 40°C D11 | N/A | 37.7 | 94.55 |
| | Shaking D3 | lots of particles | N/A | N/A |
| Histidine-hydrochlori c acid (His-HCl) 5.5 | D0 | clear | 55.8 | 97.11 |
| | 40°C D11 | N/A | 25.5 | 91.31 |
| | Shaking D3 | clear | N/A | N/A |
| Histidine-hydrochlori c acid 6.0 | D0 | clear | 59.9 | 97.41 |
| | 40°C D11 | N/A | 37.3 | 95.38 |
| | Shaking D3 | lots of fine particles | N/A | N/A |
| Histidine-hydrochlori c acid 6.5 | D0 | clear | 57.0 | 97.63 |
| | 40°C D11 | N/A | 45.9 | 94.38 |
| | Shaking D3 | lots of fine particles | N/A | N/A |
| Citric acid-sodium citrate (CA) 5.5 | D0 | clear, opalescent | 55.2 | 97.64 |
| | 40°C D11 | N/A | 24.5 | 90.57 |
| | Shaking D3 | Transparent filament, protein precipitate | N/A | N/A |
| Citric acid-sodium | D0 | clear, opalescent | 56.6 | 97.05 |
| citrate 6.0 | 40°C D11 | N/A | 36.4 | 93.53 |
| | Shaking D3 | a few particles | N/A | N/A |
| Citric acid-sodium citrate 6.5 | D0 | clear, opalescent | 55.4 | 97.48 |
| | 40°C D11 | N/A | 43.3 | 94.64 |
| | Shaking D3 | a few particles | N/A | N/A |
| Sodium hydrogen phosphate-disodium hydrogen phosphate (PB) 6.0 | D0 | lots of particles | 54.9 | 97.30 |
| | 40°C D11 | N/A | 36.8 | 95.16 |
| | Shaking D3 | lots of fine particles | N/A | N/A |
| Sodium hydrogen phosphate-disodium hydrogen phosphate 6.5 | D0 | lots of particles | 55.2 | 97.28 |
| | 40°C D11 | N/A | 47.0 | 94.07 |
| | Shaking D3 | lots of fine particles | N/A | N/A |
| Sodium hydrogen phosphate-disodium hydrogen phosphate 7.0 | D0 | lots of particles | 56.4 | 97.69 |
| | 40°C D11 | N/A | 46.5 | 93.71 |
| | Shaking D3 | lots of fine particles | N/A | N/A |

| | | | | |
|---|---|---|---|---|
| Note: D indicates day; N/A means not detected. | | | | |

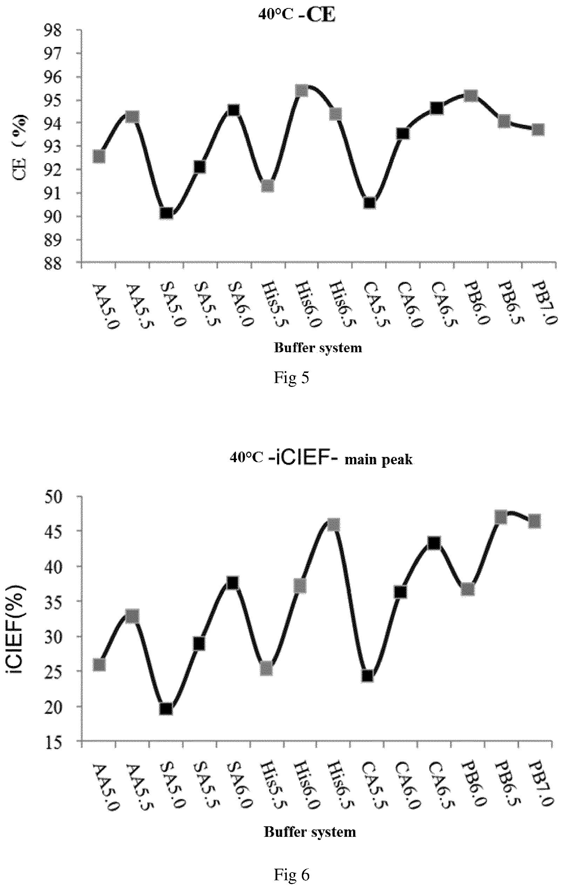

The results showed:
(1) Antibody Hu229-013 exhibited the best appearance in acetic acid-sodium acetate (AA) system, followed by succinic acid-sodium succinate (SA) and histidine-hydrochloric acid (His-HCl) system. 40°C CE, IEC purity was higher in acetic acid-sodium acetate (AA), pH5.5, succinic acid-sodium succinate (SA), pH6.0, citric acid-sodium citrate (CA), pH 6.0, histidine-hydrochloric acid (His), pH 6.0 system. Considering the appearance and CE, IEC results, LAG-3 antibody Hu229-013 was relatively stable in AA (pH 5.5), SA (pH 6.0), His-HCl (pH 6.0) system, see Figure 3 and Figure 4.
(2) The shaking appearance data of Antibody Hu303-005 showed that the appearance was better when the pH was lower, and the buffer system histidine-hydrochloride (His-HCl) and acetic acid-sodium acetate (AA) were superior. CE-SDS (non-reducing) and iCIEF indicate significant decrease under accelerating condition at 40 °C, wherein CE-SDS data showed that pH 6.0 was superior, and buffer system acetic acid-sodium acetate, histidine-hydrochloric acid (His-HCl) and phosphate system were superior. The iCE data showed that the neutral peaks were decreased less at higher pH, see Figure 5 and Figure 6. The preferred buffer system is 10 mM His-HCl pH 6.0 after overall consideration.

### Example 14. Screening adjuvant for LAG-3 antibody formulation

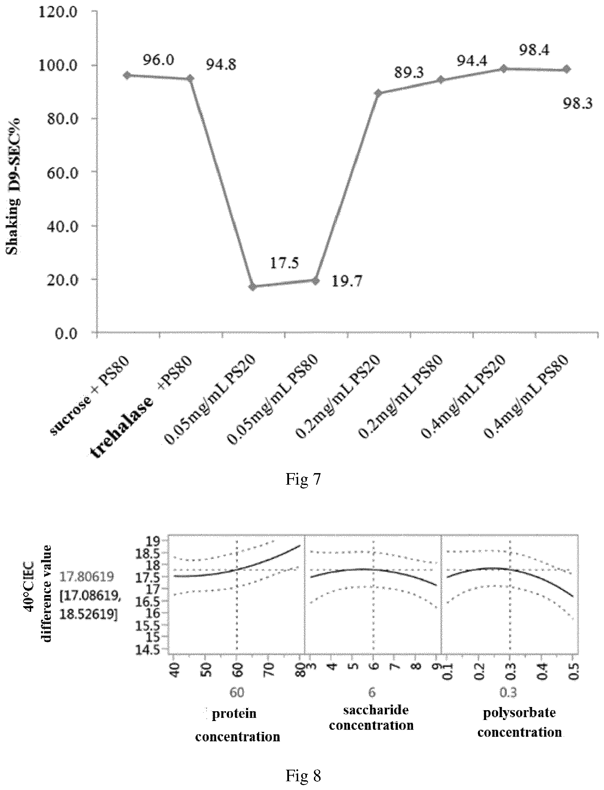

(1) LAG-3 Hu229-013 formulations were prepared in 10 mM succinic acid-sodium succinate buffer, pH 6.0, at a protein concentration of 50 mg/mL, with various concentrations of surfactant and saccharide as indicated below. The results were shown in Table 14-1.
   1) 0.1mg/mL polysorbate 20 (PS20)
   2) 0.1mg/mL polysorbate 80 (PS80)
   3) 70mg/mL sucrose
   4) 70mg/mL trehalose
   5) 50mg/mL mannitol
   6) 50mg/mL sorbitol
(2) Antibody Hu303-005 formulations were prepared in 10 mM acetic acid (sodium), pH 5.5, at antibody concentration of 50 mg/mL, with various concentrations of surfactant and saccharide as indicated below. The results were shown in Figure 7 and Table 14 -2.
   1) 75mg/mL sucrose +0.2mg/mL PS80
   2) 75mg/mL trehalose +0.2mg/mL PS80
   3) 0.05mg/mL polysorbate 20 (PS20)
   4) 0.05mg/mL polysorbate 80 (PS80)
   5) 0.2mg/mL PS20
   6) 0.2mg/mL PS80
   7) 0.4mg/mL PS20
   8) 0.4mg/mL PS80

Each formulation was filtered and added into a stoppered vial, and the vial was capped and sealed. The samples were subjected to forced degradation testing such as 40°C high temperature, repeated freezing-thawing, shaking. The results were shown in Table 14 and Figure 7.

**Table 14-1. Effect of different adjuvants on the stability of Hu229-013**

| Formulation adjuvant | condition | appearance | SEC% | IEC % | Non-redu cing CE-SDS % |
|---|---|---|---|---|---|
| | | | mono mer | neutr al | |
| 0.1 mg/ml PS 20 | D0 | clear | 99.2 | 73.5 | 97.0 |
| | freezing-thawin g once | clear | N/A | N/A | N/A |
| | 40 °C D14 | clear, a few small particles | 98.9 | 68.3 | 95.2 |
| | Shaking D9 | opalescent, containing lots of large particles | 98.9 | 74.6 | 96.2 |
| 0.1 mg/ml PS 80 | D0 | clear | 99.2 | 74.1 | 96.9 |
| | freezing -thawin g once | clear | N/A | N/A | N/A |
| | 40 °C D14 | clear, a few small particles | 99.0 | 68.7 | 95.4 |
| | Shaking D9 | clear | 99.2 | 74.3 | 96.5 |
| 70mg/ml Sucrose | D0 | clear, containing a few particles | 99.2 | 74.9 | 96.7 |
| | freezing-thawin | a few particles | N/A | N/A | N/A |
| | g once | | | | |
| | 40 °C D14 | clear, lots of small particles | 98.9 | 68.9 | 95.2 |
| | Shaking D9 | opalescent, large flocky precipitate | 98.9 | 74.0 | 96.6 |
| 70mg/ml Trehalose | D0 | clear, containing a few particles | 99.2 | 74.0 | 96.5 |
| | freezing -thawin g once | lots of particles | N/A | N/A | N/A |
| | 40 °C D14 | clear, lots of small particles | 98.9 | 69.1 | 95.3 |
| | Shaking D9 | opalescent, large flocky precipitate | 99.1 | 73.9 | 96.7 |
| 50mg/ml Mannitol | D0 | clear, containing a few particles | 99.2 | 74.3 | 96.3 |
| | freezing -thawin g once | containing particles | N/A | N/A | N/A |
| | 40 °C D14 | clear, lots of small particles | 98.9 | 68.3 | 95.1 |
| | Shaking D9 | large flocky precipitate | 99.1 | 74.5 | 96.6 |
| 50mg/ml Sorbitol | D0 | clear, containing a few particles | 99.1 | 74.1 | 95.9 |
| | freezing -thawin g once | containing particles | N/A | N/A | N/A |
| | 40°CD14 | clear, lots of small particles | 99.0 | 67.7 | 94.6 |
| | Shaking D9 | large flocky precipitate | 99.2 | 74.1 | 96.7 |

| | | | | | |
|---|---|---|---|---|---|
| Note: D indicates day. | | | | | |

**Table 14-2. Effect of different adjuvants on the stability of Hu303-005**

| Formulation adjuvant | condition | appearance | **SEC** monomer % |
|---|---|---|---|
| 75 mg/mL Sucrose +0.2 mg/mL PS80 | D0 | clear | 99.2 |
| | shaking D9 | very few particles | 96.0 |
| 75 mg/mL Trehalose +0.2 mg/mL PS80 | D0 | clear | 99.3 |
| | shaking D9 | clear | 94.8 |
| 0.05 mg/mL Polysorbate 20(PS20) | D0 | clear | 99.3 |
| | shaking D9 | clear, deep opalescent | 17.5 |
| 0.05 mg/mL Polysorbate 80(PS80) | D0 | clear | 99.2 |
| | shaking D9 | clear, shallow opalescent | 19.7 |
| 0.2mg/mL PS20 | D0 | clear | 99.1 |
| | shaking D9 | clear | 89.3 |
| 0.2 mg/mL PS80 | D0 | clear | 98.9 |
| | shaking D9 | clear | 94.4 |
| 0.4 mg/mL PS20 | D0 | clear | 99.1 |
| | shaking D9 | clear | 98.4 |
| 0.4mg/mL PS80 | D0 | clear | 99.2 |
| | shaking D9 | clear | 98.3 |

| | | | |
|---|---|---|---|
| Note: D indicates day. | | | |

The results showed that:
(1) The appearance of antibody Hu229-013 formulation in PS80 shaking group was superior to that in PS20 shaking group; after freezing and thawing once, a few particles appeared in sucrose group, and lots of particles appeared in trehalose group; there was no difference between other groups; therefore, the adjuvant was preferably polysorbate 80 and sucrose.
(2) The appearance of antibody Hu303-005 formulation was slightly better in trehalose group, but the SEC results showed that sucrose group was slightly better; as a whole, there was no obvious difference between sucrose and trehalose group. Appearance and SEC data showed that PS80 is superior to PS20, and increasing the content of polysorbate can significantly improve the appearance and SEC stability, as shown in Figure 7. Therefore, PS80 is preferred, and the PS concentration should be greater than 0.2 mg/ml.

### Example 15. Evaluation of the stability of LAG-3 antibody

(1) LAG-3 antibody Hu229-013 formulations were prepared in various buffers as indicated below, at protein concentration of 50 mg/mL, comprising 60 mg/ml sucrose and 0.4 mg/mL polysorbate 80:
   1) 10mM acetic acid-sodium acetate (AA) pH 5.5;
   2) 10mM histidine-acetic acid (His-AA) pH 6.0;
   3) 10mM histidine-hydrochloric acid (His-HCl) pH 6.0;
   4) 10mM succinic acid-sodium succinate (SA) pH 6.0.
(2) Hu303-005 formulations were prepared in various buffers as indicated below, at protein content of 50 mg/mL, comprising 75 mg/ml sucrose and 0.4 mg/mL PS 80:
   1) 10mM acetic acid-sodium acetate (AA) pH 5.5
   2) 10mM histidine-hydrochloric acid (His-HCl) pH6.0
   3) 10mM histidine-hydrochloric acid (His-HCl) pH6.5.

Each formulation was filtered and loaded into a stoppered vial, and the vial was capped and sealed. The prepared samples were placed at 25 °C or 4 °C to observe the stability. The detection items were appearance, SEC, IEC or iCE, CE-SDS (non-reducing).

**Table 15-1. Stability results of Hu229-013**

| buffer/pH | time | appearance | SEC monomer % | IEC neutral peak% | CE purity% |
|---|---|---|---|---|---|
| AA5.5 | M0 | clear | 99.2 | 75.0 | 97.0 |
| | 4°C M3.5 | clear transparent | 99.2 | 75.1 | 96.7 |
| | 25°C M3.5 | clear transparent | 98.8 | 65.4 | 95.0 |
| His-AA6.0 | M0 | clear | 99.1 | 75.4 | 97.2 |
| | 4°C M3.5 | clear transparent | 99.2 | 76.9 | 96.9 |
| | 25°C M3.5 | clear transparent | 99.0 | 68.6 | 95.3 |
| His-HCl 6.0 | M0 | clear | 99.3 | 75.4 | 97.3 |
| | 4°C M3.5 | clear transparent | 99.2 | 75.7 | 96.7 |
| | 25°C M3.5 | a few particles | 99.0 | 67.9 | 95.2 |
| SA6.0 | M0 | clear | 99.2 | 74.9 | 97.2 |
| | 4°C M3.5 | clear transparent | 99.2 | 76.7 | 96.9 |
| | 25°C M3.5 | a few particles | 98.8 | 68.1 | 96.5 |

| | | | | | |
|---|---|---|---|---|---|
| Note: M3.5 means 3.5 months | | | | | |

**Table 15-2 Stability results of Hu303-005 at 4°C**

| buffer/pH | **time(M)** | appearance | **SEC monomer %** | **Non-reducing CE-SDS%** | **iCIEF%** | | |
|---|---|---|---|---|---|---|---|
| | | | | | **acid** | **neutral** | **alkaline** |
| AA5.5 | 0 | clear | 99.1 | 97.1 | 22.9 | 55.9 | 21.2 |
| | 1 | clear | 99.1 | 97.0 | 23.6 | 59.6 | 16.8 |
| | 3 | N/A | 99.2 | 97.0 | 20.2 | 57.5 | 22.2 |
| His-HCl 6.0 | 0 | clear | 99.1 | 97.6 | 23.3 | 58.2 | 18.5 |
| | 1 | clear | 99.1 | 97.1 | 23.4 | 60.7 | 15.9 |
| | 3 | N/A | 99.1 | 97.2 | 22.1 | 59.4 | 18.6 |
| His-HC16.5 | 0 | clear | 99.2 | 97.5 | 22.9 | 57.8 | 19.3 |
| | 1 | clear | 98.9 | 96.9 | 23.2 | 63.3 | 13.5 |
| | 3 | N/A | 99.1 | 96.3 | 23.8 | 61.4 | 14.9 |

| | | | | | | | |
|---|---|---|---|---|---|---|---|
| Note: M indicates month and N/A indicates no detection. | | | | | | | |

The results showed:
(1) LAG-3 antibody Hu229-013 was more stable in 10 mM AA pH 5.5, 10 mM His-AA pH 6.0 system.
(2) After being placed at 4 °C for 3 months, the CE of antibody Hu303-005 was decreased slightly decreased in His-HCl (pH 6.5) group, the iCE of the antibody was slightly altered in AA pH 5.5 and His-HCl pH 6.5 group, and the antibody was most stable in His-HCl (pH 6.0).

### Example 16. Optimization of LAG-3 antibody formulation

In order to further optimize the type, pH and ionic strength of the buffer, the concentration of antibody Hu229-013 was set to 50 mg/ml, and the DOE experiment was designed with JMP software. A series of formulations were obtained by using RSM model. IEC, CE (non-reducing) and microfluidic imaging (MFI) were used as evaluation indexes in the forced degradation methods. The results were statistically analyzed by least squares method. DOE parameters were shown in Table 16. The testing formulations and results were shown in Table 17 and Table 18.

**Table 16. DOE design factor and level**

| factor | level | **Observations** |
|---|---|---|
| Buffer system | AA/His-AA | High temperature 25/40°C, shaking, freezing-thawing# |
| pH | 5.0-5.8 | |
| Ionic strength | 10-30 mM | |

**Table 17. DOE design formulations for Hu229-013**

| Batch number | Buffer system | Ionic strength | pH | Other adjuvants |
|---|---|---|---|---|
| 1 | Histidine-acetic acid(His-AA) | 20 | 5.5 | 75mg/ml sucrose, 0.2mg/ml PS80 |
| 2 | His-AA | 10 | 5.8 | |
| 3 | Acetic acid-sodium acetate(AA) | 20 | 5.8 | |
| 4 | His-AA | 10 | 5.2 | |
| 5 | His-AA | 30 | 5.8 | |
| 6 | AA | 10 | 5.5 | |
| 7 | AA | 20 | 5.2 | |
| 8 | AA | 30 | 5.5 | |
| 9 | His-AA | 30 | 5.2 | |
| 10 | His-AA | 20 | 5.5 | |

**Table 18. Screening results of DOE formulation**

| Batch number | MFI(>2um particle/ml) | | | IEC-neutral peak% | | | Non-reducing CE-SDS% | | |
|---|---|---|---|---|---|---|---|---|---|
| | D0 | Freezing-thawing 5 times | shaking | D0 | 25°C M1 | 40°C D21 | D0 | 25°C M1 | 40°C D21 |
| 1 | 7594 | 7589 | 264 | 72.7 | 70.9 | 57.2 | 96.8 | 96.8 | 93.0 |
| 2 | 9184 | 7213 | 2422 | 73.1 | 70.9 | 59.7 | 96.9 | 96.9 | 93.7 |
| 3 | 1013 6 | 11806 | 274 | 76.5 | 68.4 | 59.1 | 96.7 | 96.8 | 94.1 |
| 4 | 16711 | 8399 | 474 | 76.0 | 70.2 | 56.7 | 96.6 | 96.6 | 92.8 |
| 5 | 3278 0 | 2975 | 604 | 75.6 | 67.6 | 56.9 | 96.6 | 96.7 | 93.5 |
| 6 | 1150 3 | 4013 | 1751 | 75.5 | 67.7 | 57.4 | 96.6 | 96.5 | 93.8 |
| 7 | 5973 | 11522 | 790 | 75.9 | 67.4 | 55.6 | 96.6 | 96.5 | 92.5 |
| 8 | 2920 6 | 10532 | 159 | 74.9 | 67.6 | 57.4 | 96.5 | 96.5 | 93.2 |
| 9 | 5625 | 4878 | 660 | 75.9 | 67.9 | 53.8 | 96.8 | 96.6 | 91.6 |
| 10 | 12970 | 3588 | 1464 | 76.1 | 69.2 | 56.9 | 96.7 | 96.7 | 93.1 |

| | | | | | | | | | |
|---|---|---|---|---|---|---|---|---|---|
| Note: M1 means one month and D means day. | | | | | | | | | |

The data obtained in forced degradation were subjected to fitting and the results showed that: LAG-3 antibody Hu229-013 showed good stability in 10-30 mM acetic acid-sodium acetate (AA) buffer or histidine-acetic acid buffer (His-AA) system, pH 5.2-5.8. Preferably, the buffer system is 10-30 mM acetic acid-sodium acetate (AA), pH 5.5.

### Example 17. Testing the stability of LAG-3 antibody formulation

LAG-3 Hu229-013 formulation was prepared in 10 mM acetic acid-sodium acetate pH 5.5 buffer, at a protein concentration of 60 mg/mL, comprising 60 mg/ml sucrose and 0.4 mg/mL polysorbate 80.

The formulation was filtered and added into a stoppered vial, and the vial was capped and sealed. The prepared samples were placed at 4 °C to observe the stability. The detection items involve appearance, SEC, IEC, CE-SDS (non-reducing).

**Table 19. Results of stability of Hu229-013**

| Temp. | Time (M) | appea rance | SEC% | | | Non-reducing CE-SDS% | IEC% | | |
|---|---|---|---|---|---|---|---|---|---|
| | | | aggreg ate | monom er | fragme nt | | acid | neutral | alkali ne |
| 4°C | 0 | clear | 1.1 | 98.9 | 0.0 | 97.6 | 14.4 | 72.0 | 13.6 |
| | 1 | clear | 1.0 | 98.9 | 0.1 | 96.2 | 14.5 | 71.8 | 13.6 |
| | 3 | clear | 1.0 | 98.8 | 0.1 | 96.8 | 16.2 | 68.4 | 15.5 |
| | 6 | clear | 1.2 | 98.7 | 0.1 | 96.9 | 14.0 | 73.0 | 13.0 |
| | 9 | clear | 1.2 | 98.8 | 0.1 | 97.3 | 13.8 | 71.4 | 14.9 |

The results showed that the Hu229-013 formulation retained its stability for 9 months at 4 °C.

### Example 18. Optimization of LAG-3 antibody formulation

### (1) Optimization of components comprised in Hu229-013 formulation

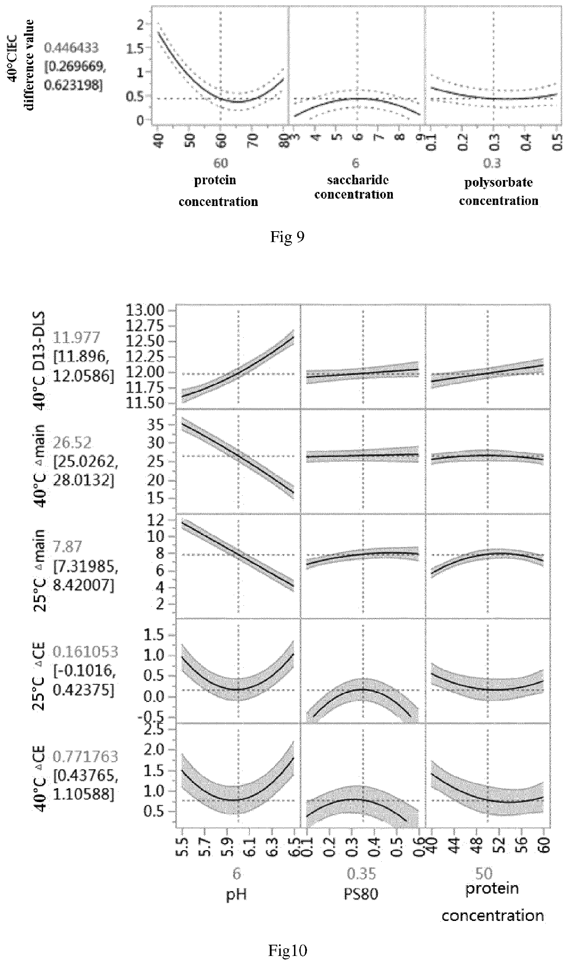

In order to further optimize the concentration of protein, sucrose and polysorbate 80, the buffer was set to 10 mM acetic acid-sodium acetate, pH 5.5. DOE experiment design was carried out by using JMP software. A series of formulations were obtained by using the RSM model, and IEC and CE (non-reducing) were used as evaluation indexes in the forced degradation methods. The results were statistically analyzed by least squares method. DOE parameters were shown in Table 20. The test results were shown in Table 21. The statistical analysis results were shown in Figure 8, Figure 9, and Table 21.

**Table 20. DOE design factor and level**

| factor | level | **Observation** |
|---|---|---|
| Protein conc. | 40-80mg/ml | High temperature 40°C |
| Sucrose conc. | 30-90mg/ml | |
| PS80 conc. | 0.1-0.5mg/ml | |

**Table 21. DOE design formulations and screening results**

| number | Protein conc. mg/ml | Sucrose conc. mg/ml | PS80 conc. mg/ml | IEC neutral peak % | | Non-reducing CE-SDS purity % | |
|---|---|---|---|---|---|---|---|
| | | | | D0 | 40°C D16 | D0 | 40°C D16 |
| 1 | 60 | 60 | 0.1 | 78.0 | 60.6 | 95.1 | 94.4 |
| 2 | 40 | 30 | 0.1 | 77.2 | 60.7 | 96.4 | 94.3 |
| 3 | 40 | 60 | 0.3 | 77.8 | 60.5 | 96.1 | 94.2 |
| 4 | 60 | 90 | 0.5 | 74.6 | 59.8 | 94.6 | 94.5 |
| 5 | 60 | 30 | 0.3 | 77.4 | 60.0 | 94.6 | 94.5 |
| 6 | 40 | 90 | 0.1 | 77.5 | 59.8 | 96.0 | 94.3 |
| 7 | 40 | 60 | 0.3 | 77.6 | 59.9 | 96.1 | 94.2 |
| 8 | 80 | 30 | 0.1 | 75.8 | 59.6 | 94.8 | 94.4 |
| 9 | 80 | 90 | 0.3 | 77.6 | 58.9 | 95.1 | 94.4 |
| 10 | 60 | 60 | 0.3 | 77.7 | 59.6 | 94.6 | 94.2 |
| 11 | 80 | 60 | 0.5 | 77.6 | 59.6 | 95.2 | 94.1 |
| 12 | 40 | 30 | 0.5 | 77.5 | 60.0 | 95.6 | 94.0 |

The data obtained from various forced degradations were subjected to fitting and the results were as follows:
The difference values between the IEC values at D0 and the ICE values at 40°C were subjected to fitting. R²>0.98, P<0.06, the model was valid, and the result was shown in Figure 8. The difference values between the CE purity values at D0 and the CE purity values at 40 °C were subjected to fitting. R²>0.99, P<0.05, the model was valid, and the result was shown in Figure 9. The fitting results of 40°C IEC showed that a more preferable formulation is: 40-60mg/ml protein concentration, 30-90mg/ml saccharide concentration, and 0.4-0.5mg/ml PS80 concentration; The fitting results of 40°C CE showed that a more preferable formulation is: 50-80mg/ml protein concentration, 30-90mg/ml saccharide concentration, and 0.1-0.5mg/ml PS80 concentration. Therefore, the most preferable range is: 50-60 mg/ml protein concentration, 30-90 mg/ml sucrose concentration, and 0.4-0.5 mg/ml PS80 concentration.

### (2) Optimization of components comprised in Hu303-005 antibody formulation

The sucrose concentration was set to 75 mg/ml, and DOE experiment was designed with pH values of 10 mM His buffer, protein concentrations and polysorbate concentrations being used as variables. The RSM model was used to obtain a series of formulations. The formulations were shown in Table 22. iCIEF, CE (non-reducing), and DLS were used as evaluation indexes in the forced degradation methods. The results were statistically analyzed by least squares method. The results were shown in Table 23 and Figure 10.

**Table 22. Screening DOE formulation for Antibody Hu303-005 formulation**

| No. | pH | PS80 mg/mL | Protein conc. mg/mL |
|---|---|---|---|
| 1 | 5.5 | 0.1 | 50 |
| 2 | 6.5 | 0.35 | 40 |
| 3 | 5.5 | 0.6 | 60 |
| 4 | 5.5 | 0.35 | 40 |
| 5 | 6 | 0.35 | 60 |
| 6 | 6.5 | 0.6 | 60 |
| 7 | 6 | 0.35 | 50 |
| 8 | 6.5 | 0.1 | 50 |
| 9 | 6 | 0.1 | 40 |
| 10 | 6 | 0.1 | 60 |
| 11 | 6 | 0.6 | 40 |
| 12 | 6 | 0.35 | 50 |

**Table 23. Screening results of DOE formulation of Hu303-005 antibody formulation**

| **No.** | **condition** | **DLS** | **iCIEF neutral peak (%)** | **Non-reducing CE-SDS%** |
|---|---|---|---|---|
| | | **average particle size nm** | | |
| 1 | D0 | N/A | 58.3 | 97.60 |
| | 25°C-D13 | N/A | 46.8 | 97.46 |
| | 40°C-D13 | 11.5 | 22.4 | 96.55 |
| 2 | D0 | N/A | 57.3 | 98.47 |
| | 25°C-D13 | N/A | 54.8 | 96.92 |
| | 40°C-D13 | 12.2 | 40.8 | 96.12 |
| 3 | D0 | N/A | 57.5 | 97.54 |
| | 25°C-D13 | N/A | 47.1 | 97.35 |
| | 40°C-D13 | 11.6 | 23.5 | 96.79 |
| 4 | D0 | N/A | 56.1 | 98.46 |
| | 25°C-D13 | N/A | 47.7 | 97.19 |
| | 40°C-D13 | 11.7 | 22.9 | 96.23 |
| 5 | D0 | N/A | 59.3 | 97.82 |
| | 25°C-D13 | N/A | 51.9 | 97.58 |
| | 40°C-D13 | 12.0 | 32.7 | 96.86 |
| 6 | D0 | N/A | 58.5 | 97.61 |
| | 25°C-D13 | N/A | 55.3 | 97.37 |
| | 40°C-D13 | 13.0 | 42.6 | 96.57 |
| 7 | D0 | N/A | 59.1 | 97.65 |
| | 25°C-D13 | N/A | 51.4 | 97.51 |
| | 40°C-D13 | 11.9 | 32.3 | 96.69 |
| 8 | D0 | N/A | 57.0 | 97.43 |
| | 25°C-D13 | N/A | 55.0 | 97.38 |
| | 40°C-D13 | 12.5 | 42.1 | 95.89 |
| 9 | D0 | N/A | 57.0 | 97.31 |
| | 25°C-D13 | N/A | 52.8 | 97.75 |
| | 40°C-D13 | 11.8 | 31.4 | 96.38 |
| 10 | D0 | N/A | 56.4 | 97.30 |
| | 25°C-D13 | N/A | 50.5 | 97.56 |
| | 40°C-D13 | 12.1 | 32.3 | 96.88 |
| 11 | D0 | N/A | 57.1 | 97.07 |
| | 25°C-D13 | N/A | 51.2 | 97.30 |
| | 40°C-D13 | 11.9 | 31.9 | 96.34 |
| 12 | D0 | N/A | 58.7 | 97.36 |
| | 25°C-D13 | N/A | 50.7 | 97.09 |
| | 40°C-D13 | 12.1 | 32.3 | 96.88 |

The data obtained from forced degradations were subjected to fitting, and the iCIEF/CE/DLS data at 25°C and 40°C were well subjected to fitting, and the model was valid. The results were shown in figure 10.

The results showed that under high temperature condition, the increase in pH will increase the particle size and the neutral peak. The changing rate of iCIEF will be slowed down with the decrease of temperature. The CE data were the most excellent at pH 6.0. In combination with the previous experimental results (more stable in 10 mM His pH 6.0), the most preferable pH is determined as 6.0; The CE data obtained at 40°C indicated that protein concentration of 45-60mg/ml is more preferable, hence, most preferably, the concentration is 50mg/ml; In combination with the results showing the effect of polysorbate concentrations on protein stability under various conditions, and screening results of polysorbate concentration in other experiments (concentration of greater than 0.2 mg/ml was more preferable), PS80 concentration is set at 0.3 mg/ml; For Hu303-005 antibody liquid formulation in this and other examples, iCIEF showed a significant decrease in high temperature conditions, and it is contemplated to be formulated as a lyophilized formulation. In order to ensure good moldability and suitable osmotic pressure of the lyophilized formulation, the sucrose concentration is set to be 75 mg/ml.

### Example 19. Lyophilization of LAG-3 antibody formulation

A lyophilized formulation of Hu303-005 was prepared in 10 mM histidine-hydrochloric acid, pH 5.5 or 6.0, at protein content of 50 mg/ml, comprising 75 mg/ml sucrose, and 0.4 mg/ml PS80. The lyophilizing procedures were as follows:

**Table 24. Lyophilizing procedures**

| procedures | Set Temp. °C | Set Time (min) | Section time | vacuum degree (mBar) |
|---|---|---|---|---|
| pre-freezing | 5°C | 10 | 60 min | N/A |
| | -45°C | 50 | 120 min | N/A |
| Primary drying | -20°C | 120 | 36 h | 0.1 |
| secondary drying | 25°C | 60 | 5 h | 0.01 |

The samples were placed at 4 °C and 25 °C to observe the stability. Samples were taken at various time points and reconstituted with an appropriate amount of water for injection. The results were shown in Table 25 and Table 26. The results showed that there were no significant changes in various indexes of Hu303-005 formulation during acceleration test at 25 °C during long-term storage at 4 °C for M3, and the stability was good.

**Table 25. Results of stability of Hu303-005 lyophilized formulation at 4 °C**

| **Batch number** | **Time (M)** | **Appearance of reconstitution solution** | **SEC monomer%** | **Non-reducing CE-SDS%** | **iCIEF neutral peak%** |
|---|---|---|---|---|---|
| pH 5.5 | 0 | clear | 99.9 | 98.2 | 68.5 |
| | 3 | clear | 99.8 | 97.5 | 69.0 |
| pH 6.0 | 0 | clear | 99.9 | 98.1 | 69.8 |
| | 3 | clear | 99.9 | 97.5 | 70.1 |

**Table 26. Results of stability of Hu303-005 lyophilized formulation at 25°C**

| **Batch number** | **Time (M)** | **Appearance of reconstitution solution** | **SEC monomer %** | **Non-reducing CE-SDS%** | **iCIEF neutral peak%** |
|---|---|---|---|---|---|
| pH 5.5 | 0 | clear | 99.9 | 98.2 | 68.5 |
| | 1 | clear | 99.8 | 97.8 | 68.1 |
| | 3 | clear | 99.8 | 97.7 | 68.1 |
| pH 6.0 | 0 | clear | 99.9 | 98.1 | 69.8 |
| | 1 | clear | 99.8 | 97.7 | 69.4 |
| | 3 | clear | 99.7 | 97.2 | 69.4 |

At the same time, the stability of the reconstitution solution of the lyophilized antibody Hu229-013 was determined. LAG-3 antibody formulation was prepared in 10 mM acetic acid-sodium acetate pH 5.5, at a protein concentration of 50 mg/ml, comprising 75 mg/ml sucrose and 0.4 mg/ml polysorbate 80. The antibody was filled into a 2 mL vial, 1.1mL/vial, placed in a lyophilization box, and lyophilized. Comparison was made on the samples before and after lyophilization to observe the stability. The results showed that there was no change in the quality of the antibody Hu229-013 before and after lyophilization, and the lyophilized formulation had good stability during storage.

### Example 20. Optimization of parameters in freeze-drying process

A formulation comprising 50 mg/ml antibody Hu303-005, 10 mM histidine-hydrochloric acid, pH 6.0, 75mg/ml sucrose and 0.4 mg/ml PS80 was prepared and lyophilized. The temperature at which Hu303-005 collapse occurred was about -19 °C, as measured by freeze-drying microscope. The temperature for primary drying is an important parameter of the freeze-drying process. Therefore, the shelf temperature during the primary drying process was carefully optimized. The freeze-drying parameters were shown in Table 27. The results were shown in Table 28. The appearance of the lyophilized powder at each temperature met the requirements. However, a few particles appeared in the appearance after reconstitution at -5°C. Hence, the shelf temperature for the primary drying was set to -10°C.

**Table 27. Freeze-drying parameters for screening the shelf temperature during the primary drying**

| Screening item | | Temperature for the primary drying | | | |
|---|---|---|---|---|---|
| parameter | | Set temperature | Set time | Section time | Vacuum degree |
| Pre-freezing | | +5 °C | 10 min | 60 min | N/A |
| | | -45 °C | 50 min | 120 min | N/A |
| Primary drying | 1 | -20 °C | 120 min | 1000 - 3000 min | 0.10mbar |
| | 2 | -10 °C | | | |
| | 3 | -5 °C | | | |
| Secondary drying | | +25 °C | 60 min | 300 - 540 min | 0.01 mbar |

**Table 28. Comparison of appearance before and after reconstitution of lyophilized samples obtained by different processes**

| group | Temperature for primary drying | Appearance of the lyophilized powder | Appearance after reconstitution |
|---|---|---|---|
| 1 | -20 °C | White powder, full appearance, no collapse | clear |
| 2 | -10 °C | White powder, full appearance, no collapse | clear |
| 3 | -5 °C | White powder, full appearance, no collapse | A few particles |

The final freeze-drying process was as follows:

**Table 29. Freeze-drying process**

| | Set temperature | Set time (min) | Section time(min) | Vacuum degree (mBar) |
|---|---|---|---|---|
| Pre-freezing | 5°C | 10 | 60 | N/A |
| | -45°C | 50 | 120 | N/A |
| Primary drying | -10°C | 120 | 2000* | 0.10 |
| Secondary drying | 25°C | 60 | 300* | 0.01 |

| | | | | |
|---|---|---|---|---|
| * Primary drying and secondary drying time depend on the specific batch and pressure-rise test. | | | | |

### Example 21. Other Alternative Formulations

The present invention also provides the following stable pharmaceutical formulations comprising any one selected from the group consisting of:
(1) 90mg/ml LAG-3 antibody Hu229-013, 80mg/ml sucrose, 0.4mg/ml polysorbate 80, 15 mM acetic acid-sodium acetate buffer, pH 5.5;
(2) 90mg/ml LAG-3 antibody Hu229-013, 80mg/ml sucrose, 0.4mg/ml polysorbate 80, 15mM acetic acid-sodium acetate buffer, pH 6.5;
(3) 90mg/ml LAG-3 antibody Hu229-013, 50mg/ml sucrose, 0.4mg/ml polysorbate 80, 25mM acetic acid-sodium acetate buffer, pH 5.5;
(4) 70mg/ml LAG-3 antibody Hu229-013, 50mg/ml sucrose, 0.3mg/ml polysorbate 80, 10 mM acetic acid-sodium acetate buffer, pH 5.5;
(5) 70mg/ml LAG-3 antibody Hu229-013, 80mg/ml sucrose, 0.3mg/ml polysorbate 80, 15mM acetic acid-sodium acetate buffer, pH 5.2;
(6) 40mg/ml LAG-3 antibody Hu229-013, 75mg/ml sucrose, 0.4mg/ml polysorbate 80, 10 mM acetic acid-sodium acetate buffer, pH 6.0;
(7) 55mg/ml LAG-3 antibody Hu229-013, 75mg/ml sucrose, 0.4mg/ml polysorbate 80, lOmM acetic acid-sodium acetate buffer, pH 5.7;
(8) 30mg/ml LAG-3 antibody Hu229-013, 70mg/ml sucrose, 0.5mg/ml polysorbate 80, lOmM acetic acid-sodium acetate buffer, pH 5.5;
(9) 20mg/ml LAG-3 antibody Hu229-013, 70mg/ml sucrose, 0.2mg/ml polysorbate 80, lOmM acetic acid-sodium acetate buffer, pH 5.4;
(5) 15mg/ml LAG-3 antibody Hu229-013, 85mg/ml sucrose, 0.1mg/ml polysorbate 80, 25mM acetic acid-sodium acetate buffer, pH 5.6;
(11) 50mg/ml LAG-3 antibody Hu229-013, 85mg/ml sucrose, 0.3mg/ml polysorbate 80, 25mM acetic acid-sodium acetate buffer, pH 5.8;
(12) 50mg/ml LAG-3 antibody Hu229-013, 75mg/ml sucrose, 0.4mg/ml polysorbate 80, 25 mM acetic acid-sodium acetate buffer, pH 6.0;
(13) 55mg/ml LAG-3 antibody Hu229-013, 90mg/ml sucrose, 0.4mg/ml polysorbate 80, lOmM acetic acid-sodium acetate buffer, pH 5.3;
(14) 50mg/ml LAG-3 antibody Hu229-013, 90mg/ml sucrose, 0.4mg/ml polysorbate 80, lOmM acetic acid-sodium acetate buffer, pH 5.0;
(15) 90mg/ml LAG-3 antibody Hu303-005, 75mg/ml sucrose, 0.3mg/ml polysorbate 80, 30mM histidine-hydrochloric acid buffer, pH6.0;
(16) 90mg/ml LAG-3 antibody Hu303-005, 75mg/ml sucrose, 0.3mg/ml polysorbate 80, 30mM histidine-hydrochloric acid buffer, pH 5.5;
(17) 75mg/ml LAG-3 antibody Hu303-005, 75mg/ml sucrose, 0.3mg/ml polysorbate 80, 30mM histidine-hydrochloric acid buffer, pH5.0;
(18) 1mg/ml LAG-3 antibody Hu303-005, 75mg/ml sucrose, 0.3mg/ml polysorbate 80, 5mM histidine-hydrochloric acid buffer, pH6.0;
(19) 70mg/ml LAG-3 antibody Hu303-005, 30mg/ml sucrose, 0.3mg/ml polysorbate 80, 5mM histidine-hydrochloric acid buffer, pH6.0;
(20) 50mg/ml LAG-3 antibody Hu303-005, 60mg/ml trehalose, 0.3mg/ml polysorbate 80, lOmM histidine-hydrochloric acid buffer, pH6.0;
(21) 50 mg/ml LAG-3 antibody Hu303-005, 90mg/ml trehalose, 0.3mg/ml polysorbate 80, 10 mM histidine-hydrochloric acid buffer, pH 6.0.

## Claims

1. A pharmaceutical composition comprising a LAG-3 antibody or an antigen-binding fragment thereof, and a buffer, wherein the buffer is selected from the group consisting of acetate buffer, histidine buffer, citrate buffer, succinate buffer and Tris buffer.

2. The pharmaceutical composition according to claim 1, wherein the acetate buffer is selected from acetic acid-sodium acetate buffer; wherein the histidine buffer is selected from the group consisting of histidine-hydrochloric acid buffer and histidine-acetic acid buffer; wherein the citrate buffer is citric acid-sodium citrate buffer; wherein the succinate buffer is succinic acid-sodium succinate buffer; preferably, the buffer is histidine- hydrochloric acid buffer, or acetic acid-sodium acetate buffer.

3. The pharmaceutical composition according to claim 1 or 2, wherein the concentration of the LAG-3 antibody or antigen-binding fragment thereof is from 1mg/ml to 90 mg/ml, preferably from 40 mg/ml to 60 mg/ml, more preferably 50 mg/ml.

4. The pharmaceutical composition according to any one of claims 1 to 3, wherein the buffer has a pH of from about 5.0 to 6.5, preferably from about 5.0 to 6.0.

5. The pharmaceutical composition according to any one of claims 1 to 4, wherein the concentration of the buffer is from 5 mM to 30 mM, preferably from 10 mM to 30 mM.

6. The pharmaceutical composition according to any one of claims 1 to 5, further comprising an adjuvant, wherein the adjuvant is one or more selected from the group consisting of saccharide and surfactant.

7. The pharmaceutical composition according to claim 6, wherein the saccharide is disaccharide, preferably is trehalose, sucrose, mannitol or sorbitol, more preferably is sucrose.

8. The pharmaceutical composition according to claim 7, wherein the concentration of the sucrose is from 30 mg/ml to 90 mg/ml, preferably from 60 mg/ml to 90 mg/ml, more preferably 75 mg/ml.

9. The pharmaceutical composition according to claim 6, wherein the surfactant is polysorbate, wherein the polysorbate is selected from the group consisting of polysorbate 80 and polysorbate 20, preferably is polysorbate 80.

10. The pharmaceutical composition according to claim 9, wherein the concentration of the surfactant is from 0.02 mg/ml to 0.8 mg/ml, preferably from 0.2 mg/ml to 0.6 mg/ml, more preferably from 0.3 mg/ml to 0.5 mg/ml.

11. The pharmaceutical composition according to any one of claims 1 to 10, which comprises the components shown in the following i) or ii):
i) (a) 1 mg/ml to 90 mg/ml LAG-3 antibody or antigen-binding fragment thereof, (b) 5 mM to 30 mM acetic acid-sodium acetate buffer, pH is about 5.0-6.5, (c) 30 mg/ml to 90 mg/ml sucrose and (d) 0.02 mg/ml to 0.8 mg/ml polysorbate 80; or
ii) (a) 1 mg/ml to 90 mg/ml LAG-3 antibody or antigen-binding fragment thereof, (b) 5 mM to 30 mM histidine-hydrochloric acid buffer, pH is about 5.0-6.5, (c) 30 mg/ml to 90 mg/ml sucrose or trehalose, and (d) 0.05 mg/ml to 0.6 mg/ml polysorbate 80, preferably, the pharmaceutical composition comprises:
(e) 40 mg/ml to 80 mg/ml LAG-3 antibody or antigen-binding fragment thereof, (f) 10 mM to 30 mM acetic acid-sodium acetate buffer, pH is about 5.2-5.8, (g) 70 mg/ml to 80 mg /ml sucrose, and (h) 0.4 mg/ml to 0.5 mg/ml polysorbate 80; or the pharmaceutical composition comprises:
(e) 45 mg/ml to 60 mg/ml LAG-3 antibody or antigen-binding fragment thereof, (f) 10 mM to 30 mM histidine-hydrochloric acid buffer, pH is about 5.5-6.0, (g) 60 mg/ml to 90 mg/ml sucrose or trehalose, and (h) 0.2 mg/ml to 0.6 mg/ml polysorbate 80.

12. The pharmaceutical composition according to any one of claims 1 to 11, wherein the humanized LAG-3 antibody comprises a heavy chain variable region and a light chain variable region, wherein the amino acid sequence of the heavy chain variable region is set forth in any one of SEQ ID NO: 21, SEQ ID NO: 23, SEQ ID NO: 24 or SEQ ID NO: 25, or the sequence has at least 85% sequence identity to SEQ ID NO: 21, SEQ ID NO: 23, SEQ ID NO: 24 or SEQ ID NO: 25; and
wherein the amino acid sequence of the light chain variable region is set forth in any one of SEQ ID NO: 22, SEQ ID NO: 26, SEQ ID NO: 27 or SEQ ID NO: 28, or the sequence has at least 85% sequence identity to SEQ ID NO: 22, SEQ ID NO: 26, SEQ ID NO: 27 or SEQ ID NO: 28.

13. The pharmaceutical composition of claim 12, comprising:
(a) 1 to 90 mg/ml LAG-3 antibody or antigen-binding fragment thereof, (b) 5 mM to 30 mM acetic acid-acetate buffer, pH is about 5.0-6.5, (c) 30 mg/ml to 90 mg/ml sucrose, and (d) 0.02 mg/ml to 0.8 mg/ml polysorbate 80;
preferably, the pharmaceutical composition comprises:
(e) 40 mg/ml to 80 mg/ml LAG-3 antibody or antigen-binding fragment thereof, (f) 10 mM to 30 mM acetic acid-sodium acetate buffer, pH is about 5.2-5.8, (g) 70 mg/ml to 80 mg/ml of sucrose, and (h) 0.4 mg/ml to 0.5 mg/ml polysorbate 80;
more preferably, the pharmaceutical composition comprises:
(i) about 50 mg/ml LAG-3 antibody or antigen-binding fragment thereof, (j) 10 mM acetic acid-sodium acetate buffer, pH is about 5.5, (k) about 75 mg/ml sucrose, and (l) about 0.4 mg/ml polysorbate 80.

14. The pharmaceutical composition according to claim 13, wherein the humanized LAG3 antibody comprises a combination of a heavy chain variable region and a light chain variable region as shown in followings:
1) a heavy chain variable region of SEQ ID NO: 21 and a light chain variable region of SEQ ID NO: 22;
2) a heavy chain variable region of SEQ ID NO: 21 and a light chain variable region of SEQ ID NO: 26;
3) a heavy chain variable region of SEQ ID NO: 21 and a light chain variable region of SEQ ID NO: 27;
4) a heavy chain variable region of SEQ ID NO: 21 and a light chain variable region of SEQ ID NO: 28;
5) a heavy chain variable region of SEQ ID NO: 23 and a light chain variable region of SEQ ID NO: 22;
6) a heavy chain variable region of SEQ ID NO: 23 and a light chain variable region of SEQ ID NO: 26;
7) a heavy chain variable region of SEQ ID NO: 23 and a light chain variable region of SEQ ID NO: 27;
8) a heavy chain variable region of SEQ ID NO: 23 and a light chain variable region of SEQ ID NO: 28;
9) a heavy chain variable region of SEQ ID NO: 24 and a light chain variable region of SEQ ID NO: 22;
10) a heavy chain variable region of SEQ ID NO: 24 and a light chain variable region of SEQ ID NO: 26;
11) a heavy chain variable region of SEQ ID NO: 24 and a light chain variable region of SEQ ID NO: 27;
12) a heavy chain variable region of SEQ ID NO: 24 and a light chain variable region of SEQ ID NO: 28;
13) a heavy chain variable region of SEQ ID NO: 25 and a light chain variable region of SEQ ID NO: 22;
14) a heavy chain variable region of SEQ ID NO: 25 and a light chain variable region of SEQ ID NO: 26;
15) a heavy chain variable region of SEQ ID NO: 25 and a light chain variable region of SEQ ID NO: 27; and
16) a heavy chain variable region of SEQ ID NO: 25 and a light chain variable region of SEQ ID NO: 28.

15. The pharmaceutical composition according to any one of claims 12 to 14, wherein the humanized LAG-3 antibody comprises a heavy chain constant region and a light chain constant region, wherein the heavy chain constant region is preferably set forth in SEQ ID NO: 38; wherein the light chain constant region is preferably set forth in SEQ ID NO:39.

16. The pharmaceutical composition according to claim 15, wherein the heavy chain amino acid sequence of the humanized LAG-3 antibody is set forth in SEQ ID NO: 40 or the sequence has at least 95% sequence identity to SEQ ID NO: 40, and the light chain amino acid sequence is set forth in SEQ ID NO: 41 or the sequence has at least 95% sequence identity to SEQ ID NO: 41.

17. The pharmaceutical composition according to any one of claims 1 to 11, wherein the humanized LAG-3 antibody comprises a heavy chain variable region and a light chain variable region, wherein the amino acid sequence of the heavy chain variable region is set forth in any one of SEQ ID NO: 29, SEQ ID NO: 31, SEQ ID NO: 32 or SEQ ID NO: 33, or the sequence has at least 85% sequence identity to SEQ ID NO: 29, SEQ ID NO: 31, SEQ ID NO: 32 or SEQ ID NO: 33, and
wherein the amino acid sequence of the light chain variable region is set forth in any one of SEQ ID NO: 30, SEQ ID NO: 34, SEQ ID NO: 35, SEQ ID NO: 36 or SEQ ID NO: 37, or the sequence has at least 85% sequence identity to SEQ ID NO: 30, SEQ ID NO: 34, SEQ ID NO: 35, SEQ ID NO: 36 or SEQ ID NO: 37.

18. The pharmaceutical composition of claim 17, comprising:
(a) 1 mg/ml to 90 mg/ml LAG-3 antibody or antigen-binding fragment thereof, (b) 5 mM to 30 mM histidine-hydrochloric acid buffer, pH is about 5.0-6.5, (c) 30 mg/ml to 90 mg/ml sucrose or trehalose, and (d) 0.05 mg/ml to 0.6 mg/ml polysorbate 80; preferably, the pharmaceutical composition comprises:
(e) 45 mg/ml to 60 mg/ml LAG-3 antibody or antigen-binding fragment thereof, (f) about 10 mM to 30 mM histidine-hydrochloric acid buffer, pH is about 5.5-6.0, (g) 60 mg/ml to 90 mg/ml sucrose or trehalose, and (h) 0.2 mg/ml to 0.6 mg/ml polysorbate 80;
more preferably, the pharmaceutical composition comprises:
(i) about 50 mg/ml LAG-3 antibody or antigen-binding fragment thereof, (j) 10 mM histidine-hydrochloric acid buffer, pH is about 6.0, (k) about 75 mg/ml sucrose, (l) about 0.3 mg/ml polysorbate 80.

19. The pharmaceutical composition according to claim 18, wherein the humanized LAG3 antibody comprises a combination of a heavy chain variable region and a light chain variable region as shown in followings:
1) a heavy chain variable region of SEQ ID NO: 29 and a light chain variable region of SEQ ID NO: 30;
2) a heavy chain variable region of SEQ ID NO: 29 and a light chain variable region of SEQ ID NO: 34;
3) a heavy chain variable region of SEQ ID NO: 29 and a light chain variable region of SEQ ID NO: 35;
4) a heavy chain variable region of SEQ ID NO: 29 and a light chain variable region of SEQ ID NO: 36;
5) a heavy chain variable region of SEQ ID NO: 29 and a light chain variable region of SEQ ID NO: 37;
6) a heavy chain variable region of SEQ ID NO: 31 and a light chain variable region of SEQ ID NO: 30;
7) a heavy chain variable region of SEQ ID NO: 31 and a light chain variable region of SEQ ID NO: 34;
8) a heavy chain variable region of SEQ ID NO: 31 and a light chain variable region of SEQ ID NO: 35;
9) a heavy chain variable region of SEQ ID NO: 31 and a light chain variable region of SEQ ID NO: 36;
10) a heavy chain variable region of SEQ ID NO: 31 and a light chain variable region of SEQ ID NO: 37;
11) a heavy chain variable region of SEQ ID NO: 32 and a light chain variable region of SEQ ID NO: 30;
12) a heavy chain variable region of SEQ ID NO: 32 and a light chain variable region of SEQ ID NO: 34;
13) a heavy chain variable region of SEQ ID NO: 32 and a light chain variable region of SEQ ID NO: 35;
14) a heavy chain variable region of SEQ ID NO: 32 and a light chain variable region of SEQ ID NO: 36;
15) a heavy chain variable region of SEQ ID NO: 32 and a light chain variable region of SEQ ID NO: 37;
16) a heavy chain variable region of SEQ ID NO: 33 and a light chain variable region of SEQ ID NO: 30;
17) a heavy chain variable region of SEQ ID NO: 33 and a light chain variable region of SEQ ID NO: 34;
18) a heavy chain variable region of SEQ ID NO: 33 and a light chain variable region of SEQ ID NO: 35;
19) a heavy chain variable region of SEQ ID NO: 33 and a light chain variable region of SEQ ID NO: 36; and
20) a heavy chain variable region of SEQ ID NO: 33 and a light chain variable region of SEQ ID NO: 37.

20. The pharmaceutical composition according to any one of claims 17 to 19, wherein the humanized LAG-3 antibody comprises a heavy chain constant region and a light chain constant region, wherein the heavy chain constant region is preferably set forth in SEQ ID NO: 38; and the light chain constant region is preferably set forth in SEQ ID NO:39.

21. The pharmaceutical composition according to claim 20, wherein the heavy chain amino acid sequence of the humanized LAG-3 antibody is set forth in SEQ ID NO: 42 or the sequence has at least 95% sequence identity to SEQ ID NO: 42, and the light chain amino acid sequence is set forth in SEQ ID NO: 43 or the sequence has at least 95% sequence identity to SEQ ID NO: 43.

22. A method for preparing the pharmaceutical composition according to any one of claims 1 to 21, which comprises mixing the LAG-3 antibody or antigen-binding fragment thereof with a buffer, wherein the buffer is selected from the group consisting of acetic acid-sodium acetate buffer, histidine-hydrochloric acid buffer, citric acid-sodium citrate buffer, succinic acid-sodium succinate buffer and Tris buffer, preferably is acetic acid-sodium acetate buffer, or histidine-hydrochloric acid buffer.

23. A method of preparing a lyophilized formulation comprising a LAG-3 antibody or an antigen-binding fragment thereof, which comprises the step of freeze-drying the pharmaceutical composition according to any one of claims 1 to 21.

24. The method of preparing a lyophilized formulation comprising a LAG-3 antibody or an antigen-binding fragment thereof according to claim 23, wherein the freeze-drying includes the steps of pre-freezing, primary drying, and secondary drying, in sequence.

25. The method of preparing a lyophilized formulation comprising a LAG-3 antibody or an antigen-binding fragment thereof according to claim 24, wherein the primary drying is performed at the temperature of from -5°C to -20°C, preferably -10°C.

26. A lyophilized formulation comprising a LAG-3 antibody or an antigen-binding fragment thereof prepared by the method of any one of claims 23 to 25.

27. A lyophilized formulation comprising a LAG3 antibody or an antigen-binding fragment thereof, **characterized in that** the lyophilized formulation can be reconstituted to obtain the pharmaceutical composition according to any one of claims 1 to 21.

28. A method for preparing a reconstituted solution comprising a LAG-3 antibody or an antigen-binding fragment thereof, which comprises the step of reconstituting the lyophilized formulation of claim 26 or 27, wherein the solvent for reconstitution is preferably water for injection.

29. A reconstituted solution comprising a LAG-3 antibody or an antigen-binding fragment thereof prepared by the method of claim 28.

30. A reconstituted solution comprising a LAG-3 antibody or an antigen-binding fragment according to claim 29, which comprises the following components:
i) (a) 40 mg/ml to 80 mg/ml LAG-3 antibody or antigen-binding fragment thereof, (b) 10 mM to 30 mM acetic acid-sodium acetate buffer, pH is about 5.2-5.8, (c) 70 mg/ml to 80 mg/ml sucrose, and (d) 0.4 mg/ml to 0.5 mg/ml polysorbate 80; or
ii) (a) 45 mg/ml to 60 mg/ml LAG-3 antibody or antigen-binding fragment thereof, (b) 10 mM to 30 mM histidine-hydrochloric acid buffer, pH is about 5.5-6.0, (c) 60 mg/ml to 90 mg/ml sucrose or trehalose, and (d) 0.2 mg/ml to 0.6 mg/ml polysorbate 80.

31. The pharmaceutical composition according to any one of claims 1 to 21 or the lyophilized formulation according to claim 26 or 27 or the reconstituted solution according to claim 29 or 30, as a medicament.

32. Use of the pharmaceutical composition according to any one of claims 1 to 21, or the lyophilized formulation according to claim 26 or 27, or the reconstituted solution according to claim 29 or 30, for the preparation of a medicament for treating a disease or condition associated with LAG-3, wherein the disease or condition is a disease or condition involving pathogenic T cells, preferably is a cancer; the cancer is selected from the group consisting of ovarian cancer, melanoma, prostate cancer, intestinal cancer, gastric cancer, esophageal cancer, breast cancer, lung cancer, kidney cancer, pancreatic cancer, uterine cancer, liver cancer, bladder cancer, cervical cancer, oral cancer, brain cancer, testicular cancer, skin cancer, thyroid cancer, and hematological malignancies, including myeloma, chronic and acute leukemia.

33. A method of treating and preventing a disease or condition associated with LAG-3, comprising administering to a patient in need thereof a therapeutically effective amount of the pharmaceutical composition of any one of claims 1 to 21 or the lyophilized formulation according to claim 26 or 27, or the reconstituted solution according to claim 29 or 30, wherein the disease or condition is a disease or condition involving pathogenic T cells, preferably is a cancer; the cancer is selected from the group consisting of ovarian cancer, melanoma, prostate cancer, intestinal cancer, gastric cancer, esophageal cancer, breast cancer, lung cancer, kidney cancer, pancreatic cancer, uterine cancer, liver cancer, bladder cancer, cervical cancer, oral cancer, brain cancer, testicular cancer, skin cancer, thyroid cancer, and hematological malignancies, including myeloma, chronic and acute leukemia.

34. An article comprising a container comprising the pharmaceutical composition according to any one of claims 1 to 21 or the lyophilized formulation according to claim 26 or 27 or the reconstituted solution according to claim 29 or 30.
